# EUROPEAN PATENT APPLICATION

(11) **EP 4 556 460 A1**
(43) Date of publication of application: **21.05.2025**
(21) Application number: 23839045.4
(22) Date of filing: 14.07.2023
(51) Int. Cl.: C07C 235/04, A61K 47/14, A61P 35/00, A61P 37/00

(54) **AMINO LIPID COMPOUND, PREPARATION METHOD THEREFOR, COMPOSITION THEREOF AND APPLICATION THEREOF**

(30) Priority: 15.07.2022 WO PCT/CN2022/105872
(71) Applicant: Shenzhen Shenxin Biotechnology Co., Ltd., Shenzhen, Guangdong 518000 (CN)
(72) Inventor: LI, Linxian, Shenzhen, Guangdong 518000 (CN); CHEN, Yonghao, Shenzhen, Guangdong 518000 (CN); HUANG, Xing, Shenzhen, Guangdong 518000 (CN)
(74) Representative: Nederlandsch Octrooibureau
(86) International application number: PCT/CN2023/107401
(87) International publication number: WO 2024/012556

(57) **Abstract**

An amino lipid compound, a preparation method therefor, a composition thereof and an application thereof. Specifically disclosed are an amino lipid compound represented by formula (I), or a pharmaceutically acceptable salt thereof, or a stereoisomer thereof, and a use thereof in preparing a lipid nanoparticle for delivering an active ingredient, and a composition containing the amino lipid compound, especially a lipid nanoparticle, and a use thereof.

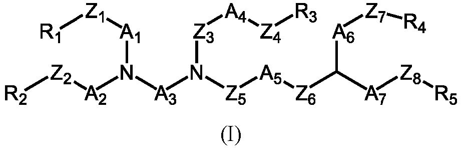

## Description

### Technical Field

The present disclosure relates to an amino lipid compound that can be used to prepare a lipid nanoparticle for delivering an active ingredient and a preparation method therefor. The present disclosure also relates to a composition, especially a lipid nanoparticle, containing the amino lipid compound, and use thereof.

### Background

Gene therapeutic agents deliver genes with specific genetic information to target cells by artificial means, and the expressed target proteins interfere with or regulate the expression of related genes to achieve clinical therapeutic effects. However, it is difficult for naked nucleic acids to be directly introduced into cells. External forces or vectors are required to achieve gene delivery. However, the differences in genetic and cellular structures require matching of lipid particles with different properties. Therefore, there is a need to develop different vectors or adjuvants, especially amino lipid compounds that can be used to deliver nucleic acid active ingredients, as well as related preparation methods and uses, in order to meet the requirements for delivery of gene therapeutic agents.

### Summary of the Invention

One aspect of the present disclosure provides an amino lipid compound represented by formula (I): 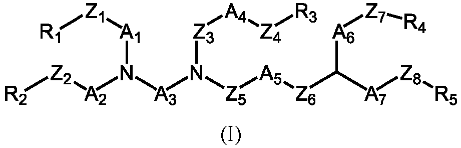 wherein R₁, R₂, R₃, R₄, R₅, Z₁, Z₂, Z₃, Z₄, Z₅, Z₆, Z₇, Z₈, A₁, A₂, A₃, A₄, A₅, A₆ and A₇ are each defined as follows.

Another aspect of the present disclosure provides a method for preparing the amino lipid compound.

Another aspect of the present disclosure provides a use of the amino lipid compound in the manufacture of a vehicle for an active ingredient.

Another aspect of the present disclosure provides a lipid nanoparticle comprising the amino lipid compound.

Another aspect of the present disclosure provides a composition comprising the amino lipid compound.

Another aspect of the present disclosure provides a use of the amino lipid compound, lipid nanoparticle, or composition in the manufacture of a medicament.

Another aspect of the present disclosure provides a use of the amino lipid compound, lipid nanoparticle, or composition in the manufacture of a medicament for nucleic acid transfer.

### Detailed Description of the Invention

### Definitions

Unless otherwise defined below, all technical and scientific terms used herein are intended to have the same meaning as commonly understood by one of ordinary skill in the art. Reference to a technique as used herein is intended to mean a technique as commonly understood in the art, including those variations that are apparent to those skilled in the art, or substitutions of equivalence technique. While it is believed that the following terms are well understood by those skilled in the art, the following definitions are set forth to better explain the present disclosure.

As used herein, the terms "comprising", "including", "having", "containing", or "involving" as well as other variations thereof, are inclusive or open-ended and do not exclude other non-recited elements or method steps.

As used herein, the term "hydrocarbyl" refers to the group remaining after the loss of one hydrogen atom from an aliphatic hydrocarbon, including straight or branched, saturated or unsaturated hydrocarbyl groups. Hydrocarbyl groups include alkyl, alkenyl, and alkynyl groups. Preferably, a hydrocarbyl group has from 1 to 24 carbon atoms (C₁-C₂₄ hydrocarbyl), for example, 1, 2, 3, 4, 5, 6, 7, 8, 9, 10, 11, 12, 13, 14, 15, 16, 17, 18, 19 or 20 carbon atoms (C₁, C₂, C₃, ... C₁₇, C₁₈, C₁₉, or C₂₀ hydrocarbyl). Examples of hydrocarbyl groups include, but are not limited to, C₁-C₂₀ hydrocarbyl, C₁-C₁₈ hydrocarbyl, C₁-C₁₆ hydrocarbyl, C₁-C₁₂ hydrocarbyl, C₁-C₁₀ hydrocarbyl, C₁-C₈ hydrocarbyl, C₁-C₇ hydrocarbyl, C₁-C₆ hydrocarbyl, C₁-C₄ hydrocarbyl, C₁-C₃ hydrocarbyl, C₁-C₂ hydrocarbyl, C₂-C₈ hydrocarbyl, C₂-C₄ hydrocarbyl, C₄-C₈ hydrocarbyl, C₄-C₉ hydrocarbyl, C₅-C₈ hydrocarbyl, C₁-C₄ hydrocarbyl, C₂-C₈ hydrocarbyl, C₃ hydrocarbyl, C₄ hydrocarbyl, C₅ hydrocarbyl, C₆ hydrocarbyl, C₇ hydrocarbyl, and C₈ hydrocarbyl.

As used herein, the term "hydrocarbylene" refers to a divalent group remaining after further loss of one hydrogen atom from the hydrocarbyl as defined above.

As used herein, the term "alkyl" is a straight or branched saturated monovalent hydrocarbyl. Preferably, an alkyl group has from 1 to 24 carbon atoms (C₁-C₂₄ alkyl), for example, 1, 2, 3, 4, 5, 6, 7, 8, 9, 10, 11, 12, 13, 14, 15, 16, 17, 18, 19 or 20 carbon atoms (C₁, C₂, C₃, ... C₁₇, C₁₈, C₁₉, or C₂₀ alkyl). Examples of alkyl groups include, but are not limited to, C₁-C₂₀ alkyl, C₁-C₁₈ alkyl, C₁-C₁₆ alkyl, C₁-C₁₂ alkyl, C₁-C₁₀ alkyl, C₁-C₈ alkyl, C₁-C₇ alkyl, C₁-C₆ alkyl, C₁-C₄ alkyl, C₁-C₃ alkyl, C₁-C₂ alkyl, C₂-C₈ alkyl, C₂-C₄ alkyl, C₄-C₈ alkyl, C₄-C₉ alkyl, C₅-C₈ alkyl, C₁-C₄ alkyl, C₂-C₈ alkyl, methyl, ethyl, n-propyl, isopropyl, n-butyl, sec-butyl, isobutyl, tert-butyl, n-pentyl, n-hexyl, n-heptyl, n-octyl, n-nonyl, n-decyl, n-undecyl, n-dodecyl, n-tridecyl, and tridecan-7-yl.

As used herein, the term "alkylene" refers to a divalent group remaining after further loss of one hydrogen atom from the alkyl as defined above.

As used herein, the term "alkenyl" is a straight or branched monovalent hydrocarbyl containing one or more double bonds (C=C). Preferably, an alkenyl group has from 2 to 24 carbon atoms (C₂-C₂₄ alkenyl), for example, 2, 3, 4, 5, 6, 7, 8, 9, 10, 11, 12, 13, 14, 15, 16, 17, 18, 19 or 20 carbon atoms (C₂, C₃, ... C₁₇, C₁₈, C₁₉, or C₂₀ alkenyl), and has 1, 2, 3, 4, or more double bonds. Alkenyl groups include, but is not limited to, C₂-C₂₀ alkenyl, C₂-C₁₈ alkenyl, C₂-C₁₆ alkenyl, C₂-C₁₂ alkenyl, C₂-C₁₀ alkenyl, C₂-C₈ alkenyl, C₂-C₇ alkenyl, C₂-C₆ alkenyl, C₂-C₄ alkenyl, C₂-C₃ alkenyl, C₄-C₈ alkenyl, C₄-C₉ alkenyl, C₅-C₈ alkenyl having 1, 2, 3, 4 or more double bonds. Some more specific examples include, but are not limited to, vinyl, propenyl, but-1-enyl, but-2-enyl, pent-1-enyl, pent-2-enyl, hex-1-enyl, hex-2-enyl, hex-3-enyl, hept-1-enyl, hept-2-enyl, hept-3-enyl, oct-1-enyl, oct-2-enyl, oct-3-enyl, non-1-enyl, non-2-enyl, and non-3-enyl. **In** some preferred embodiments, the alkenyl group has one double bond.

As used herein, the term "alkenylene" refers to a divalent group remaining after further loss of one hydrogen atom from the alkenyl as defined above.

As used herein, the term "alkynyl" is a straight or branched monovalent hydrocarbyl group containing one or more triple bonds (C≡C). Preferably, an alkynyl group has from 2 to 24 carbon atoms (C₂-C₂₄ alkynyl), for example, 2, 3, 4, 5, 6, 7, 8, 9, 10, 11, 12, 13, 14, 15, 16, 17, 18, 19 or 20 carbon atoms (C₂, C₃, ... C₁₇, C₁₈, C₁₉, or C₂₀ alkynyl), and having 1, 2, 3, 4, or more triple bonds. Alkynyl groups include, but are not limited to, C₂-C₂₀ alkynyl, C₂-C₁₈ alkynyl, C₂-C₁₆ alkynyl, C₂-C₁₂ alkynyl, C₂-C₁₀ alkynyl, C₂-C₈ alkynyl, C₂-C₇ alkynyl, C₂-C₆ alkynyl, C₂-C₄ alkynyl, C₂-C₃ alkynyl, C₄-C₈ alkynyl, C₄-C₉ alkynyl, C₅-C₈ alkynyl having 1, 2, 3, 4 or more triple bonds. Some more specific examples include, but are not limited to, ethynyl, propynyl, but-1-ynyl, but-2-ynyl, pent-1-ynyl, pent-2-ynyl, hex-1-ynyl, hex-2-ynyl, hex-3-ynyl, hept-1-ynyl, hept-2-ynyl, hept-3-ynyl, oct-1-ynyl, oct-2-ynyl, oct-3-ynyl, non-1-ynyl, non-2-ynyl, and non-3-ynyl. In some preferred embodiments, the alkynyl group has one triple bond.

As used herein, the term "alkynylene" refers to a divalent group remaining after further loss of one hydrogen atom from the alkynyl as defined above.

As used herein, the terms "cyclohydrocarbyl", "cyclohydrocarbylene", and "hydrocarbon ring" refer to saturated (i.e., "cycloalkyl" and "cycloalkylene") or unsaturated (i.e., having one or more double bonds (cycloalkenyl) and/or triple bonds (cycloalkynyl) in the ring) monocyclic or polycyclic hydrocarbon rings having, for example, from 3 to 10 (suitably from 3 to 8, more suitably from 3 to 6, such as from 5 to 6 or from 5 to 7) ring carbon atoms, which include, but are not limited to, cyclopropyl(ene) (ring), cyclobutyl(ene) (ring), cyclopentyl(ene) (ring), cyclohexyl(ene) (ring), cycloheptyl(ene) (ring), cyclooctyl(ene) (ring), cyclononyl(ene) (ring), cyclohexenyl(ene) (ring), etc.

As used herein, the term "cycloalkyl" refers to a saturated monocyclic or polycyclic (such as bicyclic) hydrocarbon ring (e.g., monocyclic, such as cyclopropyl, cyclobutyl, cyclopentyl, cyclohexyl, cycloheptyl, cyclooctyl, cyclononyl, or bicyclic, including spirocyclic, fused, or bridged systems, such as bicyclo[1.1.1] pentyl, bicyclo[2.2.1]heptyl, bicyclo[3.2.1]octyl or bicyclo[5.2.0]nonyl, decalin, etc.). Cycloalkyl has, for example, 3 to 10, such as 3-7, 5-6, or 5-7 carbon atoms.

As used herein, the term "heterocycle", "heterocyclyl", or "heterocyclylene" means a saturated or unsaturated cyclic group having a ring heteroatom selected from N, O, and S. Preferably, the heterocycle is an optionally substituted 4- to 10-membered heterocycle having 1, 2, 3, 4, 5, or 6 ring heteroatoms selected from N, O, and S. More preferably, the heterocycle is an optionally substituted 4- to 7-membered saturated heterocycle having 1, 2, 3, or 4 ring heteroatoms selected from N, O, and S, more preferably an optionally substituted 5- to 7-membered (e.g., 5- to 6-membered) saturated heterocycle having 1, 2, or 3 ring heteroatoms selected from N, O, and S. Examples of heterocycle include, but are not limited to, azetidine, oxetanyl, tetrahydrofuran, pyrrolidine, imidazolidine, pyrazolidine, tetrahydropyran, piperidine, morpholine, thiomorpholine, piperazine, preferably pyrrolidine, piperidine, piperazine, and morpholine. The heterocycle may be optionally substituted with one or more substituents, and for the substituents, reference is made to the definition of "optionally substituted" below.

As used herein, the term "aryl" refers to an all-carbon monocyclic or fused polycyclic aromatic group having a conjugated π-electron system. For example, as used herein, the term "C₆₋₁₄ aryl" means an aromatic group containing 6 to 14 (e.g., 6 to 12) carbon atoms, such as phenyl or naphthyl. The aryl group is optionally substituted with 1 or more (such as 1 to 3) suitable substituents.

As used herein, the term "heteroaryl" refers to a monocyclic or polycyclic (e.g., bicyclic or tricyclic) aromatic group having a conjugated π-electron system, of which ring atoms consist of carbon atoms and at least one heteroatom, for example, it has from 5 to 14 (e.g., 5, 6, 7, 8, 9, 10, 11, 12, 13, or 14) ring atoms, including 1, 2, 3, 4, 5, 6, 7, 8, 9, 10, 11, 12, or 13 carbon atoms and 1, 2, 3, 4, or 5 identical or different heteroatoms independently selected from N, O, S, and S(O)₂. One or more ring carbon atoms in the heteroaryl group may be substituted with C(O). The heteroaryl group may be benzo-fused. The heteroaryl group may be optionally substituted with one or more suitable substituents.

As used herein, the term "optionally substituted" means that one or more hydrogen atoms attached to an atom or group are independently unsubstituted or substituted with one or more (e.g., 1, 2, 3, or 4) substituents. The substituents are independently selected from deuterium (D), halogen, -OH, mercapto, cyano, -CD₃ , C₁-C₆ alkyl (preferably C₁-C₃ alkyl), C₂-C₆ alkenyl, C₂-C₆ alkynyl, cycloalkyl (preferably C₃-C₈ cycloalkyl), aryl, heterocyclyl (preferably 3- to 8-membered heterocyclyl), heteroaryl, arylC₁-C₆ alkyl-, heteroarylC₁-C₆ alkyl, C₁-C₆ haloalkyl, -OC₁-C₆ alkyl (preferably -OC₁-C₃ alkyl), -OC₂-C₆ alkenyl, OC₁-C₆ alkylphenyl, C₁-C₆ alkyl-OH (preferably C₁-C₄ alkyl-OH), C₁-C₆ alkyl-SH, C₁-C₆ alkyl-O-C₁-C₆ alkyl, OC₁-C₆ haloalkyl, -NH₂ , C₁-C₆ alkyl-NH₂ (preferably C₁-C₃ alkyl-NH₂), -N(C₁-C₆ alkyl)₂ (preferably -N(C₁-C₃ alkyl)₂), -NH(C₁-C₆ alkyl) (preferably -NH(C₁-C₃ alkyl)), -N(C₁-C₆ alkyl)(C₁-C₆ alkylphenyl), -NH(C₁-C₆ alkylphenyl), nitro, - C(O)-OH, -C(O)OC₁-C₆ alkyl (preferably -C(O)OC₁-C₃ alkyl), -CONRiRii (wherein Ri and Rii are H, D and C₁-C₆ alkyl, preferably C₁-C₃ alkyl), -NHC(O)(C₁-C₆ alkyl), -NHC(O)(phenyl), -N(C₁-C₆ alkyl)C(O)(C₁-C₆ alkyl), -N(C₁-C₆ alkyl)C(O)(phenyl), -C(O)C₁-C₆ alkyl, -C(O)heteroaryl (preferably -C(O)-5- to 7-membered heteroaryl), -C(O)C₁-C₆ alkylphenyl, -C(O)C₁-C₆ haloalkyl, -OC(O)C₁-C₆ alkyl (preferably -OC(O)C₁-C₃ alkyl), -S(O)₂-C₁-C₆ alkyl, -S(O)-C₁-C₆ alkyl, -S(O)₂-phenyl, -S(O)₂-C₁-C₆ haloalkyl, -S(O)₂NH₂, -S(O)₂NH (C₁-C₆ alkyl), -S(O)₂NH(phenyl), -NHS(O)₂(C₁-C₆ alkyl), -NHS(O)₂(phenyl) and -NHS(O)₂(C₁-C₆ haloalkyl), wherein each of the alkyl, cycloalkyl, phenyl, aryl, heterocyclyl, and heteroaryl is optionally further substituted with one or more substituents selected from halogen, -OH, -NH₂, cycloalkyl, 3- to 8-membered heterocyclyl, C₁-C₄ alkyl, C₁-C₄ haloalkyl-, -OC₁-C₄ alkyl, -C₁-C₄ alkyl-OH, -C₁-C₄ alkyl-O-C₁-C₄ alkyl, -OC₁-C₄ haloalkyl, cyano, nitro, -C(O)-OH, -C(O)OC₁-C₆ alkyl, -CON(C₁-C₆ alkyl)₂, -CONH(C₁-C₆ alkyl), -CONH₂ , -NHC(O)(C₁-C₆ alkyl), -NH(C₁-C₆ alkyl)C(O)(C₁-C₆ alkyl), -SO₂(C₁-C₆ alkyl), - SO₂(phenyl), -SO₂(C₁-C₆ haloalkyl), -SO₂NH₂, -SO₂NH(C₁-C₆ alkyl), -SO₂NH(phenyl), -NHSO₂(C₁-C₆ alkyl), -NHSO₂(phenyl), and -NHSO₂(C₁-C₆haloalkyl). When an atom or group is substituted with a plurality of substituents, the plurality of substituents may be the same or different.

In certain embodiments, the substituents may be independently selected from, but are not limit to, a halogen (such as a chlorine, bromine, fluorine, or iodine), deuterium (D), tritium (T), a carboxylic acid (such as -C(=O)-OH), an oxygen (such as =O), a sulfur (such as =S), a hydroxyl (such as -OH), an ester group (such as -C(=O)ORiii or -OC(=O)Riii), an aldehyde group (such as -C(=O)H), a carbonyl (such as -C(=O)Riii, or represented by C=O), an acyl halide (such as -C(=O)X, wherein X is selected from bromine, fluorine, chlorine, or iodine), a carbonic ester group (such as -OC(=O)ORiii), an alkoxy (such as -ORiii), an acetal (such as -C(ORiii)₂Riii, wherein each ORiii is the same or different and is an alkoxy group), a phosphate (such as P(=O)₄³⁻), a thiol (such as -SH), a sulfoxide (such as -S(=O)Riii), a sulfinic acid (such as -S(=O)OH), a sulfonic acid (such as -S(=O)₂OH), a thioaldehyde (such as -C(=S)H), a sulfate (such as S(=O)₄²⁻), a sulfonyl (such as -S(=O)₂Riii), a sulfinyl (such as -S(=O)Riii), an amide (such as -C(=O)N(Riii)₂ or - N(Riii)C(=O)Riii), an azido (such as -N₃), a nitro (such as -NO₂), a cyano (such as -CN), an isocyano (such as -NC), an acyloxy (such as -OC(=O)Riii), an amino (such as -N(Riii)₂, -N(Riii)H or -NH₂), a carbamoyl (such as -OC(=O)N(Riii)₂, -OC(=O)N(Riii)H or -OC(=O)NH₂), a sulfonamide (such as -S(=O)₂N(Riii)₂, - S(=O)₂N(Riii)H, -S(=O)₂NH₂, -N(Riii)S(=O)₂Riii, -N(H)S(=O)₂Riii, -N(Riii)S(=O)₂H or -N(H)S(=O)₂H), an alkyl, an alkenyl, an alkynyl, a cyclohydrocarbyl (such as cycloalkyl, cycloalkenyl or cycloalkynyl), a heterocyclohydrocarbyl (such as heterocycloalkyl containing one or more heteroatoms selected from S, N, and O, or heterocycloalkenyl containing one or more heteroatoms selected from S, N, and O), an aryl (such as phenyl, or a fused ring group), a heteroaryl (such as an 8- to 10-membered bicyclic heteroaryl containing 1 to 4 heteroatoms independently selected from nitrogen, oxygen, or sulfur), -C(=O)SRiii, -C(=N-CN)N(Riii)₂, -C(=N-O-CH₃)N(Riii)₂, -C(=N-SO₂-NH₂)N(Riii)₂, -C(=CH-NO₂)N(Riii)₂, -OC(=O)N(Riii)₂, - CHN(Riii)N(Riii)₂, -C(=O)N(Riii)ORiii, -N(Riii)₂C(=O)ORiii, -OP(=O)(ORiii)₂, -P(=O)(ORiii)₂, - N(ORiii)C(=O)Riii, -N(ORiii)S(=O)₂Riii, -N(ORiii)C(=O)ORiii, -N(ORiii)C(=O)N(Riii)₂, - N(ORiii)C(=S)N(Riii)₂, -N(ORiii)C(NRiii)N(Riii)₂, -N(ORiii)C(CHRiii)N(Riii)₂. In any of the foregoing, Riii is a hydrogen, or alkyl, or alkenyl, or alkynyl, or heteroalkyl, or heteroalkenyl, or heteroalkynyl, as defined herein. In some embodiments, Riii is a hydrogen, or C₁-C₁₂ alkyl, or C₁-C₁₂ alkenyl, or C₁-C₁₂ alkynyl, or C₁-C₁₂ heteroalkyl, or C₁-C₁₂ heteroalkenyl, or C₁-C₁₂ heteroalkynyl, as defined herein.

In certain embodiments, the substituent itself may be further substituted with, for example, one or more substituents as defined herein. For example, the C₁-C₆ alkyl as a substituent may be further substituted with one or more substituents as define herein.

In certain embodiments, the hydrocarbyl, hydrocarbylene, alkyl, alkylene, alkenyl, alkenylene, alkynyl, alkynylene, cyclohydrocarbyl, cyclohydrocarbylene, hydrocarbon ring, cycloalkyl, heterocycle, heterocyclyl, heterocyclylene, aryl, and heteroaryl groups as described herein may be optionally substituted with one or more substituents.

As used herein, that term "halo" or "halogen" group is defined to include F, Cl, Br, or I.

As used herein, the "C(=O)" and "(C=O)" groups can be used interchangeably and represent 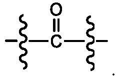 For example, the -(C=O)O- group represents

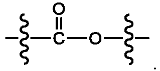

As used herein, the term "compound" encompasses all compounds that are isotopically labeled by substituting one or more atoms with atoms having different atomic weights or mass numbers. "Isotopes" refer to atoms having the same atomic numbers but different mass numbers due to different numbers of neutrons in the nucleus. For example, isotopes of hydrogen include tritium and deuterium.

A numerical range stated herein should be understood to encompass any and all subranges contained therein. For example, a range of "1 to 10" should be understood to include not only the explicitly recited values of 1 and 10, but also any individual values in the range of 1 to 10 (e.g., 2, 3, 4, 5, 6, 7, 8, and 9) and subranges (e.g., 1 to 2, 1.5 to 2.5, 1 to 3, 1.5 to 3.5, 2.5 to 4, 3 to 4.5, etc.). This principle also applies to ranges that use only one value as a minimum or maximum.

As used herein, "pharmaceutically acceptable salt" refers to an acid-addition salt or a base-addition salt of a compound of the present disclosure which retains the biological effectiveness and properties of the compound of the present disclosure and is not typically biologically or otherwise undesirable. In many cases, the compound of the present invention can form an acid and/or base salt due to the presence of an amino and/or carboxyl group or a similar group.

Pharmaceutically acceptable acid addition salts can be formed from the compound of the present disclosure and inorganic and/or organic acids, the inorganic acids being such as, but not limited to, hydrochloric acid, hydrobromic acid, sulfuric acid, nitric acid, and phosphoric acid; and the organic acids being such as, but not limited to, acetic acid, 2,2-dichloroacetic acid, adipic acid, alginic acid, ascorbic acid, aspartic acid, benzenesulfonic acid, benzoic acid, 4-acetamidobenzoic acid, camphanic acid, camphor-10-sulfonic acid, capric acid, hexanoic acid, octanoic acid, carbonic acid, cinnamic acid, citric acid, cyclamic acid, dodecyl sulfuric acid, ethane-1,2-disulfonic acid, ethanesulfonic acid, 2-hydroxyethanesulfonic acid, formic acid, fumaric acid, galactaric acid, gentisic acid, glucoheptonic acid, gluconic acid, glucuronic acid, glutamic acid, glutaric acid, 2-oxoglutaric acid, glycerophosphoric acid, glycolic acid, hippuric acid, isobutyric acid, lactic acid, lactobionic acid, lauric acid, maleic acid, malic acid, malonic acid, mandelic acid, methanesulfonic acid, mucic acid, naphthalene-1,5-disulfonic acid, naphthalene-2-sulfonic acid, 1-hydroxy-2-naphthoic acid, nicotinic acid, oleic acid, orotic acid, oxalic acid, palmitic acid, pamoic acid, propionic acid, pyroglutamic acid, pyruvic acid, salicylic acid, 4-aminosalicylic acid, sebacic acid, stearic acid, succinic acid, tartaric acid, thiocyanic acid, p-toluenesulfonic acid, trifluoroacetic acid, and undecylenic acid.

Pharmaceutically acceptable base addition salts can be formed from the compound of the present disclosure and inorganic and/or organic bases. Salts derived from inorganic bases include, but are not limited to, sodium, potassium, lithium, ammonium, calcium, magnesium, iron, zinc, copper, manganese, aluminum salts, etc. Preferred inorganic salts are ammonium, sodium, potassium, calcium, and magnesium salts. Salts derived from organic bases include, but are not limited to, salts of primary amines, secondary amines, tertiary amines, substituted amines (including naturally occurring substituted amines), cyclic amines, and basic ion exchange resins as following: such as ammonia, isopropylamine, trimethylamine, diethylamine, triethylamine, tripropylamine, diethanolamine, ethanolamine, deanol, 2-dimethylaminoethanol, 2-diethylaminoethanol, dicyclohexylamine, lysine, arginine, histidine, caffeine, procaine, hydrabamine, choline, betaine, benethamine, benzathine, ethylenediamine, glucosamine, methylglucosamine, theobromine, triethanolamine, tromethamine, purine, piperazine, piperidine, N-ethylpiperidine, and polyamine resins. Particularly preferred organic bases are isopropylamine, diethylamine, ethanolamine, trimethylamine, dicyclohexylamine, choline, and caffeine.

The compound of the present disclosure, or a pharmaceutically acceptable salt thereof may contain one or more asymmetric centers and thus may give rise to enantiomers, diastereomers, and other stereoisomeric forms, such as amino acids, which may be defined as (R)- or (S)-, or as (D)- or (L)- on the basis of the absolute stereochemistry. The present invention is intended to include all these possible isomers and racemic and optically pure forms thereof. Optically active (+) and (-), (R)- and (S)-, or (D)- and (L)-isomers can be prepared using chiral synthons or chiral reagents, or by resolution by conventional techniques such as chromatography and fractional crystallization. Conventional techniques for preparing/isolating single enantiomers include chiral synthesis from suitable optically pure precursors, or resolution of racemates (or racemates of salts or derivatives) using, for example, chiral high-pressure liquid chromatography (HPLC). In the case where the compound described herein contains an olefinic double bond or other geometrically asymmetric center, the compound includes E- and Z-geometric isomers, unless otherwise specified. Likewise, all tautomeric forms are also intended to be contemplated.

As used herein, the term "stereoisomer" refers to a compounds consisting of the same atoms bonded via the same bonds but having different three-dimensional structures, which are not interchangeable. "Enantiomers" are a pair of stereoisomers that are non-overlapping mirror images of each other. A mixture of any ratio of a pair of enantiomers may be referred to as a "racemic" mixture. "Diastereomers" are stereoisomers that have at least two asymmetric atoms and are not mirror images of each other.

"Stereoisomers" may also include the E and Z isomers, or mixtures thereof, as well as the *cis* and *trans* isomers, or mixtures thereof. In certain embodiments, the compound described herein is isolated as an E or Z isomer. In other embodiments, the compound described herein is a mixture of E and Z isomers.

"Tautomers" refer to isomeric forms of a compound that are in equilibrium with each other. The concentration of the isomeric forms will depend on the environment in which the compound is found and may vary, for example, depending on whether the compound is solid or in an organic or aqueous solution.

### Amino lipid compound

In one aspect, the present disclosure provides an amino lipid compound represented by the formula (I) as following: 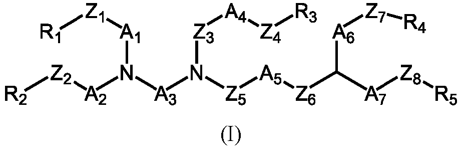
or a pharmaceutically acceptable salt thereof or a stereoisomer thereof,
wherein:
   Z₁, Z₂, Z₅, Z₆, Z₇ and Z₈ are each independently -C(=O)-, -CH(OH)-, -C=C-, -C≡C-, -O-, -(C=O)O-, - O(C=O)-, -C(=O)S-, -SC(=O)-, -S-S-, or a bond;
   Z₃ is -C(=O)- or a bond;
   Z₄ is -O-, or -CH (OH)-;
   A₁, A₂, A₃, A₄, A₅, A₆ and A₇ are each independently C₁-C₁₂ hydrocarbylene, cyclohydrocarbyl, phenyl, heterocycle, or a bond;
   R₁ and R₂ are each independently H or C₁-C₁₈ hydrocarbyl, or cyclohydrocarbyl, phenyl, heterocycle; or R₁ and R₂, together with the nitrogen atom to which they are attached, form 4- to 7-membered heterocycle;
   R₃ is H or C₁-C₁₈ hydrocarbyl, or cyclohydrocarbyl, phenyl, heterocycle; and
   R₄ and R₅ are each independently C₁-C₂₄ hydrocarbyl, or cyclohydrocarbyl, phenyl, heterocycle;
   preferably, A₅, Z₅, and Z₆ are bonds.

In some embodiments, the present disclosure provides the amino lipid compound of formula (I), or a pharmaceutically acceptable salt thereof or a stereoisomer thereof, as described above, wherein A₁ and A₂ are each independently C₁-C₈ hydrocarbylene or a bond, such as C₁-C₆ hydrocarbylene or a bond, or C₁-C₄ hydrocarbylene or a bond. In some embodiments, A₁ and A₂ are each independently C₁-C₈ alkylene, C₂-C₈ alkenylene, or C₂-C₈ alkynylene. In some embodiments, A₁ and A₂ are each independently C₁-C₈ alkylene, such as C₁-C₆ alkylene, C₁-C₄ alkylene, C₂-C₄ alkylene, or C₁-C₃ alkylene. In some embodiments, both A₁ and A₂ are bonds.

In some embodiments, the present disclosure provides the amino lipid compound of formula (I), or a pharmaceutically acceptable salt thereof or a stereoisomer thereof, as described above, wherein A₃ is C₁-C₁₂ hydrocarbylene or a bond. In some embodiments, A₃ is straight C₁-C₁₂ hydrocarbylene. In some embodiments, A₃ is straight C₁-C₁₂ alkylene, C₂-C₁₂ alkenylene, or C₂-C₁₂ alkynylene. In some embodiments, A₃ is straight C₁-C₅ alkylene, such as straight C₁, C₂, C₃, C₄, or C₅ alkylene. In some embodiments, A₃ is -CH₂CH₂-, - CH₂CH₂CH₂-, or -CH₂CH₂CH₂CH₂-.

In some embodiments, the present disclosure provides the amino lipid compound of formula (I), or a pharmaceutically acceptable salt thereof or a stereoisomer thereof, as described above, wherein A₄ is C₁-C₁₂ hydrocarbylene or a bond. In some embodiments, A₄ is straight C₁-C₁₂ hydrocarbylene. In some embodiments, A₄ is straight C₁-C₁₂ alkylene, C₂-C₁₂ alkenylene, or C₂-C₁₂ alkynylene. In some embodiments, A₄ is straight C₁-C₇ alkylene, such as straight C₁, C₂, C₃, C₄, C₅, C₆, or C₇ alkylene. In some embodiments, A₄ is straight C₁-C₃ alkylene, such as straight C₁, C₂, or C₃ alkylene. In some embodiments, A₄ is -CH₂-, -CH₂CH₂-, or - CH₂CH₂CH₂-.

In some embodiments, the present disclosure provides the amino lipid compound of formula (I), or a pharmaceutically acceptable salt thereof or a stereoisomer thereof, as described above, wherein A₅ is C₁-C₈ hydrocarbylene or a bond, such as C₁-C₆ hydrocarbylene or a bond, C₁-C₄ hydrocarbylene or a bond, or C₁-C₃ hydrocarbylene or a bond. In some embodiments, A₅ is C₁-C₈ alkylene, C₂-C₈ alkenylene, or C₂-C₈ alkynylene. In some embodiments, A₅ is C₁-C₈ alkylene, such as C₁-C₆ alkylene, C₁-C₄ alkylene, C₂-C₄ alkylene, or C₁-C₃ alkylene. In some embodiments, A₅ is a bond.

In some embodiments, the present disclosure provides the amino lipid compound of formula (I), or a pharmaceutically acceptable salt thereof or a stereoisomer thereof, as described above, wherein A₆ and A₇ are each independently C₁-C₁₂ hydrocarbylene. In some embodiments, A₆ and A₇ are each independently straight C₁-C₁₂ hydrocarbylene. In some embodiments, A₆ and A₇ are each independently straight C₁-C₁₂ alkylene, C₂-C₁₂ alkenylene, or C₂-C₁₂ alkynylene. In some embodiments, A₆ and A₇ are each independently straight C₁-C₁₂ alkylene, such as straight C₂-C₁₂ alkylene, C₄-C₁₀ alkylene, C₆-C₈ alkylene, C₆-C₁₀ alkylene, or C₄-C₈ alkylene. In some embodiments, A₆ and A₇ are each independently straight C₆-C₁₀ alkylene, such as straight C₆, C₇, C₈, C₉, or C₁₀ alkylene. In some embodiments, A₆ and A₇ are each independently -(CH₂)₆-, - (CH₂)₇-, -(CH₂)₈-, or -(CH₂)₉-.

In some embodiments, the present disclosure provides the amino lipid compound of formula (I), or a pharmaceutically acceptable salt thereof or a stereoisomer thereof, as described above, wherein R₁ and R₂ are each independently H; C₁-C₁₈ hydrocarbyl, such as C₁-C₁₆ hydrocarbyl, C₁-C₁₂ hydrocarbyl, C₁-C₁₀ hydrocarbyl, C₁-C₈ hydrocarbyl, C₁-C₆ hydrocarbyl, or C₁-C₄ hydrocarbyl; C₃-C₇ cyclohydrocarbyl, such as C₃-C₆ cyclohydrocarbyl or C₅-C₆ cyclohydrocarbyl; phenyl; or 3- to 7-membered heterocycle, such as 4- to 6-membered heterocycle; or R₁ and R₂, together with the nitrogen atom to which they are attached, form 4-to 7-membered heterocycle, such as 5- to 6-membered heterocycle.

In some embodiments, R₁ and R₂ are each independently H; C₁-C₁₈ alkylene, C₂-C₁₈ alkenylene, or C₂-C₁₈ alkynylene. In some embodiments, R₁ and R₂ are each independently C₁-C₁₈ alkyl, such as C₁-C₄ alkyl; C₃-C₆ cycloalkyl, such as cyclopropyl, cyclobutyl, cyclopentyl, or cyclohexyl; phenyl; or 5- to 6-membered heterocycle. In some embodiments, R₁ and R₂, together with the nitrogen atom to which they are attached, form 5- to 6-membered heterocycle, such as pyrrolidine, piperidine, piperazine, or morpholine.

In some embodiments, R₁ and R₂ are each independently C₁-C₄ alkyl, such as C₁, C₂, C₃, or C₄ alkyl. In some embodiments, R₁ and R₂ are each independently methyl, ethyl, n-propyl, isopropyl, n-butyl, sec-butyl, isobutyl, or tert-butyl. In some embodiments, R₁ and R₂ are each independently C₁-C₃ alkyl, such as C₁, C₂, or C₃ alkyl. In some embodiments, R₁ and R₂ are each independently methyl, ethyl, or n-propyl.

In some embodiments, the present disclosure provides the amino lipid compound of formula (I), or a pharmaceutically acceptable salt thereof or a stereoisomer thereof, as described above, wherein R₃ is H; C₁-C₁₈ hydrocarbyl, such as C₁-C₁₆ hydrocarbyl, C₂-C₁₂ hydrocarbyl, C₄-C₁₀ hydrocarbyl, or C₄-C₈ hydrocarbyl; C₃-C₇ cyclohydrocarbyl, such as C₃-C₆ cyclohydrocarbyl or C₅-C₆ cyclohydrocarbyl; phenyl; or 3- to 7-membered heterocycle, such as 4- to 6-membered heterocycle. In some embodiments, R₃ is C₁-C₁₈ alkyl, C₂-C₁₈ alkenyl, or C₂-C₁₈ alkynyl. In some embodiments, R₃ is C₁-C₁₈ alkyl, such as C₂-C₁₀ alkyl, C₄-C₈ alkyl; C₃-C₆ cycloalkyl, such as cyclopropyl, cyclobutyl, cyclopentyl, or cyclohexyl; phenyl; or 5- to 6-membered heterocycle. In some embodiments, R₃ is straight C₄-C₈ alkyl, such as straight C₄, C₅, C₆, C₇, or C₈ alkyl. In some embodiments, R₃ is n-butyl, n-pentyl, n-hexyl, n-heptyl, or n-octyl.

In some embodiments, the present disclosure provides the amino lipid compound of formula (I), or a pharmaceutically acceptable salt thereof or a stereoisomer thereof, as described above, wherein Z₁, Z₂, Z₅ and Z₆ are each independently -C(=O)-, -O(C=O)-, -O-, or a bond. In some embodiments, Z₁, Z₂, Z₅ and Z₆ are each independently a bond.

In some embodiments, the present disclosure provides the amino lipid compound of formula (I), or a pharmaceutically acceptable salt thereof or a stereoisomer thereof, as described above, wherein Z₃ is -C(=O)-.

In some embodiments, the present disclosure provides the amino lipid compound of formula (I), or a pharmaceutically acceptable salt thereof or a stereoisomer thereof, as described above, wherein Z₇ and Z₈ are each independently -(C=O)O-, -O(C=O)-, or a bond.

In some embodiments, the present disclosure provides the amino lipid compound of formula (I), or a pharmaceutically acceptable salt thereof or a stereoisomer thereof, as described above, wherein one of Z₇ or Z₈ is -(C=O)O-, -O(C=O)-, or a bond.

In some embodiments, the present disclosure provides the amino lipid compound of formula (I), or a pharmaceutically acceptable salt thereof or a stereoisomer thereof, as described above, wherein each of Z₇ and Z₈ is -(C=O)O-. In some embodiments, the (C=O) in Z₇ is attached to A₆, and the (C=O) in Z₈ is attached to A₇.

In some embodiments, the present disclosure provides the amino lipid compound of formula (I), or a pharmaceutically acceptable salt thereof or a stereoisomer thereof, as described above, wherein each of Z₇ and Z₈ is -O(C=O)-. In some embodiments, the (C=O) in Z₇ is attached to R₄, and the (C=O) in Z₈ is attached to R₅.

In some embodiments, the present disclosure provides the amino lipid compound of formula (I), or a pharmaceutically acceptable salt thereof or a stereoisomer thereof, as described above, wherein one of Z₇ or Z₈ is a bond. In some embodiments, Z₇ is a bond, and Z₈ is -(C=O)O-. In some embodiments, the (C=O) in Z₈ is attached to A₇. In some embodiments, Z₇ is a bond, and Z₈ is -O(C=O)-. In some embodiments, the (C=O) in Z₈ is attached to R₅. In some embodiments, Z₈ is a bond, and Z₇ is -(C=O)O-. In some embodiments, the (C=O) in Z₇ is attached to A₆. In some embodiments, Z₈ is a bond, and Z₇ is -O(C=O)-. In some embodiments, the (C=O) in Z₇ is attached to R₄.

In some embodiments, the present disclosure provides the amino lipid compound of formula (I), or a pharmaceutically acceptable salt thereof or a stereoisomer thereof, as described above, wherein each of Z₇ and Z₈ is a bond.

In some embodiments, the present disclosure provides the amino lipid compound of formula (I), or a pharmaceutically acceptable salt thereof or a stereoisomer thereof, as described above, wherein R₄ and R₅ are each independently C₁-C₂₀ hydrocarbyl; C₃-C₇ cyclohydrocarbyl, such as C₃-C₆ cyclohydrocarbyl or C₅-C₆ cyclohydrocarbyl; phenyl; or 3- to 7-membered heterocycle, such as 4- to 6-membered heterocycle. In some embodiments, R₄ and R₅ are each independently C₁-C₂₀ hydrocarbyl; C₃-C₆ cycloalkyl, such as cyclopropyl, cyclobutyl, cyclopentyl, or cyclohexyl; phenyl; or 5- to 6-membered heterocycle.

In some embodiments, the present disclosure provides the amino lipid compound of formula (I), or a pharmaceutically acceptable salt thereof or a stereoisomer thereof, as described above, wherein R₄ and R₅ are each independently C₁-C₂₀ hydrocarbyl.

In some embodiments, the present disclosure provides the amino lipid compound of formula (I), or a pharmaceutically acceptable salt thereof or a stereoisomer thereof, as described above, wherein R₄ and R₅ are each independently C₁-C₂₀ alkyl.

In some embodiments, the present disclosure provides the amino lipid compound of formula (I), or a pharmaceutically acceptable salt thereof or a stereoisomer thereof, as described above, wherein R₄ and R₅ are each independently branched C₃-C₂₀ alkyl, such as branched C₅-C₂₀ alkyl, branched C₇-C₁₉ alkyl, branched C₉-C₁₈ alkyl, branched C₁₀-C₁₈ alkyl, branched C₃-C₁₀ alkyl, branched C₁₁-C₁₇ alkyl, or branched C₁₈-C₂₀ alkyl. In some embodiments, R₄ and R₅ are each independently branched C₁₁-C₁₇ alkyl, such as branched C₁₁, C₁₂, C₁₃, C₁₄, C₁₅, C₁₆, or C₁₇ alkyl. In some embodiments, R₄ and R₅ are each independently branched C₁₁-C₁₃ alkyl, such as branched C₁₁, C₁₂, or C₁₃ alkyl. In some embodiments, R₄ is a branched alkyl group wherein branching occurs at the α, β, or γ position relative to Z₇, such as 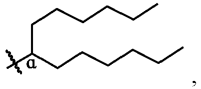 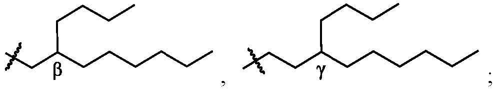 R₅ is a branched alkyl group wherein branching occurs at the α, β or γ position relative to Z₈, such as 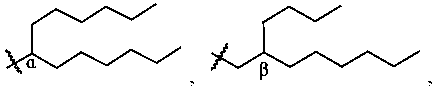 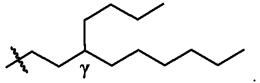 In some embodiments, R₄ is a branched alkyl group wherein branching occurs at the α or β position relative to Z₇; R₅ is a branched alkyl group wherein branching occurs at the α or β position relative to Z₈.

In some embodiments, the present disclosure provides the amino lipid compound of formula (I), or a pharmaceutically acceptable salt thereof or a stereoisomer thereof, as described above, wherein R₄ and R₅ are each independently one of the following structures:

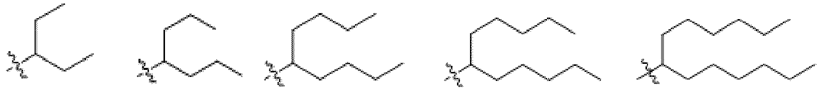

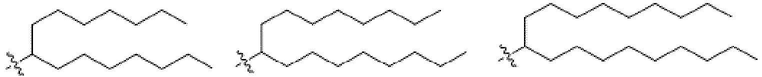

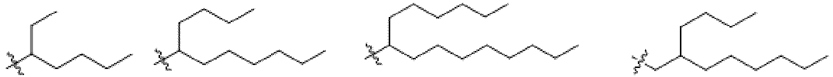

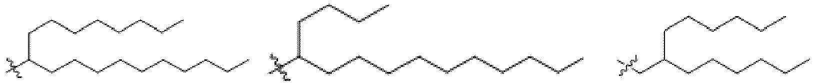

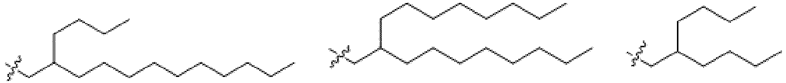

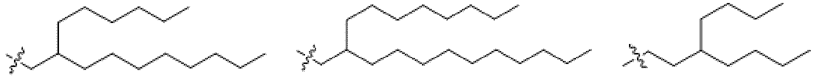

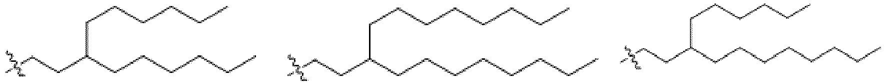

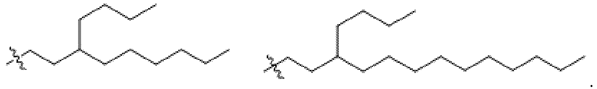

In some embodiments, the present disclosure provides the amino lipid compound of formula (I), or a pharmaceutically acceptable salt thereof or a stereoisomer thereof, as described above, wherein R₄ and R₅ are each independently one of the following structures:

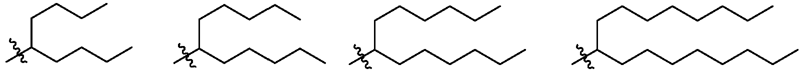

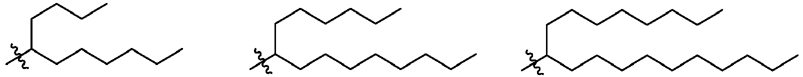

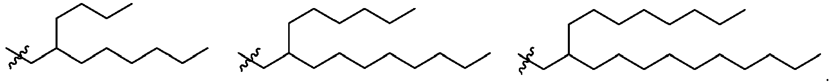

In some embodiments, the present disclosure provides the amino lipid compound of formula (I), or a pharmaceutically acceptable salt thereof or a stereoisomer thereof, as described above, wherein R₄ and R₅ are each independently

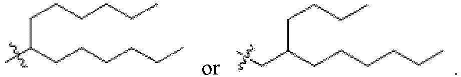

In some embodiments, the present disclosure provides the amino lipid compound of formula (I), or a pharmaceutically acceptable salt thereof or a stereoisomer thereof, as described above, wherein:
Z₃ is -C(=O)-; and
A₄ is C₁-C₁₂ alkylene. In some embodiments, A₄ is straight C₁-C₁₂ alkylene, such as straight C₁-C₁₀ alkylene, C₁-C₈ alkylene, C₁-C₆ alkylene, C₁-C₄ alkylene, or C₁-C₃ alkylene. In some embodiments, A₄ is straight C₁-C₄ alkylene, such as straight C₁, C₂, C₃, or C₄ alkylene. In some embodiments, A₄ is -CH₂-, - CH₂CH₂-, -CH₂CH₂CH₂-, or -CH₂CH₂CH₂CH₂-.

In some embodiments, the present disclosure provides the amino lipid compound of formula (I), or a pharmaceutically acceptable salt thereof or a stereoisomer thereof, as described above, wherein Z₄ is -O-.

In some embodiments, the present disclosure provides the amino lipid compound of formula (I), or a pharmaceutically acceptable salt thereof or a stereoisomer thereof, as described above, wherein Z₄ is - CH(OH)-.

In another aspect, the present disclosure provides amino lipid compounds represented by formulae (II) and (III) as following: 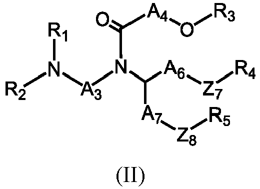 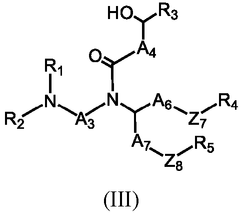
or a pharmaceutically acceptable salt thereof or a stereoisomer thereof,
wherein:
   Z₇ and Z₈ are each independently -C(=O)-, -CH(OH)-, -C=C-, -C≡C-, -O-, -(C=O)O-, -O(C=O)-, - C(=O)S-, -SC(=O)-, -S-S-, or a bond;
   A₃, A₄, A₆ and A₇ are each independently C₁-C₁₂ hydrocarbylene, cyclohydrocarbyl, phenyl, heterocycle, or a bond;
   R₁ and R₂ are each independently H or C₁-C₁₈ hydrocarbyl, or cyclohydrocarbyl, phenyl, heterocycle; or R₁ and R₂, together with the nitrogen atom to which they are attached, form 4- to 7-membered heterocycle;
   R₃ is H or C₁-C₁₈ hydrocarbyl, or cyclohydrocarbyl, phenyl, heterocycle; and
   R₄ and R₅ are each independently C₁-C₂₄ hydrocarbyl, or cyclohydrocarbyl, phenyl, heterocycle.

In some embodiments, the present disclosure provides the amino lipid compounds of formulae (II) and (III), or a pharmaceutically acceptable salt thereof or a stereoisomer thereof, as described above, wherein Z₇ and Z₈ are each independently -(C=O)O-, -O(C=O)-, or a bond. In some embodiments, each of Z₇ and Z₈ is -(C=O)O-. In some embodiments, the (C=O) in Z₇ is attached to A₆, and the (C=O) in Z₈ is attached to A₇. In some embodiments, each of Z₇ and Z₈ is -O(C=O)-. In some embodiments, the (C=O) in Z₇ is attached to R₄, and the (C=O) in Z₈ is attached to R₅.

In some embodiments, the present disclosure provides the amino lipid compounds of formulae (II) and (III), or a pharmaceutically acceptable salt thereof or a stereoisomer thereof, as described above, wherein A₃, A₄, A₆ and A₇ are each independently C₁-C₁₂ alkylene. In some embodiments, A₃, A₄, A₆ and A₇ are each independently C₁, C₂, C₃, C₄, C₅, C₆, C₇, C₈, C₉, C₁₀, C₁₁, or C₁₂ alkylene.

In some embodiments, A₃ is straight C₁, C₂, C₃, C₄, C₅, C₆, C₇, C₈, C₉, C₁₀, C₁₁, or C₁₂ alkylene. In some embodiments, A₃ is straight C₁, C₂, C₃, C₄, or C₅ alkylene. In some embodiments, A₃ is straight C₂, C₃, or C₄ alkylene, such as -CH₂CH₂-, -CH₂CH₂CH₂-, or -CH₂CH₂CH₂CH₂-.

In some embodiments, A₄ is straight C₁, C₂, C₃, C₄, C₅, C₆, C₇, C₈, C₉, C₁₀, C₁₁, or C₁₂ alkylene. In some embodiments, A₄ is straight C₁, C₂, C₃, or C₄ alkylene, such as -CH₂-, -CH₂CH₂-, or -CH₂CH₂CH₂-.

In some embodiments, A₆ and A₇ are each independently straight C₁, C₂, C₃, C₄, C₅, C₆, C₇, C₈, C₉, C₁₀, C₁₁, or C₁₂ alkylene. In some embodiments, A₆ and A₇ are each independently straight C₆, C₇, C₈, C₉, or C₁₀ alkylene, such as -(CH₂)₆-, -(CH₂)₇-, -(CH₂)₈-, or -(CH₂)₉-.

In some embodiments, the present disclosure provides the amino lipid compounds of formulae (II) and (III), or a pharmaceutically acceptable salt thereof or a stereoisomer thereof, as described above, wherein R₁ and R₂ are each independently C₁-C₁₈ alkyl. In some embodiments, R₁ and R₂ are each independently C₁-C₆ alkyl. In some embodiments, R₁ and R₂ are each independently methyl, ethyl, n-propyl, or isopropyl. In some embodiments, R₁ and R₂ are each independently C₁-C₄ alkyl, such as C₁, C₂, C₃, or C₄ alkyl. In some embodiments, R₁ and R₂ are each independently methyl.

In some embodiments, the present disclosure provides the amino lipid compounds of formulae (II) and (III), or a pharmaceutically acceptable salt thereof or a stereoisomer thereof, as described above, wherein R₁ and R₂, together with the nitrogen atom to which they are attached, form 4- to 7-membered heterocycle. In some embodiments, R₁ and R₂, together with the nitrogen atom to which they are attached, form 5- to 6-membered heterocycle.

In some embodiments, the present disclosure provides the amino lipid compounds of formulae (II) and (III), or a pharmaceutically acceptable salt thereof or a stereoisomer thereof, as described above, wherein R₃ is C₁-C₁₈ alkyl. In some embodiments, R₃ is C₁, C₂, C₃, C₄, C₅, C₆, C₇, C₈, C₉, C₁₀, C₁₁, or C₁₂ alkyl. In some embodiments, R₃ is straight C₂, C₃, C₄, C₅, C₆, C₇, C₈, C₉, or C₁₀ alkyl. In some embodiments, R₃ is straight C₄, C₅, C₆, C₇, or C₈ alkyl, such as n-butyl, n-pentyl, n-hexyl, n-heptyl, or n-octyl.

In some embodiments, the present disclosure provides the amino lipid compounds of formulae (II) and (III), or a pharmaceutically acceptable salt thereof or a stereoisomer thereof, as described above, wherein R₄ and R₅ are each independently C₁-C₂₀ hydrocarbyl.

In some embodiments, R₄ and R₅ are each independently C₁-C₂₀ alkyl.

In some embodiments, R₄ and R₅ are each independently branched C₃-C₂₀ alkyl, such as branched C₅-C₂₀ alkyl, branched C₇-C₁₉ alkyl, branched C₉-C₁₈ alkyl, branched C₁₀-C₁₈ alkyl, branched C₃-C₁₀ alkyl, branched C₁₁-C₁₇ alkyl, or branched C₁₈-C₂₀ alkyl. In some embodiments, R₄ and R₅ are each independently branched C₁₁-C₁₇ alkyl, such as branched C₁₁, C₁₂, C₁₃, C₁₄, C₁₅, C₁₆, or C₁₇ alkyl. In some embodiments, R₄ and R₅ are each independently branched C₁₁-C₁₃ alkyl, such as branched C₁₁, C₁₂, or C₁₃ alkyl. In some embodiments, R₄ is a branched alkyl group wherein branching occurs at the α, β, or γ position relative to Z₇; R₅ is a branched alkyl group wherein branching occurs at the α, β, or γ position relative to Z₈. In some embodiments, R₄ is a branched alkyl group wherein branching occurs at the α or β position relative to Z₇; R₅ is a branched alkyl group wherein branching occurs at the α or β position relative to Z₈. In some embodiments, R₄ and R₅ are each independently one of the following structures:

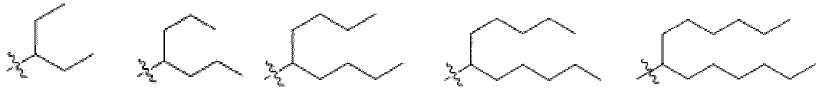

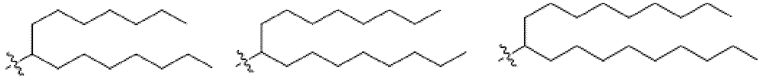

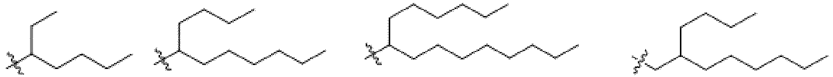

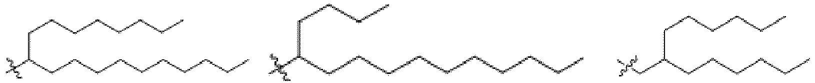

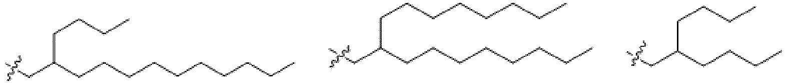

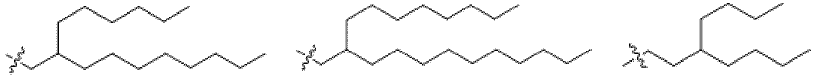

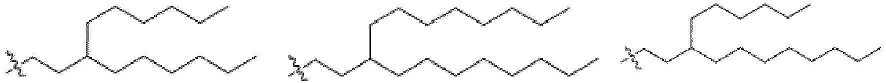

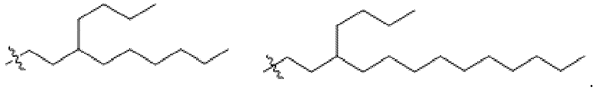

In some embodiments, the present disclosure provides the amino lipid compounds of formulae (II) and (III), or a pharmaceutically acceptable salt thereof or a stereoisomer thereof, as described above, wherein R₄ and R₅ are each independently one of the following structures:

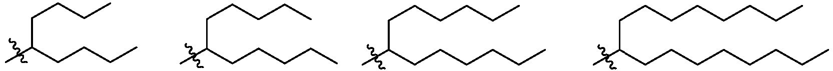

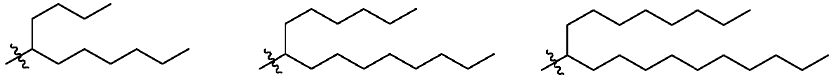

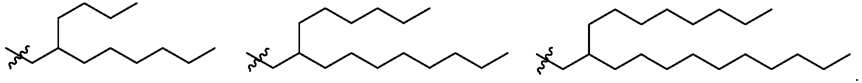

In some embodiments, the present disclosure provides the amino lipid compounds of formulae (II) and (III), or a pharmaceutically acceptable salt thereof or a stereoisomer thereof, as described above, wherein R₄ and R₅ are each independently

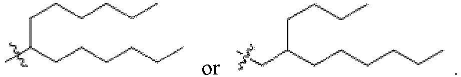

In some embodiments, the present disclosure provides the amino lipid compounds of formulae (II) and (III), or a pharmaceutically acceptable salt thereof or a stereoisomer thereof, as described above, wherein:
Z₇ and Z₈ are each independently -(C=O)O- or -O(C=O)-;
A₃ is straight C₁-C₅ alkylene;
A₄ is straight C₁-C₄ alkylene;
A₆ and A₇ are each independently C₅-C₁₁ alkylene;
R₁ and R₂ are each independently C₁-C₄ alkyl;
R₃ is straight C₂-C₁₀ alkyl; and
R₄ and R₅ are each independently C₁-C₂₄ alkyl.

In some embodiments, each of Z₇ and Z₈ is -(C=O)O-, and the (C=O) in Z₇ is attached to A₆, and the (C=O) in Z₈ is attached to A₇. In some embodiments, each of Z₇ and Z₈ is -O(C=O)-, and the (C=O) in Z₇ is attached to R₄, and the (C=O) in Z₈ is attached to R₅.

In some embodiments, A₃ is straight C₁-C₄ alkylene. In some embodiments, A₃ is straight C₂-C₄ alkylene, such as straight C₂, C₃, or C₄ alkylene. In some embodiments, A₃ is -CH₂CH₂-, -CH₂CH₂CH₂-, or - CH₂CH₂CH₂CH₂-.

In some embodiments, A₄ is straight C₁-C₃ alkylene, such as straight C₁, C₂, or C₃ alkylene. In some embodiments, A₄ is -CH₂-, -CH₂CH₂-, or -CH₂CH₂CH₂-.

In some embodiments, A₆ and A₇ are each independently straight C₅-C₁₁ alkylene. In some embodiments, A₆ and A₇ are each independently straight C₆-C₁₀ alkylene, such as straight C₆, C₇, C₈, C₉, or C₁₀ alkylene. In some embodiments, A₆ and A₇ are each independently straight C₇, C₈, or C₉ alkylene, such as -(CH₂)₇-, - (CH₂)₈- or -(CH₂)₉-.

In some embodiments, R₁ and R₂ are each independently C₁, C₂, C₃, or C₄ alkyl. In some embodiments, R₁ and R₂ are each independently methyl, ethyl, n-propyl, or isopropyl. In some embodiments, R₁ and R₂ are each independently methyl.

In some embodiments, R₃ is straight C₄-C₈ alkyl, such as straight C₄, C₅, C₆, C₇, or C₈ alkyl. In some embodiments, R₃ is n-butyl, n-pentyl, n-hexyl, n-heptyl, or n-octyl.

In some embodiments, R₄ and R₅ are each independently branched C₃-C₂₀ alkyl, such as branched C₅-C₂₀ alkyl, branched C₇-C₁₉ alkyl, branched C₉-C₁₈ alkyl, branched C₁₀-C₁₈ alkyl, branched C₃-C₁₀ alkyl, branched C₁₁-C₁₇ alkyl, or branched C₁₈-C₂₀ alkyl. In some embodiments, R₄ and R₅ are each independently branched C₁₁-C₁₇ alkyl, such as branched C₁₁, C₁₂, C₁₃, C₁₄, C₁₅, C₁₆, or C₁₇ alkyl. In some embodiments, R₄ and R₅ are each independently branched C₁₁-C₁₃ alkyl, such as branched C₁₁, C₁₂, or C₁₃ alkyl. In some embodiments, R₄ is a branched alkyl group wherein branching occurs at the α, β, or γ position relative to Z₇; R₅ is a branched alkyl group wherein branching occurs at the α, β, or γ position relative to Z₈. In some embodiments, R₄ is a branched alkyl group wherein branching occurs at the α or β position relative to Z₇; R₅ is a branched alkyl group wherein branching occurs at the α or β position relative to Z₈.

In some embodiments, R₄ and R₅ are each independently one of the following structures:

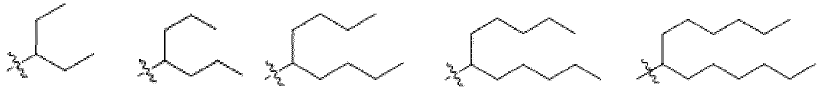

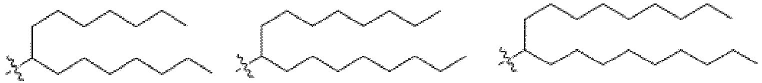

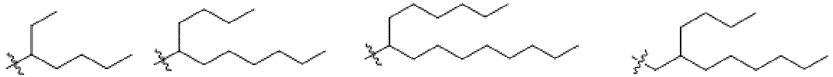

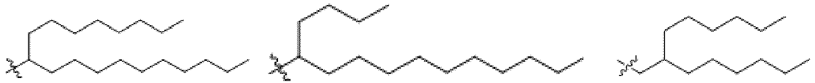

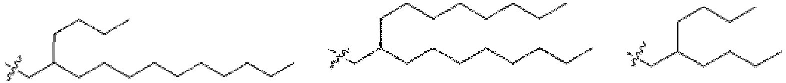

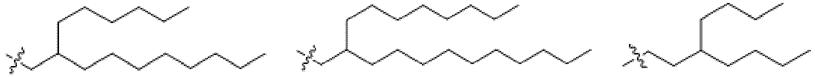

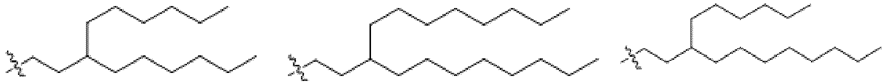

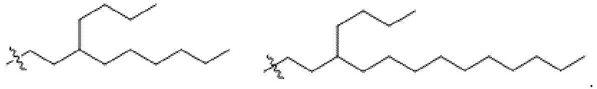

In some embodiments, R₄ and R₅ are each independently one of the following structures:

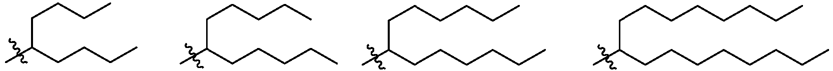

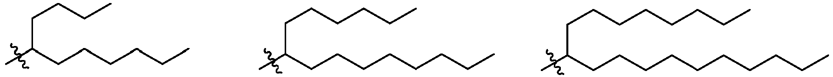

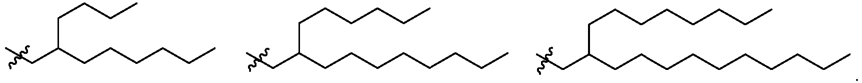

In some embodiments, R₄ and R₅ are each independently 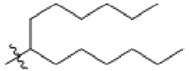 or

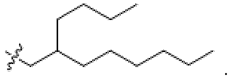

In various embodiments, the present disclosure provides the amino lipid compound of formula (I), (II), or (III), or a pharmaceutically acceptable salt thereof or a stereoisomer thereof, as described above, wherein the amino lipid compound has one of the structures shown in Table 1 below.

**Table 1**

| Amino lipid compounds | Structural formulae | Molecular weight |
|---|---|---|
| 036 | 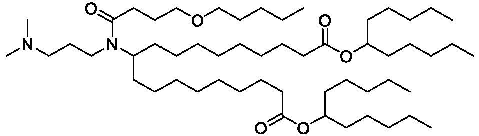 | 893.48 |
| 037 | 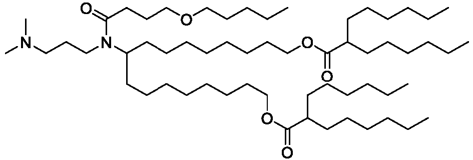 | 949.59 |
| 038 | 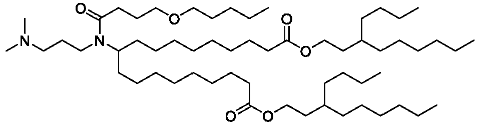 | 949.59 |
| 039 | 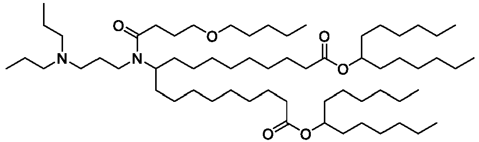 | 1005.69 |
| 040 | 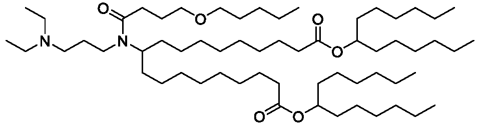 | 977.64 |
| 041 | 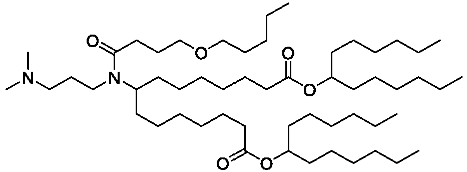 | 893.48 |
| 042 | 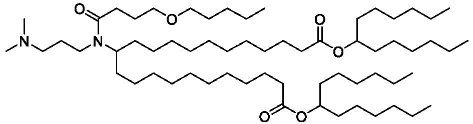 | 1005.69 |
| 044 | 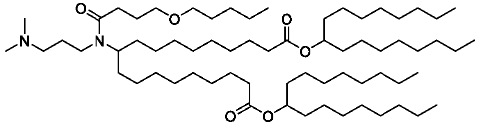 | 1061.80 |
| 045 | 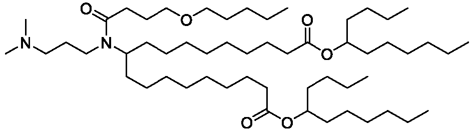 | 893.48 |
| 046 | 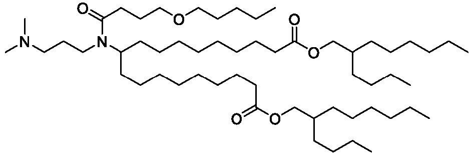 | 921.53 |
| 047 | 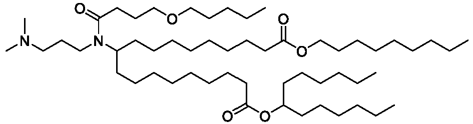 | 893.48 |
| 048 | 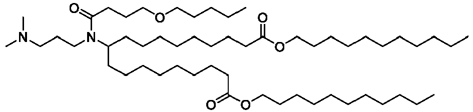 | 893.48 |
| 049 | 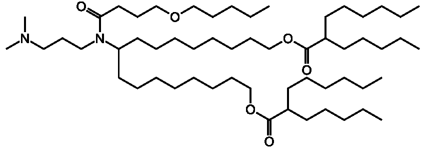 | 921.53 |
| 119 | 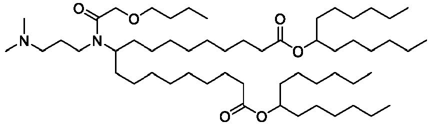 | 907.50 |
| 120 | 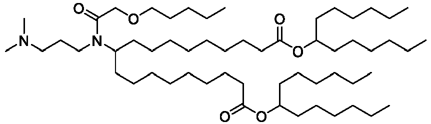 | 921.53 |
| 121 | 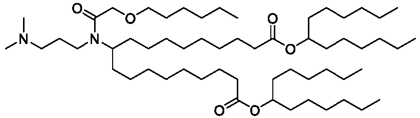 | 935.56 |
| 122 | 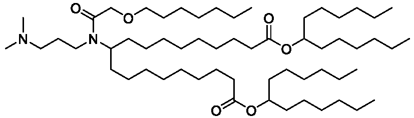 | 949.59 |
| 123 | 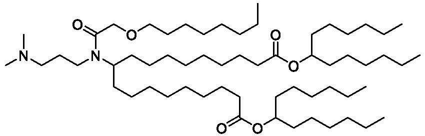 | 963.61 |
| 124 | 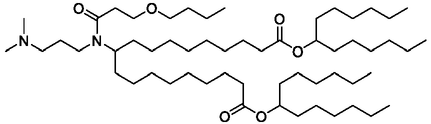 | 921.53 |
| 125 | 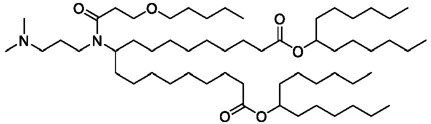 | 935.56 |
| 126 | 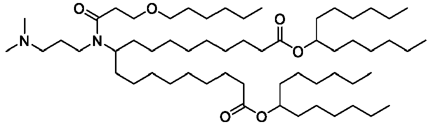 | 949.59 |
| 127 | 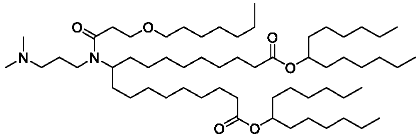 | 963.61 |
| 128 | 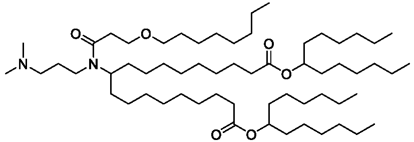 | 977.64 |
| 129 | 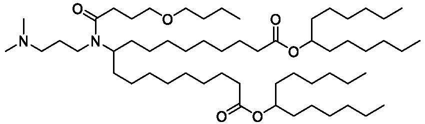 | 935.56 |
| 130 | 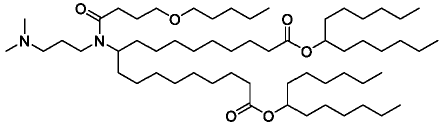 | 949.59 |
| 131 | 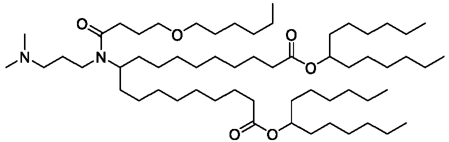 | 963.61 |
| 132 | 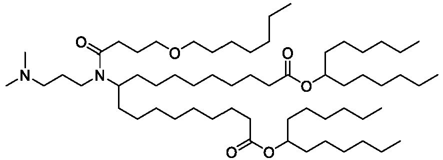 | 977.64 |
| 133 | 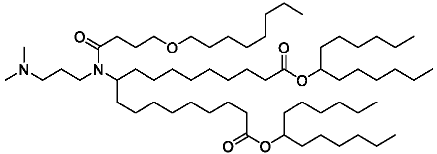 | 991.67 |
| 144 | 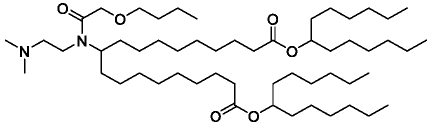 | 893.48 |
| 145 | 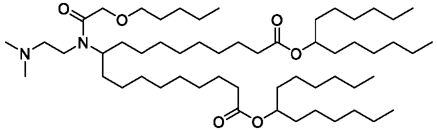 | 907.50 |
| 146 | 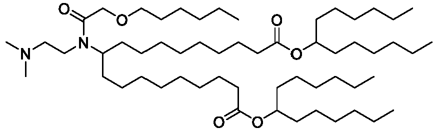 | 921.53 |
| 147 | 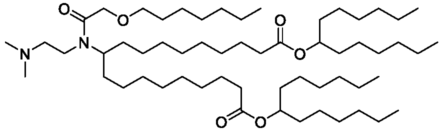 | 935.56 |
| 148 | 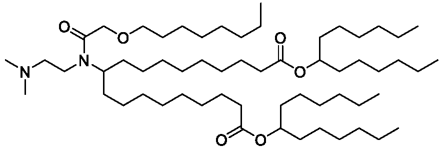 | 949.59 |
| 149 | 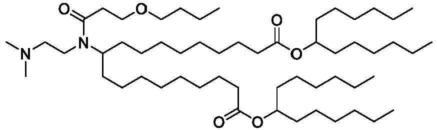 | 907.50 |
| 150 | 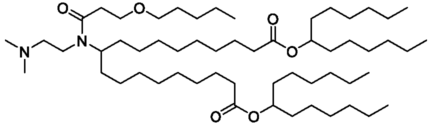 | 921.53 |
| 151 | 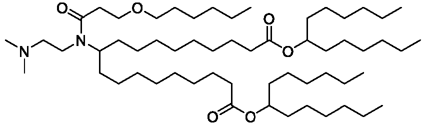 | 935.56 |
| 152 | 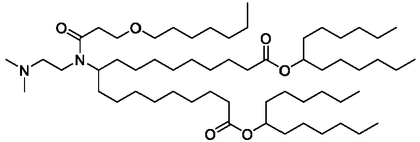 | 949.59 |
| 153 | 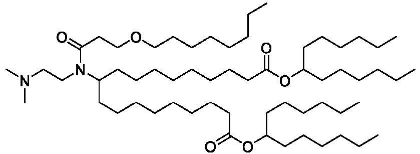 | 963.61 |
| 154 | 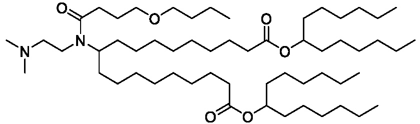 | 921.53 |
| 155 | 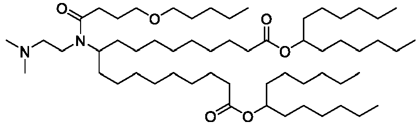 | 935.56 |
| 156 | 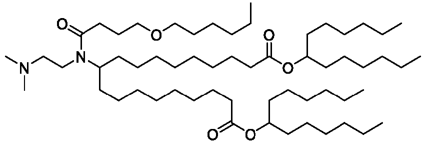 | 949.59 |
| 157 | 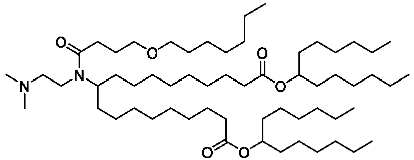 | 963.61 |
| 158 | 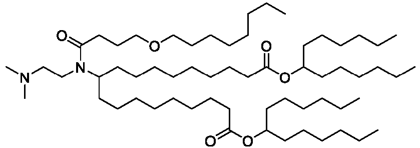 | 977.64 |
| 166 | 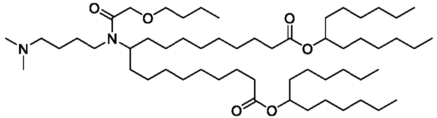 | 921.53 |
| 167 | 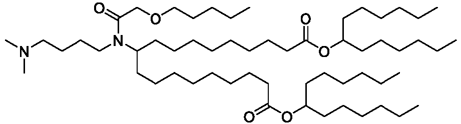 | 935.56 |
| 168 | 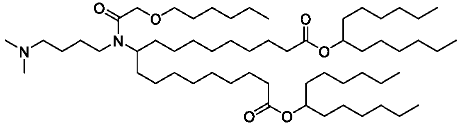 | 949.59 |
| 169 | 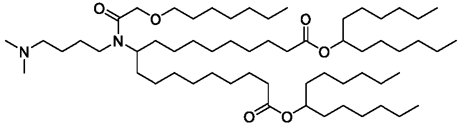 | 963.61 |
| 170 | 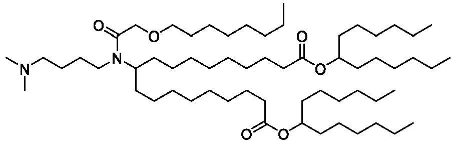 | 977.64 |
| 171 | 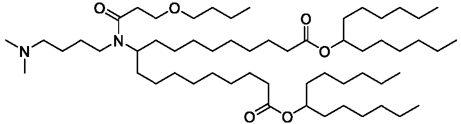 | 935.56 |
| 172 | 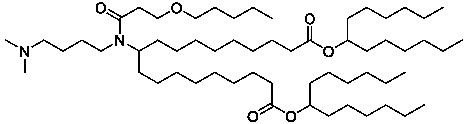 | 949.59 |
| 173 | 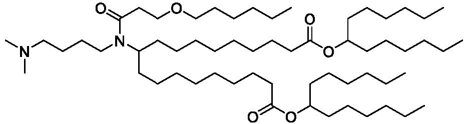 | 963.61 |
| 174 | 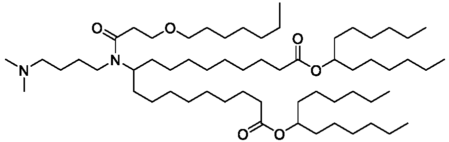 | 977.64 |
| 175 | 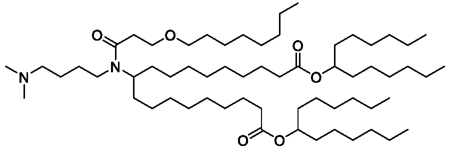 | 991.67 |
| 176 | 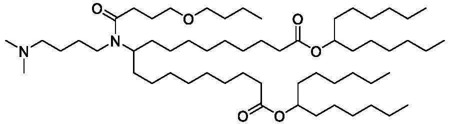 | 949.59 |
| 177 | 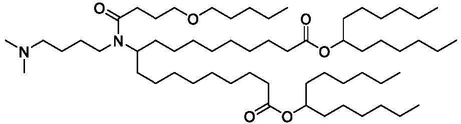 | 963.61 |
| 178 | 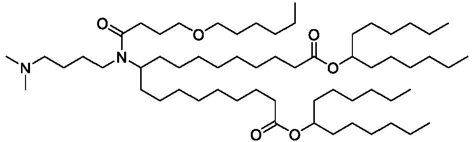 | 977.64 |
| 179 | 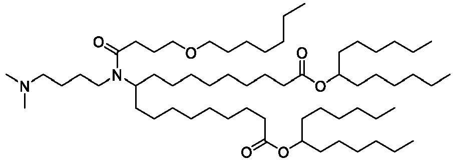 | 991.67 |
| 180 | 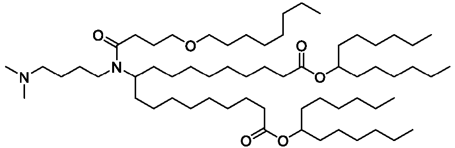 | 1005.69 |

The amino lipid compounds of the present disclosure all have a hydrophobic characteristic due to the presence of long nonpolar residues and simultaneously a hydrophilic characteristic due to the amino group. Due to this amphiphilic characteristic, the amino lipid compounds of the present disclosure can be used to form a lipid nanoparticle, such as a lipid bilayer, a micelle, a liposome, and the like.

In the context of the present disclosure, the term "lipid nanoparticle" means a nanometer-sized material produced by introducing an amino lipid compound into an aqueous solution. The particle is in particular a lipid nanoparticle, a lipid bilayer vesicle (a liposome), a multilayer vesicle or a micelle.

In some preferred embodiments, the lipid nanoparticle is a liposome containing an amino lipid compound of the present disclosure. Within the scope of the present disclosure, a liposome is a microvesicle consisting of a bilayer of lipid amphipathic molecules encapsulating an aqueous compartment.

Liposome formation is not a spontaneous process. When a lipid is introduced into water, a lipid vesicle is firstly formed, thus forming a bilayer or a series of bilayers, each of which is separated by a water molecule. Liposomes can be formed by sonicating lipid vesicles in water.

In the context of the present disclosure, the term "lipid bilayer" means a thin film formed by two layers of lipid molecules. The term "micelle" means an aggregate of surfactant molecules dispersed in a liquid colloid. Typical micelles in an aqueous solution form aggregates with the hydrophilic head region upon contact with water, chelating the hydrophobic single tail region at the center of the micelle.

In one aspect, the present disclosure provides a use of the amino lipid compound of the present disclosure for the manufacture of a vehicle for an active ingredient. In some embodiments, the vehicle is in the form of a lipid nanoparticle, such as a lipid bilayer, micelle, liposome.

### Lipid nanoparticle

In another aspect, the present disclosure provides a lipid nanoparticle containing the amino lipid compound of the present disclosure.

In some embodiments, the lipid nanoparticle further contains a pharmaceutically acceptable carrier, diluent or excipient.

In some embodiments, the lipid nanoparticle further contains one or more of a helper lipid, a structural lipid, and a PEG-lipid (polyethylene glycol-lipid).

In some further embodiments, the lipid nanoparticle further contains the helper lipid, the structural lipid, and the PEG-lipid.

In some embodiments, the lipid nanoparticle comprises the amino lipid compound in an amount (molar percent) of about 25.0% to 75.0%, such as about 25.0%-28.0%, 28.0%-32.0%, 32.0%-35.0%, 35.0%-40.0%, 40.0%-42.0%, 42.0%-45.0%, 45.0%-48.0%, 48.0%-55.0%, 55.0%-65.0%, 65.0%-75.0%, 45.0%-46.3%, 46.3%-48.0%, 48.0%-49.5%, 49.5%-50.0%, 50.0%-55.0%, or 60.0%-65.0%, based on the total amount of the amino lipid compound, the helper lipid, the structural lipid, and the PEG-lipid.

In some embodiments, the lipid nanoparticle comprises the helper lipid in an amount (molar percent) of about 5.0% to 45.0%, such as about 5.0%-10.0%, 10.0%-16.0%, 16.0%-25.0%, 25.0%-33.5%, 33.5%-37.0%, 37.0%-40.0%, 40.0%-42.0%, 42.0%-45.0%, 5.0%-9.0%, 9.0%-9.4%, 9.4%-10.0%, 10.0%-10.5%, 10.5%-11.0%, 11.0%-15.0%, 15.0%-16.0%, 16.0%-18.0%, 18.0%-20.0%, or 20.0%-25.0%, based on the total amount of the amino lipid compound, the helper lipid, the structural lipid, and the PEG-lipid.

In some embodiments, the lipid nanoparticle comprises the structural lipid in an amount (molar percent) of about 0.0% to 50.0%, such as about 0.0%-10.0%, 10.0%-15.5%, 15.5%-22.5%, 22.5%-35.0%, 35.0%-36.5%, 36.5%-39.5%, 39.5%-40.5%, 40.5%-41.5%, 41.5%-45.0%, 45.0%-46.5%, 46.5%-50.0%, 15.5%-18.5%, 18.5%-22.5%, 22.5%-23.5%, 23.5%-28.5%, 28.5%-33.5%, 33.5%-35.0%, 36.5%-38.0%, 38.0%-38.5%, 38.5%-39.0%, 39.0%-39.5%, 41.5%-42.5%, 42.5%-42.7%, 42.7%-43.0%, 43.0%-43.5%, 43.5%-45.0%, or 46.5%-48.5%, based on the total amount of the amino lipid compound, the helper lipid, the structural lipid, and the PEG-lipid. In some embodiments, the lipid nanoparticle comprises the structural lipid in an amount (molar percent) of about 50.0%-55.0%, such as about 50.0%-51.5%, 51.5%-53.5%, or 53.5%-55.0%.

In some embodiments, the lipid nanoparticle comprises the PEG-lipid in an amount (molar percent) of about 0.5% to 5.0%, such as about 0.5%-1.0%, 1.0%-1.5%, 1.5%-2.0%, 2.0%-2.5%, 2.5%-3.0%, 3.0%-3.5%, 3.5%-4.0%, 4.0%-4.5%, 4.5%-5.0%, 1.5%-1.6%, or 1.6%-2.0%, based on the total amount of the amino lipid compound, the helper lipid, the structural lipid, and the PEG-lipid.

In some embodiments as described above, the helper lipid is a phospholipid. The phospholipid is generally semi-synthetic and may also be of natural origin or chemically modified. The phospholipid includes, but is not limited to, DSPC (distearoyl phosphatidylcholine), DOPE (dioleoyl phosphatidylethanolamine), DOPC (dioleoyl lecithin), DOPS (dioleoyl phosphatidylserine), DSPG (1,2-distearoyl-sn-glycero-3-phospho-(1'-rac-glycerol)), DPPG (dipalmitoyl phosphatidylglycerol), DPPC (dipalmitoyl phosphatidylcholine), DGTS (1,2-dipalmitoyl-sn-glycero-3-O-4'-(N,N,N-trimethyl) homoserine), lysophospholipid, and the like. Preferably, the helper lipid is one or more selected from the group consisting of DSPC, DOPE, DOPC, and DOPS. In some embodiments, the helper lipid is DSPC and/or DOPE.

In some embodiments, the structural lipid is a sterol, including but not limited to, cholesterol, cholesterol esters, steroid hormones, steroid vitamins, bile acid, cholesterin, ergosterol, β-sitosterol, oxidized cholesterol derivatives, and the like. Preferably, the structural lipid is at least one selected from cholesterol, cholesteryl esters, steroid hormones, steroid vitamins, and bile acid. In some embodiments, the structural lipid is cholesterol, preferably high purity cholesterol, particularly injection grade high purity cholesterol, such as CHO-HP (manufactured by AVT).

As used herein, the term PEG-lipid (polyethylene glycol-lipid) is a conjugate of polyethylene glycol and a lipid structure. Preferably, the PEG-lipid is selected from PEG-DMG and PEG-distearoyl phosphatidylethanolamine (PEG-DSPE), preferably PEG-DMG. Preferably, the PEG-DMG is a polyethylene glycol (PEG) derivative of 1,2-dimyristoyl-sn-glycerol. Preferably, the PEG has an average molecular weight of about 2,000 to 5,000, preferably about 2,000, such as PEG2000-DMG.

In some embodiments as described above, in the lipid nanoparticle, the molar ratio of the amino lipid compound of the present disclosure: helper lipid: structural lipid: PEG-lipid is about 45:11:41.5:2.5, or 42.0:10.5:45.0:2.5, or 42.0:16.0:39.5:2.5, or 40.0:16.0:41.5:2.5, or 40.0:18.0:39.5:2.5, or 35.0:16.0:46.5:2.5, or 35.0:25.0:36.5:3.5, or 28.0:33.5:35.0:3.5, or 32.0:37.0:40.5:0.5, or 35.0:40.0:22.5:2.5, or 40.0:42.0:15.5:2.5, or 45:10:42.5:2.5, or 40.0:20.0:38.5:1.5, or 45.0:15.0:38.5:1.5, or 55.0:5.0:38.5:1.5, or 60.0:5.0:33.5:1.5, or 45.0:20.0:33.5:1.5, or 50.0:20.0:28.5:1.5, or 55.0:20.0:23.5:1.5, or 60.0:20.0:18.5:1.5, or 40.0:15.0:43.5:1.5, or 50.0:15.0:33.5:1.5, or 55.0:15.0:28.5:1.5, or 60.0:15.0:23.5:1.5, or 40.0:10.0:48.5:1.5, or 45.0:10.0:43.5:1.5, or 55.0:10.0:33.5:1.5, or 40.0:5.0:53.5:1.5, or 45.0:5.0:48.5:1.5, or 50.0:5.0:43.5:1.5. In some such embodiments, the helper lipid is DOPE, and the structural lipid is CHO-HP.

In other embodiments as described above, in the lipid nanoparticle, the molar ratio of the amino lipid compound of the present disclosure: helper lipid: structural lipid: PEG-lipid is about 48.0 : 10.0 : 40.5 : 1.5, or 50.0: 10.0: 38.5: 1.5, or 50.0 : 9.0 : 38.0 : 3.0, or 49.5 : 10.0 : 39.0 : 1.5, or 46.3 : 9.4 : 42.7 : 1.6, or 45.0 : 9.0 : 43.0 : 3.0, or 45.0:11.0:41.5:2.5, or 42.0:10.5:45.0:2.5, or 42.0:16.0:39.5:2.5, or 40.0:16.0:41.5:2.5, or 40.0:18.0:39.5:2.5, or 35.0:40.0:22.5:2.5, or 40.0:20.0:38.5:1.5, or 45.0:15.0:38.5:1.5, or 55.0:5.0:38.5:1.5, or 60.0:5.0:33.5:1.5, or 45.0:20.0:33.5:1.5, or 50.0:20.0:28.5:1.5, or 55.0:20.0:23.5:1.5, or 60.0:20.0:18.5:1.5, or 40.0:15.0:43.5:1.5, or 50.0:15.0:33.5:1.5, or 55.0:15.0:28.5:1.5, or 60.0:15.0:23.5:1.5, or 40.0:10.0:48.5:1.5, or 45.0:10.0:43.5:1.5, or 55.0:10.0:33.5:1.5, or 40.0:5.0:53.5:1.5, or 45.0:5.0:48.5:1.5, or 50.0:5.0:43.5:1.5. In some such embodiments, the helper lipid is DSPC, and the structural lipid is CHO-HP.

In some embodiments, the lipid nanoparticle has the amino lipid compound of the present disclosure, helper lipid, structural lipid, and PEG-lipid in molar percent (%) as shown in Nos. 1-12, Nos. 13-24 in Table 2 below, based on the total amount of the amino lipid compound, the helper lipid, the structural lipid, and the PEG-lipid:

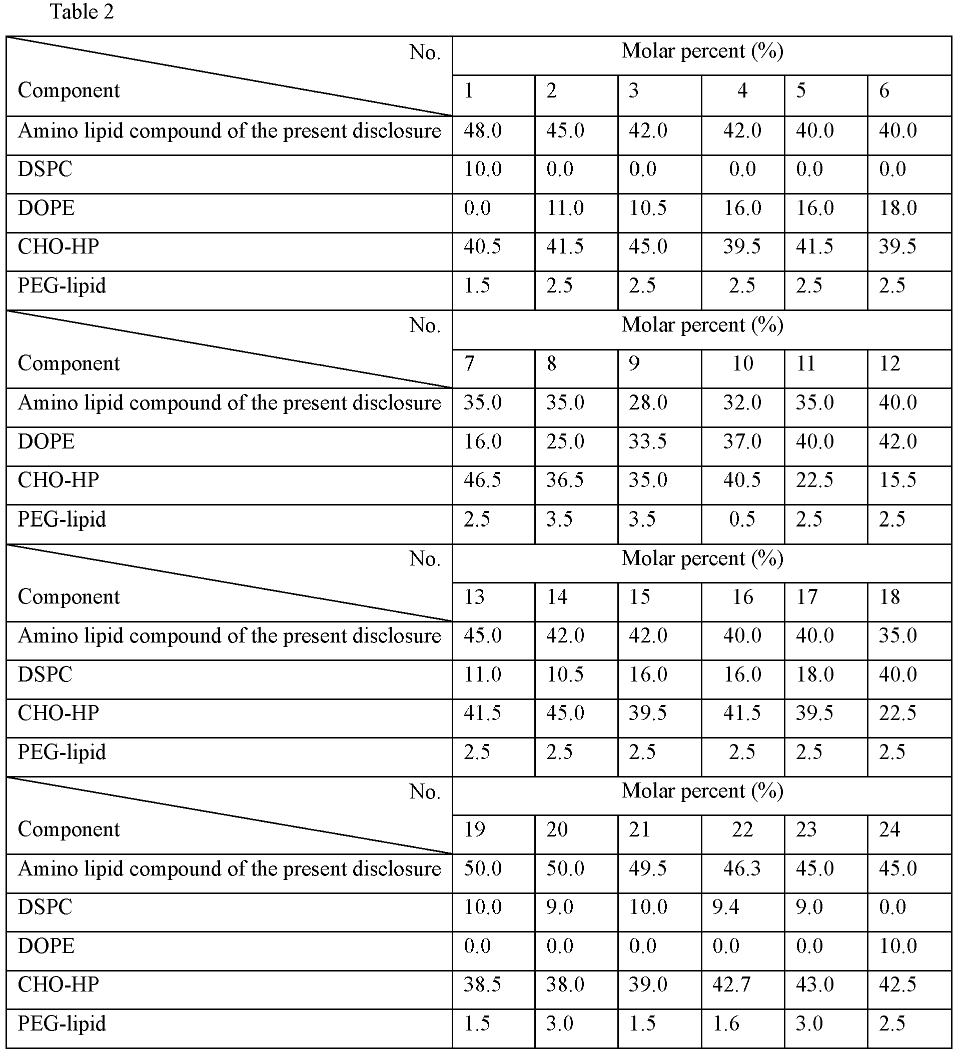

In some embodiments, the lipid nanoparticle has the amino lipid compound of the present disclosure, helper lipid, structural lipid, and PEG-lipid in molar percent (%) as shown in Nos. 25-42 in Table 3 below, based on the total amount of the amino lipid compound, the helper lipid, the structural lipid, and the PEG-lipid:

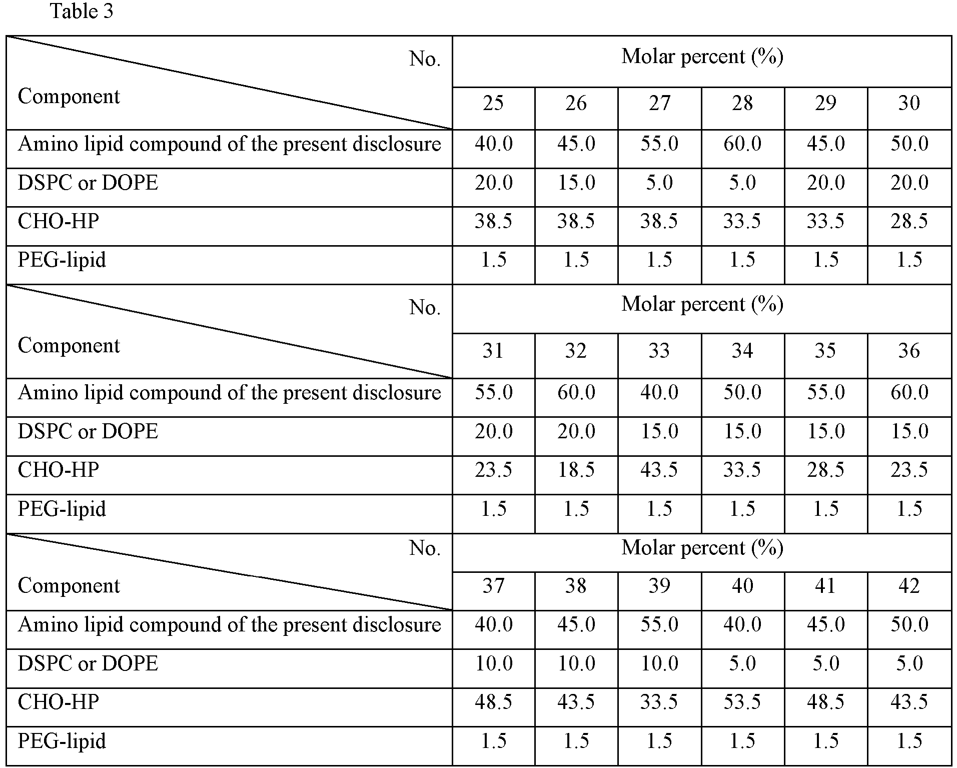

As described above, the lipid nanoparticle of the present disclosure may be used as a vehicle for an active ingredient.

In some embodiments, the active ingredient includes a therapeutic and/or a prophylactic agent.

The term "therapeutic agent" or "prophylactic agent" refers to any agent that, when administered to a subject, has therapeutic, diagnostic, and/or prophylactic effects and/or elicits desired biological and/or pharmacological effects.

An "effective amount" or "therapeutically effective amount" refers to an amount of the amino lipid compound of the present disclosure or the lipid nanoparticle comprising the amino lipid compound of the present disclosure that is sufficient to effect treatment in a mammal (preferably a human), when administered to the mammal (preferably the human). The amount of the lipid nanoparticle of the present disclosure that constitutes a "therapeutically effective amount" will depend on the amino lipid compound, the condition and its severity, the mode of administration, and the age of the mammal to be treated, but may be routinely determined by one of ordinary skill in the art in light of their own knowledge and the present disclosure.

In some embodiments, the active ingredient is a pharmaceutically active ingredient, including, but not limited to, an antineoplastic agent, an antibiotic, an immunomodulator, an anti-inflammatory agent, an agent acting on the central nervous system, an antigen or a fragment thereof, a protein, a peptide, a polypeptide, a polypeptoid, a vaccine and a small molecule, and a mixture thereof. Preferably, the pharmaceutically active ingredient is a biologically active ingredient.

In the context of the present disclosure, a biologically active ingredient is a substance that has a biological effect when introduced into a cell or a host, for example, by stimulating an immune or inflammatory response, by exerting an enzymatic activity, by complementing a mutation, or the like. The biologically active ingredient includes, but is not limited to, a nucleic acid, an antigen or a fragment thereof, a protein, a peptide, a polypeptide, a polypeptoid, an antibody, a vaccine and a small molecule, and a mixture thereof.

A lipid nanoparticle may be referred to as "a lipid nanoparticle drug" when it encapsulates the active ingredient in its internal aqueous space.

In the context of the present disclosure, the term "cell" is a generic term and includes the culture of individual cells, tissues, organs, insect cells, avian cells, fish cells, amphibian cells, mammalian cells, primary cells, continuous cell lines, stem cells, and/or genetically engineered cells (e.g., recombinant cells expressing a heterologous polypeptide or protein). Recombinant cells include, for example, cells expressing heterologous polypeptides or proteins (such as growth factors or blood factors).

In some preferred embodiments, the biologically active ingredient is a nucleic acid.

In some embodiments as described above, the lipid nanoparticle of the present disclosure further comprises a nucleic acid.

In some embodiments, the mass ratio of the amino lipid compound of the present disclosure to the nucleic acid in the lipid nanoparticle is about (5-30): 1, such as about (5-10): 1, (10-15): 1, (15-20): 1, (20-25): 1, or (25-30): 1, preferably about 10:1.

In some embodiments, the nucleic acid is selected from the group consisting of RNA, antisense oligonucleotide, and DNA.

In some embodiments, the RNA is selected from the group consisting of messenger RNA (mRNA), ribosomal RNA (rRNA), microRNA (miRNA), transfer RNA (tRNA), small interfering RNA (siRNA), small nuclear RNA (snRNA), small hairpin RNA (shRNA), single guide RNA (sgRNA), Cas9 mRNA or a mixture thereof.

In some embodiments, the messenger RNA (mRNA) encodes a polypeptide and/or protein of interest. Any naturally or non-naturally occurring or otherwise modified polypeptide is included. In some embodiments, the polypeptide and/or protein encoded by the mRNA may have therapeutic and/or prophylactic effects when expressed in a cell.

In some embodiments, the RNA is an siRNA that is capable of selectively decreasing the expression of a gene of interest or down-regulating the expression of the gene. For example, an siRNA may be selected such that a gene associated with a particular disease, disorder, or condition is silenced upon administration of a lipid nanoparticle comprising the siRNA to a subject in need thereof. An siRNA may comprise a sequence complementary to an mRNA sequence encoding a gene or protein of interest. In some embodiments, the siRNA may be an immunomodulatory siRNA.

In certain embodiments, the RNA is sgRNA and/or cas9 mRNA. The sgRNA and/or cas9 mRNA may be used as a gene editing tool. For example, the sgRNA-Cas9 complex can affect mRNA translation of cellular genes.

In some embodiments, the RNA is an shRNA or a vector or plasmid encoding the same. The shRNA may be produced inside the target cell after delivery of an appropriate construct into the nucleus. Constructs and mechanisms associated with shRNA are well known in the relevant art.

In some embodiments, the DNA is a plasmid.

In some embodiments, the lipid nanoparticle is used to transfer nucleic acids. In some embodiments, the lipid nanoparticle may be used for, for example, gene therapy, gene vaccination, protein replacement therapy, antisense therapy, or therapy by interfering RNA.

### Composition

In another aspect, the present disclosure provides a composition comprising a lipid nanoparticle as described above and a pharmaceutically acceptable carrier, diluent, or excipient.

In some embodiments, the composition further comprises a buffer solution. In some such embodiments, the buffer solution is selected from a phosphate buffer and a Tris buffer, preferably a phosphate buffer. In some embodiments, the buffer solution has a concentration of about 5 mmol/L to about 30 mmol/L, preferably about 10 mmol/L. In some embodiments, the buffer solution has a pH of about 6 to 8, preferably about 7 to 8, and more preferably about 7 to 7.5.

In some embodiments, the composition further comprises a cryoprotectant. In some such embodiments, the cryoprotectant is selected from sucrose and trehalose, preferably sucrose. In some embodiments, the cryoprotectant has a concentration of about 50 mg/ml to 100 mg/ml.

In some embodiments as described above, the composition further comprises a cryoprotectant. In some such embodiments, the cryoprotectant is selected from sucrose and trehalose, preferably sucrose. In some embodiments, the cryoprotectant has a concentration of about 50 mg/ml to 100 mg/ml.

In some embodiments, the composition is a pharmaceutical composition.

### Use

The lipid nanoparticle of the present disclosure has excellent properties of encapsulating biologically active ingredients. The lipid nanoparticle comprising biologically active ingredients can be used to transfer any of a variety of therapeutic agents into cells. The present disclosure includes a use of the lipid nanoparticle as described above to transfer a biologically active ingredient into a cell. The present disclosure also provides a method of transferring a biologically active ingredient into a cell, comprising contacting the lipid nanoparticle of the present disclosure comprising the biologically active ingredient with the cell.

The lipid nanoparticle of the present disclosure can be used to transfect multicellular tissues or organs. Accordingly, the present disclosure also provides a method for transfecting a cell, a multicellular tissue, or an organ, comprising contacting the lipid nanoparticle of the present disclosure comprising the nucleic acid with the cell, tissue, or organ. This provides a subject with the possibility of new therapeutic treatment.

In some embodiments, the cell is a mammalian cell; and further preferably, the mammalian cell is in a mammal.

In some embodiments, the tissue or organ is selected from the group consisting of spleen, liver, kidney, lung, femur, ocular tissue, vascular endothelium in blood vessels, lymph, and tumor tissue.

As used herein, a subject may be any mammal, preferably selected from the group consisting of mice, rats, pigs, cats, dogs, horses, goats, cattle, and monkeys, etc. In some preferred embodiments, the subject is a human.

In another aspect, The present disclosure provides a method of producing a polypeptide and/or protein of interest in a mammalian cell, comprising contacting the cell with the lipid nanoparticle comprising mRNA encoding the polypeptide and/or protein of interest, upon contact of the cell with the lipid nanoparticle, the mRNA being able to be taken up into the cell and translated to produce the polypeptide and/or protein of interest.

In yet another aspect, the present disclosure provides the use of the amino lipid compound, lipid nanoparticle, or composition of the present disclosure in the manufacture of a medicament. In yet another aspect, the present disclosure provides a lipid nanoparticle or composition of the present disclosure for use as a medicament. In yet another aspect, the present disclosure provides a medicament comprising the lipid nanoparticle or composition of the present disclosure. In some embodiments, the medicament is used for treatment and/or prevention of a disease. In some embodiments, the disease is selected from the group consisting of rare diseases, infectious diseases, cancers, genetic diseases, autoimmune diseases, diabetes, neurodegenerative diseases, cardiovascular, renal vascular diseases, and metabolic diseases. In some embodiments, the medicaments are used for, for example, gene therapy, gene vaccination, protein replacement therapy, antisense therapy, or therapy by interfering RNA. Preferably, the gene therapy is for use in the treatment of cancers and genetic diseases. In some embodiments, the cancers are selected from one or more of lung cancer, stomach cancer, liver cancer, esophageal cancer, colon cancer, pancreatic cancer, brain cancer, lymphoma, blood cancer, or prostate cancer. In some embodiments, the genetic disorders are one or more selected from hemophilia, thalassemia, and Gaucher's disease. In some embodiments, the gene vaccine is preferably for vaccination for treating cancer, allergy, toxicity, and pathogen infection. In some embodiments, the pathogen is selected from one or more of viruses, bacteria, or fungi.

In yet another aspect, the present disclosure provides a method of treating a disease or disorder in a mammal in need thereof, comprising administering to the mammal a therapeutically effective amount of the lipid nanoparticle as described above.

Preferably, the disease or disorder is selected from the group consisting of rare diseases, infectious diseases, cancers, genetic diseases, autoimmune diseases, diabetes, neurodegenerative diseases, cardiovascular, renal vascular diseases, and metabolic diseases.

In yet another aspect, the present disclosure provides a use of the amino lipid compound, lipid nanoparticle, or composition of the present disclosure in the manufacture of a medicament for nucleic acid transfer. In yet another aspect, the present disclosure provides the lipid nanoparticle or composition of the present disclosure for use as a medicament for nucleic acid transfer. In yet another aspect, the present disclosure provides a medicament comprising the lipid nanoparticle or composition of the present disclosure for nucleic acid transfer. In some embodiments, the nucleic acid is selected from the group consisting of RNA, antisense oligonucleotide, and DNA. In some embodiments, the RNA is selected from the group consisting of messenger RNA (mRNA), ribosomal RNA (rRNA), microRNA (miRNA), transfer RNA (tRNA), small interfering RNA (siRNA), and small nuclear RNA (snRNA). In some embodiments, the DNA is a plasmid.

### Preparation method

In another aspect, the present disclosure also provides a general synthetic process for preparing the amino lipid compound of formula (II) of the present disclosure, as follows: 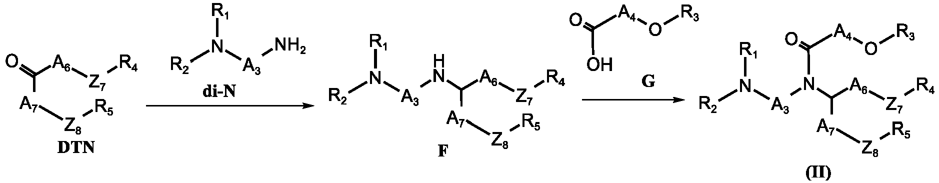 wherein A₃, A₄, A₆, A₇, R₁, R₂, R₃, R₄, R₅, Z₇ and Z₈ each have the same meanings as defined above for the amino lipid compound of formula (I) or (II).

Specifically, the method comprises:
Step 1: subjecting a compound of formula DTN and a compound of formula di-N to a reductive amination reaction to obtain a compound of formula F; and
Step 2: reacting the compound of formula F with a compound of formula G to obtain the amino lipid compound of formula (II).

In some embodiments, the reductive amination reaction in Step 1 is carried out in an organic solvent (e.g., C₁-C₃ alcohol, preferably ethanol). In some embodiments, the reductive amination reaction is carried out in the presence of a catalyst (e.g., palladium on carbon) under a hydrogen atmosphere. In some embodiments, the reductive amination reaction is carried out at an elevated pressure (e.g., about 2-8 or about 4-6 MPa) and an elevated temperature (e.g., about 50-90 or about 60-80°C).

In some embodiments, the reaction in Step 2 is carried out in the presence of a base (e.g., pyridine or DMAP).

In yet another aspect, the present disclosure also provides a general synthetic process for preparing the amino lipid compound of formula (III) of the present disclosure, as follows: 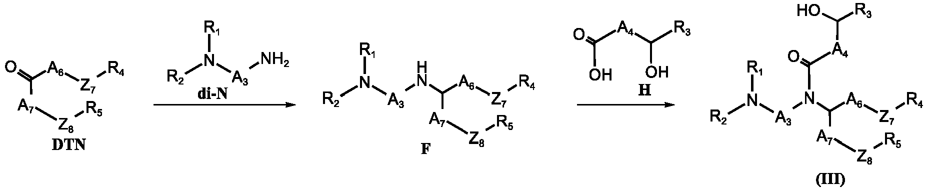 wherein A₃, A₄, A₆, A₇, R₁, R₂, R₃, R₄, R₅, Z₇ and Z₈ each have the same meanings as defined above for the compound of formula (I) or (III).

Specifically, the method comprises:
Step 1: subjecting a compound of formula DTN and a compound of formula di-N to a reductive amination reaction to obtain a compound of formula F; and
Step 2: reacting the compound of formula F with a compound of formula H to obtain the amino lipid compound of formula (III).

In some embodiments, the reductive amination reaction in Step 1 is carried out in an organic solvent (e.g., C₁-C₅ alcohol, preferably ethanol). In some embodiments, the reductive amination reaction is carried out in the presence of a catalyst (e.g., palladium on carbon) under a hydrogen atmosphere. In some embodiments, the reductive amination reaction is carried out at an elevated pressure (e.g., about 2-8 or about 4-6 MPa) and an elevated temperature (e.g., about 50-90 or about 60-80°C).

In yet another aspect, the lipid nanoparticle or composition of the present disclosure may be prepared according to methods known in the art. For example, the method may comprise the following steps:
(1) formulating: formulating a suitable aqueous phase; and formulating an organic phase comprising the amino lipid compound of the present disclosure and optionally a helper lipid, a structural lipid, and/or a PEG-lipid;
(2) encapsulation: mixing a suitable amount of the aqueous phase with the organic phase;
(3) dialysis: optionally dialyzing the mixture of step (2); and
(4) sterilization: optionally sterilizing the product of step (3), for example, by means of a sterilizing filter, such as a 0.22 µm microporous membrane.

In yet another aspect, the lipid nanoparticle or composition of the present disclosure comprising a nucleic acid, particularly mRNA may be prepared by a method comprising the following steps:
(1) formulating: formulating an aqueous phase comprising the nucleic acid; and formulating an organic phase (e.g., an ethanol phase) comprising the amino lipid compound of the present disclosure and optionally a helper lipid, a structural lipid, and/or a PEG-lipid;
(2) encapsulation: mixing a suitable amount of the aqueous phase with the organic phase;
(3) dialysis: optionally dialyzing the mixture of step (2);
(4) sterilization: optionally sterilizing the product of step (3), for example, by means of a sterilizing filter, such as a 0.22 µm microporous membrane.

The present disclosure also includes the following embodiments:
Embodiment 1. An amino lipid compound having a structure of formula (I): 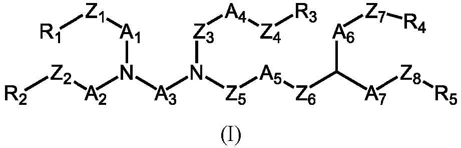
   or a pharmaceutically acceptable salt or stereoisomer thereof,
   wherein:
      Z₁, Z₂, Z₅, Z₆, Z₇ and Z₈ are each independently -C(=O)-, -CH(OH)-, -C=C-, -C≡C-, -O-, -(C=O)O-, - O(C=O)-, -C(=O)S-, -SC(=O)-, -S-S-, or a bond;
      Z₃ is -C(=O)- or a bond;
      Z₄ is -O-, or -CH(OH)-;
      A₁, A₂, A₃, A₄, A₅, A₆ and A₇ are each independently C₁-C₁₂ hydrocarbylene, cyclohydrocarbyl, phenyl, heterocycle, or a bond;
      R₁ and R₂ are each independently H or C₁-C₁₈ hydrocarbyl, or cyclohydrocarbyl, phenyl, heterocycle; or R₁ and R₂, together with the nitrogen atom to which they are attached, form 4- to 7-membered heterocycle;
      R₃ is H or C₁-C₁₈ hydrocarbyl, or cyclohydrocarbyl, phenyl, heterocycle;
      R₄ and R₅ are each independently C₁-C₂₄ hydrocarbyl, or cyclohydrocarbyl, phenyl, heterocycle;
      preferably, A₅, Z₅ and Z₆ are bonds.
Embodiment 2. The amino lipid compound according to Embodiment 1, wherein Z₇ and Z₈ are each independently -(C=O)O-, -O(C=O)-, or a bond.
Embodiment 3. The amino lipid compound according to Embodiment 1, wherein one of Z₇ or Z₈ is - (C=O)O-, -O(C=O)-, or a bond.
Embodiment 4. The amino lipid compound according to Embodiment 1, wherein each of Z₇ and Z₈ is - (C=O)O-.
Embodiment 5. The amino lipid compound according to Embodiment 1, wherein one of Z₇ or Z₈ is a bond.
Embodiment 6. The amino lipid compound according to Embodiment 1, wherein each of Z₇ and Z₈ is a bond.
Embodiment 7. The amino lipid compound according to Embodiment 1, wherein R₄ and R₅ are each independently C₁-C₂₀ hydrocarbyl.
Embodiment 8. The amino lipid compound according to Embodiment 1, wherein R₄ and R₅ are each independently C₁-C₂₀ alkyl.
Embodiment 9. The amino lipid compound according to Embodiment 1, wherein R₄ and R₅ are each independently branched C₃-C₂₀ alkyl.
Embodiment 10. The amino lipid compound according to Embodiment 1, wherein R₄ and R₅ are each independently: 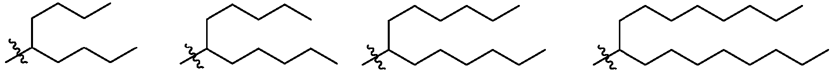 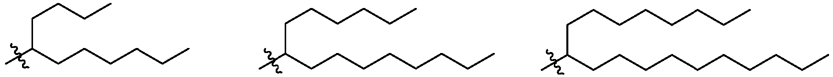 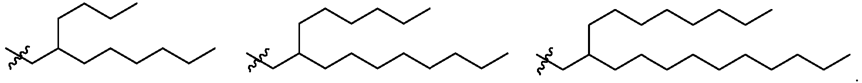
Embodiment 11. An amino lipid compound having a structure of formula (II): 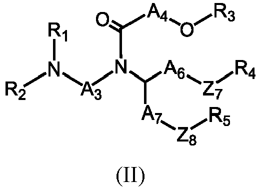
   or a pharmaceutically acceptable salt or stereoisomer thereof,
   wherein:
      Z₇ and Z₈ are each independently -C(=O)-, -CH(OH)-, -C=C-, -C≡C-, -O-, -(C=O)O-, -O(C=O)-, - C(=O)S-, -SC(=O)-, -S-S-, or a bond;
      A₃, A₄, A₆ and A₇ are each independently C₁-C₁₂ hydrocarbylene, cyclohydrocarbyl, phenyl, heterocycle, or a bond;
      R₁ and R₂ are each independently H or C₁-C₁₈ hydrocarbyl, or cyclohydrocarbyl, phenyl, heterocycle; or R₁ and R₂, together with the nitrogen atom to which they are attached, form 4- to 7-membered heterocycle;
      R₃ is H or C₁-C₁₈ hydrocarbyl, or cyclohydrocarbyl, phenyl, heterocycle;
      R₄ and R₅ are each independently C₁-C₂₄ hydrocarbyl, or cyclohydrocarbyl, phenyl, heterocycle.
Embodiment 12. The amino lipid compound according to Embodiment 11, wherein Z₇ and Z₈ are each independently -(C=O)O-, or -O(C=O)-.
Embodiment 13. The amino lipid compound according to Embodiment 11, wherein A₃, A₄, A₆ and A₇ are each independently C₁-C₁₂ alkylene.
Embodiment 14. The amino lipid compound according to Embodiment 11, wherein R₁ and R₂ are each independently C₁-C₁₈ alkyl.
Embodiment 15. The amino lipid compound according to Embodiment 14, wherein R₁ and R₂ are each independently C₁-C₆ alkyl.
Embodiment 16. The amino lipid compound according to Embodiment 14, wherein R₁ and R₂ are each independently methyl, ethyl, n-propyl, or isopropyl.
Embodiment 17. The amino lipid compound according to Embodiment 11, wherein R₁ and R₂, together with the nitrogen atom to which they are attached, form 4- to 7-membered heterocycle.
Embodiment 18. The amino lipid compound according to Embodiment 17, wherein R₁ and R₂, together with the nitrogen atom to which they are attached, form 5- to 6-membered heterocycle.
Embodiment 19. The amino lipid compound according to Embodiment 11, wherein R₃ is C₁-C₁₈ alkyl.
Embodiment 20. The amino lipid compound according to Embodiment 11, wherein R₄ and R₅ are each independently C₁-C₂₀ hydrocarbyl.
Embodiment 21. The amino lipid compound according to Embodiment 11, wherein R₄ and R₅ are each independently C₁-C₂₀ alkyl.
Embodiment 22. The amino lipid compound according to Embodiment 11, wherein R₄ and R₅ are each independently branched C₃-C₁₈ alkyl.
Embodiment 23. The amino lipid compound according to Embodiment 11, wherein R₄ and R₅ are each independently: 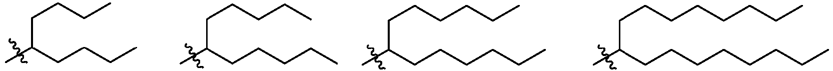 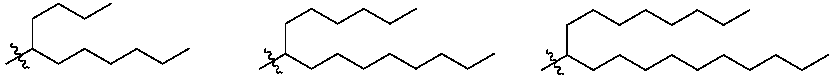 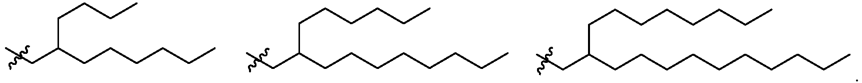
Embodiment 24. An amino lipid compound having a structure of formula (III): 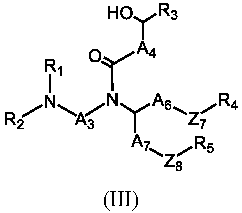
   or a pharmaceutically acceptable salt or stereoisomer thereof,
   wherein:
      Z₇ and Z₈ are each independently -C(=O)-, -CH(OH)-, -C=C-, -C≡C-, -O-, -(C=O)O-, -O(C=O)-, - C(=O)S-, -SC(=O)-, -S-S-, or a bond;
      A₃, A₄, A₆ and A₇ are each independently C₁-C₁₂ hydrocarbylene, cyclohydrocarbyl, phenyl, heterocycle, or a bond;
      R₁ and R₂ are each independently H or C₁-C₁₈ hydrocarbyl, or cyclohydrocarbyl, phenyl, heterocycle; or R₁ and R₂, together with the nitrogen atom to which they are attached, form 4- to 7-membered heterocycle;
      R₃ is H or C₁-C₁₈ hydrocarbyl, or cyclohydrocarbyl, phenyl, heterocycle;
      R₄ and R₅ are each independently C₁-C₂₄ hydrocarbyl, or cyclohydrocarbyl, phenyl, heterocycle.
Embodiment 25. The amino lipid compound according to Embodiment 24, wherein Z₇ and Z₈ are each independently -(C=O)O-, or -O(C=O)-.
Embodiment 26. The amino lipid compound according to Embodiment 24, wherein A₃, A₄, A₆ and A₇ are each independently C₁-C₁₂ alkylene.
Embodiment 27. The amino lipid compound according to Embodiment 24, wherein R₁ and R₂ are each independently C₁-C₁₈ alkyl.
Embodiment 28. The amino lipid compound according to Embodiment 27, wherein R₁ and R₂ are each independently C₁-C₆ alkyl.
Embodiment 29. The amino lipid compound according to Embodiment 28, wherein R₁ and R₂ are each independently methyl, ethyl, n-propyl, or isopropyl.
Embodiment 30. The amino lipid compound according to Embodiment 24, wherein R₁ and R₂, together with the nitrogen atom to which they are attached, form 4- to 7-membered heterocycle.
Embodiment 31. The amino lipid compound according to Embodiment 24, wherein R₁ and R₂, together with the nitrogen atom to which they are attached, form 5- to 6-membered heterocycle.
Embodiment 32. The amino lipid compound according to Embodiment 24, wherein R₃ is C₁-C₁₈ alkyl.
Embodiment 33. The amino lipid compound according to Embodiment 24, wherein R₄ and R₅ are each independently C₁-C₂₀ hydrocarbyl.
Embodiment 34. The amino lipid compound according to Embodiment 24, wherein R₄ and R₅ are each independently C₁-C₂₀ alkyl.
Embodiment 35. The amino lipid compound according to Embodiment 24, wherein R₄ and R₅ are each independently branched C₃-C₁₈ alkyl.
Embodiment 36. The amino lipid compound according to Embodiment 24, wherein R₄ and R₅ are each independently: 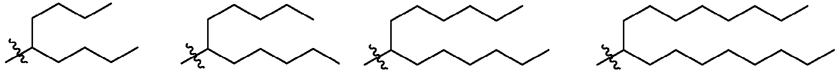 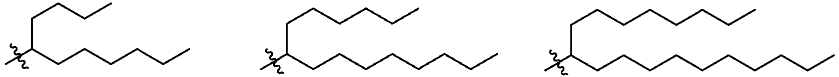 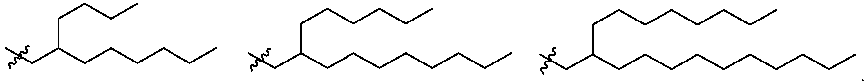
Embodiment 37. A lipid nanoparticle comprising the amino lipid compound according to any one of Embodiments 1 to 36 and optionally a pharmaceutically acceptable carrier, diluent, or excipient.
Embodiment 38. The lipid nanoparticle according to Embodiment 37, further comprising one or more of a helper lipid, a structural lipid, and a PEG-lipid;
   further, the lipid nanoparticle comprises the helper lipid, the structural lipid, and the PEG-lipid.
Embodiment 39. The lipid nanoparticle according to Embodiment 38, wherein the lipid nanoparticle comprises the amino lipid compound according to any one of Embodiments 1 to 36 in an amount (molar percent) of about 25.0%-75.0%, such as about 25.0%-28.0%, 28.0%-32.0%, 32.0%-35.0%, 35.0%-40.0%, 40.0%-42.0%, 42.0%-45.0%, 45.0%-48.0%, 48.0%-55.0%, 55.0%-65.0%, or 65.0%-75.0%, based on the total amount of the amino lipid compound according to any one of Embodiments 1 to 36, the helper lipid, the structural lipid, and the PEG-lipid.
Embodiment 40. The lipid nanoparticle according to any one of Embodiments 38 or 39, wherein the lipid nanoparticle comprises the helper lipid in an amount (molar percent) of about 5.0%-45.0%, such as about 5.0%-10.0%, 10.0%-16.0%, 16.0%-25.0%, 25.0%-33.5%, 33.5%-37.0%, 37.0%-40.0%, 40.0%-42.0%, or 42.0%-45.0%, based on the total amount of the amino lipid compound according to any one of Embodiments 1 to 36, the helper lipid, the structural lipid, and the PEG-lipid.
Embodiment 41. The lipid nanoparticle according to any one of Embodiments 38 to 40, wherein the lipid nanoparticle comprises the structural lipid in an amount (molar percent) of about 0.0%-50.0%, such as about 0.0%-10.0%, 10.0%-15.5%, 15.5%-22.5%, 22.5%-35.0%, 35.0%-36.5%, 36.5%-39.5%, 39.5%-40.5%, 40.5%-41.5%, 41.5%-45.0%, 45.0%-46.5%, or 46.5%-50.0%, based on the total amount of the amino lipid compound according to any one of Embodiments 1 to 36, the helper lipid, the structural lipid, and the PEG-lipid.
Embodiment 42. The lipid nanoparticle according to any one of Embodiments 38 to 41, wherein the lipid nanoparticle comprises the PEG-lipid in an amount (molar percent) of about 0.5%-5.0%, such as about 0.5%-1.0%, 1.0%-1.5%, 1.5%-2.0%, 2.0%-2.5%, 2.5%-3.0%, 3.0%-3.5%, 3.5%-4.0%, 4.0%-4.5%, or 4.5%-5.0%, based on the total amount of the amino lipid compound according to any one of Embodiments 1 to 36, the helper lipid, the structural lipid, and the PEG-lipid.
Embodiment 43. The lipid nanoparticle according to any one of Embodiments 38 to 42, wherein the helper lipid is one or more selected from the group consisting of DSPC, DOPE, DOPC, DOPS, DSPG, DPPG, DPPC, DGTS, and lysophospholipid, preferably one or more selected from the group consisting of DSPC, DOPE, DOPC, and DOPS, and more preferably DSPC and/or DOPE.
Embodiment 44. The lipid nanoparticle according to any one of Embodiments 38 to 43, wherein the structural lipid is at least one selected from the group consisting of cholesterol, cholesterol esters, steroid hormones, steroid vitamins, bile acid, cholesterin, ergosterol, β-sitosterol, and oxidized cholesterol derivatives, preferably at least one selected from the group consisting of cholesterol, cholesterol esters, steroid hormones, steroid vitamins, and bile acid, more preferably cholesterol, even more preferably high purity cholesterol, and particularly injection grade high purity cholesterol, such as CHO-HP.
Embodiment 45. The lipid nanoparticle according to any one of Embodiments 38 to 44, wherein the PEG-lipid is selected from PEG-DMG and PEG-DSPE, preferably PEG-DMG;
   preferably, the PEG-DMG is a polyethylene glycol (PEG) derivative of 1,2-dimyristoyl-sn-glycerol;
   preferably, the PEG has an average molecular weight of about 2,000 to 5,000, preferably about 2,000.
Embodiment 46. The lipid nanoparticle according to any one of Embodiments 38 to 45, wherein the molar ratio of the amino lipid compound according to any one of Embodiments 1 to 36: helper lipid: structural lipid: PEG-lipid is about 45:11:41.5:2.5, or 42.0:10.5:45.0:2.5, or 42.0:16.0:39.5:2.5, or 40.0:16.0:41.5:2.5, or 40.0:18.0:39.5:2.5, or 35.0:16.0:46.5:2.5, or 35.0:25.0:36.5:3.5, or 28.0:33.5:35.0:3.5, or 32.0:37.0:40.5:0.5, or 35.0:40.0:22.5:2.5, or 40.0:42.0:15.5:2.5.
Embodiment 47. The lipid nanoparticle according to Embodiment 46, wherein the helper lipid is DOPE, and the structural lipid is CHO-HP.
Embodiment 48. The lipid nanoparticle according to any one of Embodiments 38 to 45, wherein the molar ratio of the amino lipid compound according to any one of Embodiments 1 to 36: helper lipid: structural lipid: PEG-lipid is about 48.0:10.0:40.5:1.5.
Embodiment 49. The lipid nanoparticle according to Embodiment 48, wherein the helper lipid is DSPC, and the structural lipid is CHO-HP.
Embodiment 50. The lipid nanoparticle according to any one of Embodiments 37 to 49, further comprising a nucleic acid.
Embodiment 51. The lipid nanoparticle according to Embodiment 50, wherein the mass ratio of the amino lipid compound according to any one of Embodiments 1 to 36 to the nucleic acid is about (5-30):1, such as about (5-10):1, (10-15):1, (15-20):1, (20-25):1, or (25-30):1, preferably about 10:1.
Embodiment 52. The lipid nanoparticle according to Embodiment 50 or 51, wherein the nucleic acid is selected from the group consisting of RNA, antisense oligonucleotide, and DNA;
   preferably, the RNA is selected from the group consisting of messenger RNA (mRNA), ribosomal RNA (rRNA), microRNA (miRNA), transfer RNA (tRNA), small interfering RNA (siRNA), and small nuclear RNA (snRNA);
   preferably, the DNA is a plasmid.
Embodiment 53. A composition comprising the amino lipid compound according to any one of Embodiments 1 to 36 and a pharmaceutically acceptable carrier, diluent, or excipient.
Embodiment 54. The composition according to Embodiment 53, further comprising one or more of a helper lipid, a structural lipid, and a PEG-lipid;
   further, the composition further comprises the helper lipid, the structural lipid, and the PEG-lipid.
Embodiment 55. The composition according to Embodiment 54, wherein the composition comprises the amino lipid compound according to any one of Embodiments 1 to 36 in an amount (molar percent) of about 25.0%-75.0%, such as about 25.0%-28.0%, 28.0%-32.0%, 32.0%-35.0%, 35.0%-40.0%, 40.0%-42.0%, 42.0%-45.0%, 45.0%-48.0%, 48.0%-55.0%, 55.0%-65.0%, or 65.0%-75.0%, based on the total amount of the amino lipid compound according to any one of Embodiments 1 to 36, the helper lipid, the structural lipid, and the PEG-lipid.
Embodiment 56. The composition according to any one of Embodiments 54 to 55, wherein the composition comprises the helper lipid in an amount (molar percent) of about 5.0%-45.0%, such as about 5.0%-10.0%, 10.0%-16.0%, 16.0%-25.0%, 25.0%-33.5%, 33.5%-37.0%, 37.0%-40.0%, 40.0%-42.0%, or 42.0%-45.0%, based on the total amount of the amino lipid compound according to any one of Embodiments 1 to 36, the helper lipid, the structural lipid, and the PEG-lipid.
Embodiment 57. The composition according to any one of Embodiments 54 to 56, wherein the composition comprises the structural lipid in an amount (molar percent) of about 0.0%-50.0%, such as about 0.0%-10.0%, 10.0%-15.5%, 15.5%-22.5%, 22.5%-35.0%, 35.0%-36.5%, 36.5%-39.5%, 39.5%-40.5%, 40.5%-41.5%, 41.5%-45.0%, 45.0%-46.5%, or 46.5%-50.0%, based on the total amount of the amino lipid compound according to any one of Embodiments 1 to 36, the helper lipid, the structural lipid, and the PEG-lipid.
Embodiment 58. The composition according to any one of Embodiments 54 to 57, wherein the composition comprises the PEG-lipid in an amount (molar percent) of about 0.5%-5.0%, such as about 0.5%-1.0%, 1.0%-1.5%, 1.5%-2.0%, 2.0%-2.5%, 2.5%-3.0%, 3.0%-3.5%, 3.5%-4.0%, 4.0%-4.5%, or 4.5%-5.0%, based on the total amount of the amino lipid compound according to any one of Embodiments 1 to 36, the helper lipid, the structural lipid, and the PEG-lipid.
Embodiment 59. The composition according to any one of Embodiments 54 to 58, wherein the helper lipid is one or more selected from the group consisting of DSPC, DOPE, DOPC, DOPS, DSPG, DPPG, DPPC, DGTS, and lysophospholipid, preferably one or more selected from the group consisting of DSPC, DOPE, DOPC, and DOPS, and more preferably DSPC and/or DOPE.
Embodiment 60. The composition according to any one of Embodiments 54 to 58, wherein the structural lipid is at least one selected from the group consisting of cholesterol, cholesterol esters, steroid hormones, steroid vitamins, bile acid, cholesterin, ergosterol, β-sitosterol, and oxidized cholesterol derivatives, preferably at least one selected from the group consisting of cholesterol, cholesterol esters, steroid hormones, steroid vitamins, and bile acid, more preferably cholesterol, even more preferably high purity cholesterol, and particularly injection grade high purity cholesterol, such as CHO-HP.
Embodiment 61. The composition according to any one of Embodiments 54 to 58, wherein the PEG-lipid is selected from PEG-DMG and PEG-DSPE, preferably PEG-DMG;
   preferably, the PEG-DMG is a polyethylene glycol (PEG) derivative of 1,2-dimyristoyl-sn-glycerol;
   preferably, the PEG has an average molecular weight of about 2,000 to 5,000, preferably about 2,000.
Embodiment 62. The composition according to any one of Embodiments 54 to 61, wherein the molar ratio of the amino lipid compound according to any one of Embodiments 1 to 36: helper lipid: structural lipid: PEG-lipid is about 45:11:41.5:2.5, or 42.0:10.5:45.0:2.5, or 42.0:16.0:39.5:2.5, or 40.0:16.0:41.5:2.5, or 40.0:18.0:39.5:2.5, or 35.0:16.0:46.5:2.5, or 35.0:25.0:36.5:3.5, or 28.0:33.5:35.0:3.5, or 32.0:37.0:40.5:0.5, or 35.0:40.0:22.5:2.5, or 40.0:42.0:15.5:2.5.
Embodiment 63. The composition according to Embodiment 62, wherein the helper lipid is DOPE, and the structural lipid is CHO-HP.
Embodiment 64. The composition according to any one of Embodiments 54 to 61, wherein the molar ratio of the amino lipid compound according to any one of Embodiments 1 to 36: helper lipid: structural lipid: PEG-lipid is about 48.0:10.0:40.5:1.5.
Embodiment 65. The composition according to Embodiment 64, wherein the helper lipid is DSPC, and the structural lipid is CHO-HP.
Embodiment 66. The composition according to any one of Embodiments 53 to 65, further comprising a nucleic acid.
Embodiment 67. The composition according to Embodiment 66, wherein the mass ratio of the amino lipid compound according to any one of Embodiments 1 to 36 to the nucleic acid is about (5-30):1, such as about (5-10):1, (10-15):1, (15-20):1, (20-25):1, or (25-30):1, preferably about 10:1.
Embodiment 68. The composition according to Embodiment 66 or 67, wherein the nucleic acid is selected from the group consisting of RNA, antisense oligonucleotide, and DNA;
   preferably, the RNA is selected from the group consisting of messenger RNA (mRNA), ribosomal RNA (rRNA), microRNA (miRNA), transfer RNA (tRNA), small interfering RNA (siRNA), and small nuclear RNA (snRNA);
   preferably, the DNA is a plasmid.
Embodiment 69. The composition according to any one of Embodiments 53 to 68, further comprising a buffer;
   preferably, the buffer is selected from phosphate buffer and Tris buffer, preferably phosphate buffer; and/or
   preferably, the buffer has a concentration of about 5 mmol/L to 30 mmol/L, preferably about 10 mmol/L; and/or
   preferably, the buffer has a pH of about 6-8, preferably about 7-8, and more preferably about 7-7.5.
Embodiment 70. The composition according to any one of Embodiments 53 to 68, further comprising a cryoprotectant;
   preferably, the cryoprotectant is selected from sucrose and trehalose, preferably sucrose; and/or
   preferably, the cryoprotectant has a concentration of about 50 mg/ml to 100 mg/ml.
Embodiment 71. Use of the amino lipid compound according to any one of Embodiments 1 to 36 in the manufacture of a vehicle for an active ingredient.
Embodiment 72. The use according to Embodiment 71, wherein:
   the vehicle is the lipid nanoparticle according to any one of Embodiments 37 to 52; and/or
   the active ingredient is a pharmaceutically active ingredient, preferably a nucleic acid; and/or
   preferably, the nucleic acid is selected from the group consisting of RNA, antisense oligonucleotides, and DNA;
   preferably, the RNA is selected from the group consisting of messenger RNA (mRNA), ribosomal RNA (rRNA), microRNA (miRNA), transfer RNA (tRNA), small interfering RNA (siRNA), and small nuclear RNA (snRNA);
   preferably, the DNA is a plasmid.
Embodiment 73. Use of the amino lipid compound according to any one of Embodiments 1 to 36, the lipid nanoparticle according to any one of Embodiments 37 to 52, or the composition according to any one of Embodiments 53 to 70 in the manufacture of a medicament.
Embodiment 74. The use according to Embodiment 73, wherein the medicament is for use in gene therapy, gene vaccination, protein replacement therapy, antisense therapy, or therapy by interfering RNA.
Embodiment 75. The use according to Embodiment 74, wherein the gene therapy is for use in the treatment of cancers and genetic diseases.
Embodiment 76. The use according to Embodiment 75, wherein the cancers are one or more selected from the group consisting of lung cancer, stomach cancer, liver cancer, esophageal cancer, colon cancer, pancreatic cancer, brain cancer, lymphoma, blood cancer, or prostate cancer; and the genetic diseases are one or more selected from the group consisting of hemophilia, thalassemia, and Gaucher's disease.
Embodiment 77. The use according to Embodiment 75, wherein the gene vaccination is for use in the treatment of cancer, allergy, toxicity, and pathogen infection.
Embodiment 78. The use according to Embodiment 77, wherein the pathogen is one or more selected from the group consisting of viruses, bacteria, or fungi.
Embodiment 79. Use of the amino lipid compound according to any one of Embodiments 1 to 36, the lipid nanoparticle according to any one of Embodiments 37 to 52, or the composition according to any one of Embodiments 53 to 70 in the manufacture of a medicament for nucleic acid transfer.
Embodiment 80. The use according to Embodiment 79, wherein the nucleic acid is selected from the group consisting of RNA, antisense oligonucleotides, and DNA;
   preferably, the RNA is selected from the group consisting of messenger RNA (mRNA), ribosomal RNA (rRNA), microRNA (miRNA), transfer RNA (tRNA), small interfering RNA (siRNA), and small nuclear RNA (snRNA);
   preferably, the DNA is a plasmid.

### Beneficial effect

The amino lipid compound of the present disclosure can form a vehicle, such as a lipid nanoparticle, with excellent properties of encapsulating biologically active ingredients, can be used for delivering biologically active ingredients, especially water-insoluble drugs or active ingredients that are easily decomposed or degraded (such as nucleic acids), and improving its bioavailability and efficacy, or immunologic activity, or transfection efficiency (for nucleic acids), or safety, or preference to a particular organ or tissue. Particularly, the lipid nanoparticle comprising the amino lipid compound of the present disclosure has a clear delivery preference to spleen.

The lipid nanoparticle comprising the amino lipid compound of the present disclosure has a clear delivery preference to immune cells in different tissues and organs, wherein the tissues and organs include spleen, lymph, peripheral blood, bone marrow, lung, liver, etc., and the immune cells include neutrophils, NK cells-NK T cells, macrophages-monocytes, dendritic cells, B cells, T cells, CD4 T cells, etc.

### Brief description of the drawings

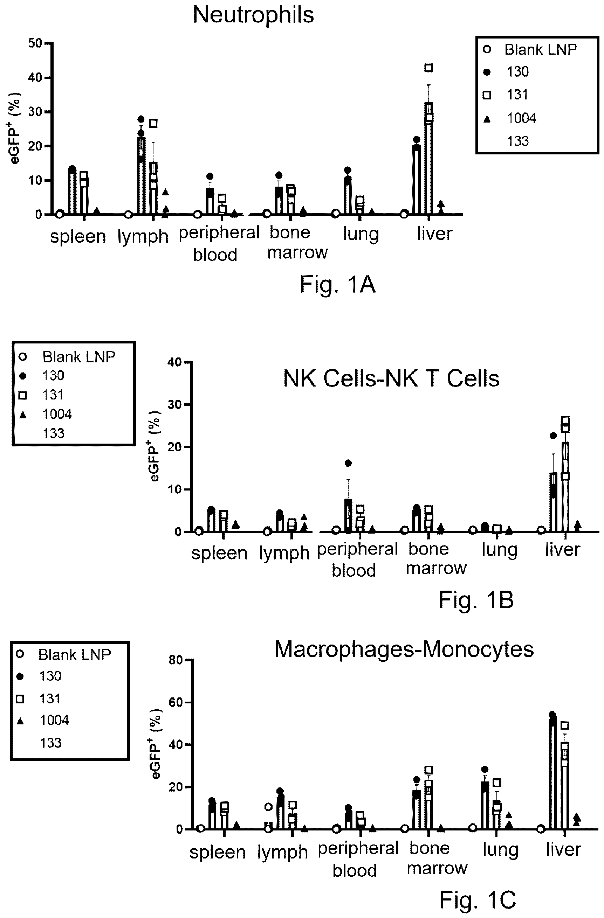

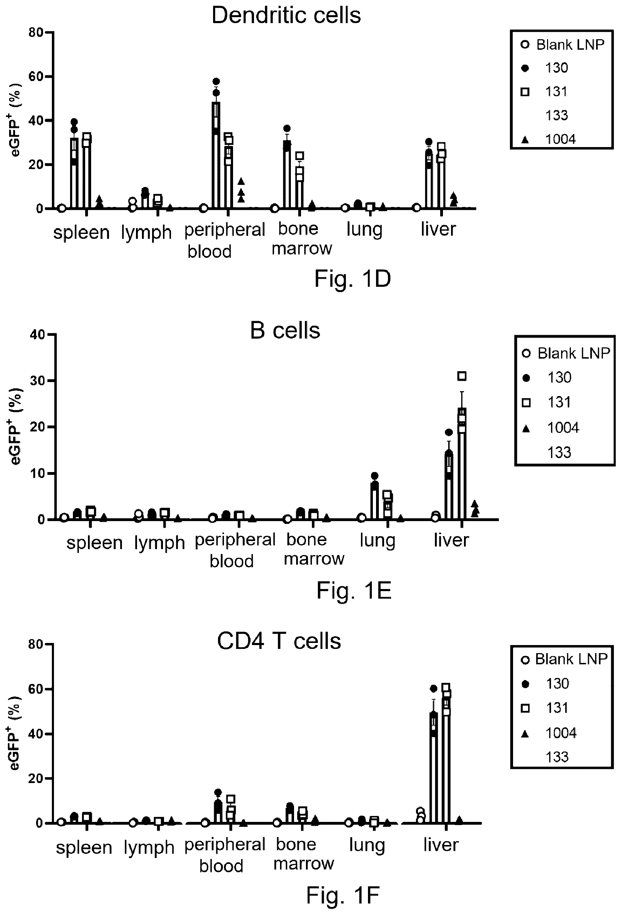

FIGS. 1A to 1F show the delivery efficiency of lipid nanoparticles comprising different amino lipid compounds to immune cell populations in different tissues and organs, wherein FIG. 1A shows neutrophils; FIG. 1B shows NK cells-NK T cells; FIG. 1C shows macrophages-monocytes; FIG. 1D shows dendritic cells; FIG. 1E shows B cells; and FIG. 1F shows CD4 T cells.

In order to make the purposes, technical solutions, and advantages of the present disclosure clearer, the present disclosure is described below with reference to specific examples. The following examples are merely illustrative of the present disclosure and are not intended to be limiting.

### Examples

The following examples are provided for purposes of illustration and not limitation.

The experimental methods for which specific conditions are not specified in the examples are usually under conventional conditions or conditions as recommended by the manufacturer of the raw material or commodity; and the reagents of unspecified origin are generally conventional reagents commercially available.

The abbreviations used in the examples have the following meanings:
- Pd/C: Palladium/carbon;
- EA: Ethyl acetate;
- DCM: Dichloromethane;
- MPa: MegaPascal;
- DMF: N,N-dimethylformamide;
- EDCI: 1-(3-Dimethylaminopropyl)-3-ethylcarbodiimide hydrochloride;
- DMAP: 4-Dimethylaminopyridine;
- TEMPO: 2,2,6,6-Tetramethylpiperidinooxy;
- NaDCC: Sodium dichloroisocyanurate;
- h: Hour;
- min: Minute.

### Example 1: Synthesis of Amino Lipid Compound 125

### (1) Synthesis of Compound 1522F-1

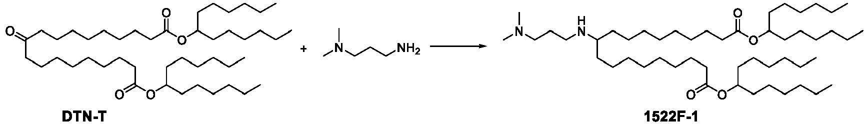

10.0 g DTN-T (14.1 mmol), 2.88 g N,N-dimethylaminopropylamine, 0.5 g Pd/C (10%), and 80 ml absolute ethanol were added to a 200 ml autoclave, sealed, and replaced with nitrogen three times, and replaced with hydrogen three times, then filled with hydrogen to a pressure of 4 MPa, and then heated to 70°C. After reacting for 48 hours, suction filtration was performed, the filter cake was washed with absolute ethanol, and the filtrate was concentrated to obtain 11 g crude product. The crude product was purified by silica gel column chromatography and eluted with EA: methanol = 9:1 to obtain 9 g 1522F-1 with a yield of 80%.

### (2) Synthesis of Compound 125-G

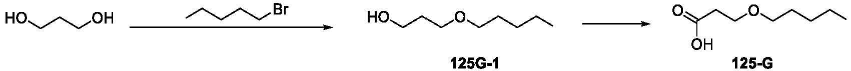

8.67 g Sodium hydride and 195 ml DMF were added to a 500 mL two-necked flask, the device was cooled to 0°C under nitrogen protection, and then slowly added with 15.0 g 1,3-propanediol (197.1 mmol). After reacting for 30 min, the mixture was heated to room temperature, continued to react for 1 h, and then cooled to 0°C. 35.7 g 1-bromopentane was slowly added dropwise to the reaction solution. After reacting for 30 min, the mixture was heated to room temperature, continued to react for 15 h, and added with 100 mL water, extracted with 100 mL EA, dried over anhydrous sodium sulfate, and concentrated under reduced pressure. The concentrate was subjected to silica gel column chromatography and elution with EA: n-hexane = 1:8 to obtain 17.10 g 125G-1 with a yield of 59.3%.

17 g 125G-1 (116.2 mmol), 100 ml DCM, 0.54 g TEMPO, 23.3 g KHCO₃, and 1.2 g NaBr were added to a 1000 mL round-bottom flask, added with 38.34 g aqueous NaDCC solution while stirring, reacted with stirring at room temperature for 15 h, and then filtered. The organic phase was separated from the filtrate, and the aqueous phase was extracted twice with 100 ml DCM. The organic phases were combined and concentrated under reduced pressure. The concentrate was subjected to silica gel column chromatography and elution with EA: n-hexane = 1:8 to obtain 11 g 125-G with a yield of 64.7%.

¹H NMR in (CDCl₃) δ 3.71-3.69 (m, 2H), 3.47-3.44 (m, 2H), 2.64-2.61 (m, 2H), 1.57-1.54 (m, 2H), 1.34-1.25 (m, 6H), 0.89-0.87 (m, 3H).

### (3) Synthesis of Amino Lipid Compound 125

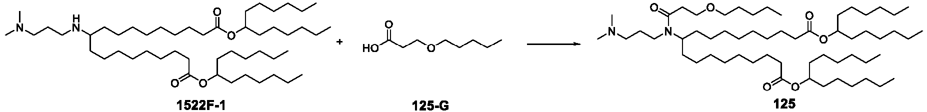

0.47 g EDCI, 0.02 g DMAP, and 30 ml DCM were sequentially added to a 100 ml round-bottom flask, and after stirring for 10 min, a solution of 1522F-1 (1.3 g, 1.64 mmol) in DCM (3 ml), a solution of 125-G (0.34 g) in DCM (3 ml), and a solution of pyridine (0.2 g) in DCM (3 ml) were sequentially added dropwise. After stirring at room temperature for 15 h, the mixture was adjusted to pH 3 with dilute hydrochloric acid, added with 30 ml water, and extracted with 30 ml DCM. The organic phase was dried over anhydrous sodium sulfate and concentrated under reduced pressure to obtain a 1.5 g crude product. The crude product was subjected to silica gel column chromatography and elution with EA: n-hexane = 1.5:1 to obtain amino lipid compound 125 (1.1 g) with a purity of 95.88% and a yield of 72%.

¹H NMR (CDCl₃) δ 4.86 (m, 2H), 3.74-3.72 (m, 2H), 3.62 (m, 1H), 3.43-3.42 (m, 2H), 3.12 (m, 2H), 2.60 (m, 2H), 2.28-2.21(m, 12H), 1.71-1.44 (m, 20H), 1.25 (m, 56H), 0.87-0.86 (m, 15H).

LC-MS (ESI): (M+H) 936.4.

### Example 2: Synthesis of Amino Lipid Compound 120

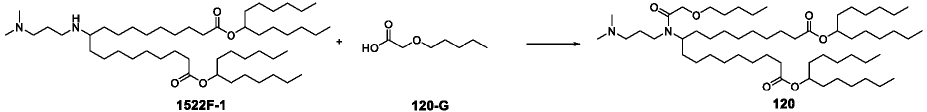

According to the general synthetic process, amino lipid compound 120 (0.56 g) was prepared from compound 1522F-1 (1.3 g, 1.64 mmol) and 0.31 g 120-G, with a purity of 90.85% and a yield of 37%.

¹H NMR (CDCl₃) δ 4.86-4.84 (m, 2H), 4.13-4.09 (m, 2H), 3.55-3.46 (m, 3H), 3.14-3.11 (m, 2H), 2.30-2.21 (m, 12H), 1.74-1.44 (m, 20H), 1.31-1.25 (m, 56H), 0.87-0.85 (m, 15H).

LC-MS (ESI): (M+H) 922.4.

### Example 3: Synthesis of Amino Lipid Compound 130

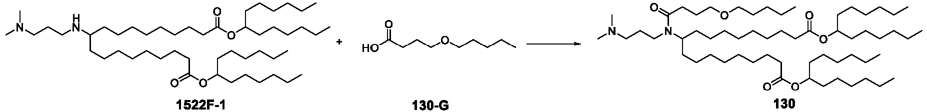

According to the general synthetic process, amino lipid compound 130 (1.05 g) was prepared from compound 1522F-1 (1.3 g, 1.64 mmol) and 0.37 g 130-G, with a purity of 92.04% and a yield of 67%.

¹H NMR (CDCl₃) δ 4.87-4.85 (m, 2H), 3.65 (m, 1H), 3.45-3.43 (m, 2H), 3.39-3.37 (m, 2H), 3.12-3.11 (m, 2H), 2.41-2.37 (m, 2H), 2.30-2.22(m, 12H), 1.93-1.89 (m, 2H), 1.71-1.44 (m, 20H), 1.25 (m, 56H), 0.88-0.86 (m, 15H).

LC-MS (ESI): (M+H) 950.4.

### Example 4: Synthesis of Amino Lipid Compound 144

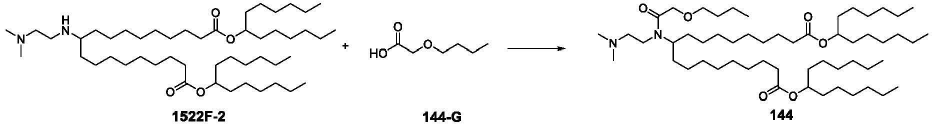

According to the general synthetic process, amino lipid compound 144 (1.0 g) was prepared from compound 1522F-2 (1.3 g, 1.67 mmol) and 0.28 g 144-G, as a colorless oil with a purity of 95.67% and a yield of 68%.

¹H NMR (CDCl₃) δ 4.87-4.85 (m, 2H), 4.15-4.09 (m, 2H), 3.51-3.47 (m, 3H), 3.25-3.23 (m, 2H), 2.47-2.45 (m, 2H), 2.28-2.25 (m, 10H), 1.60-1.35 (m, 20H), 1.25 (m, 52H), 0.92-0.85 (m, 15H).

LC-MS (ESI): (M+H) 894.3.

### Example 5: Synthesis of Amino Lipid Compound 149

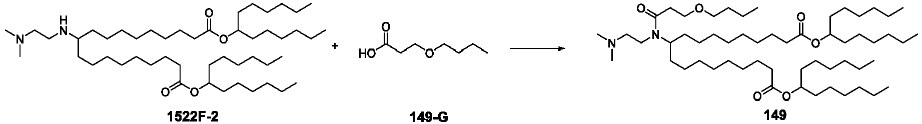

According to the general synthetic process, amino lipid compound 149 (0.73 g) was prepared from compound 1522F-2 (1.3 g, 1.67 mmol) and 0.31 g 149-G, as a colorless oil with a purity of 91.68% and a yield of 49%.

¹H NMR (CDCl₃) δ 4.87-4.85 (m, 2H), 3.75-3.72 (m, 2H), 3.63-3.61 (m, 1H), 3.45-3.41 (m, 2H), 3.26-3.22 (m, 2H), 2.61-2.58 (m, 2H), 2.44-2.42 (m, 2H), 2.28-2.25 (m, 10H), 1.61-1.33 (m, 20H),1.30-1.25 (m, 52H), 0.92-0.86 (m, 15H).

LC-MS (ESI): (M+H) 908.3.

### Example 6: Synthesis of Amino Lipid Compound 154

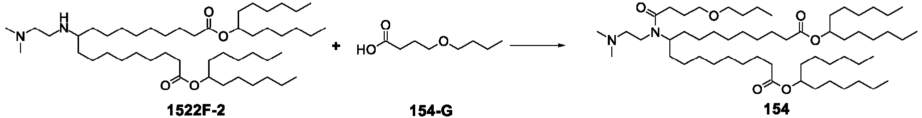

According to the general synthetic process, amino lipid compound 154 (1.0 g) was prepared from compound 1522F-2 (1.3 g, 1.67 mmol) and 0.34 g 154-G, as a colorless oil with a purity of 93.80% and a yield of 66%.

¹H NMR (CDCl₃) δ 4.87-4.85 (m, 2H), 3.65-3.63 (m, 1H), 3.45-3.43 (m, 2H), 3.40-3.38 (m, 2H), 3.24-3.21 (m, 2H), 2.44-2.36 (m, 4H), 2.29-2.25 (m, 10H), 1.93-1.89 (m, 2H), 1.60-1.33 (m, 20H), 1.25 (m, 52H), 0.92-0.85 (m, 15H).

LC-MS (ESI): (M+H) 922.3.

### Example 7: Synthesis of Amino Lipid Compound 166

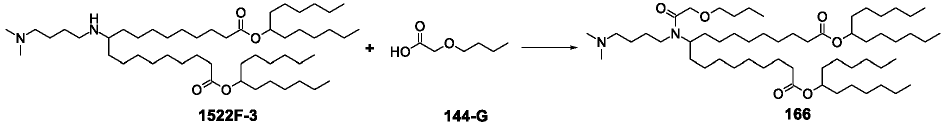

According to the general synthetic process, amino lipid compound 166 (0.33 g) was prepared from compound 1522F-3 (1.3 g, 1.61 mmol) and 0.28 g 144-G, as a colorless oil with a purity of 91.31% and a yield of 22%.

¹H NMR (CDCl₃) δ 4.87-4.84 (m, 2H), 4.10 (d, J = 20 Hz, 1H), 3.50-3.47 (m, 3H), 3.09-3.07 (m, 2H), 2.30-2.25 (m, 6H), 2.22 (d, J = 3.7 Hz, 6H), 1.61-1.36 (m, 24H), 1.29-1.25 (m, 52H), 0.92-0.85 (m, 15H).

LC-MS (ESI): (M+H) 922.3.

### Example 8: Synthesis of Amino Lipid Compound 172

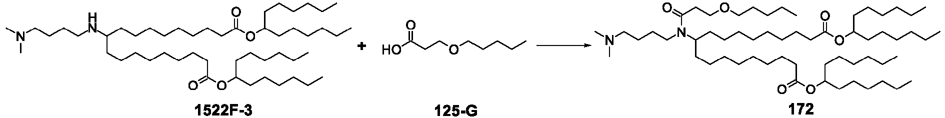

According to the general synthetic process, amino lipid compound 172 (0.43 g) was prepared from compound 1522F-3 (1.3 g, 1.61 mmol) and 0.37 g 125-G, as a colorless oil with a purity of 97.49% and a yield of 28%.

¹H NMR (CDCl₃) δ 4.87-4.85 (m, 2H), 3.74-3.72 (m, 2H), 3.64-3.59 (m, 1H), 3.44-3.42 (m, 2H), 3.10-3.06 (m, 2H), 2.61-2.60 (m, 2H), 2.28-2.23 (m, 12H), 1.62-1.43 (m, 22H), 1.31-1.26 (m, 52H), 0.90-0.86 (m, 15H).

LC-MS (ESI): (M+H) 950.4.

### Example 9: Synthesis of Amino Lipid Compound 176

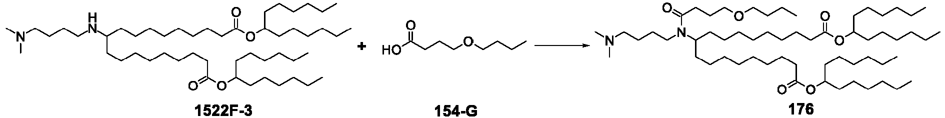

According to the general synthetic process, amino lipid compound 176 (0.26 g) was prepared from compound 1522F-3 (1.3 g, 1.61 mmol) and 0.37 g 154-G, as a colorless oil with a purity of 92.77% and a yield of 17%.

¹H NMR (CDCl₃) δ 4.87-4.85 (m, 2H), 3.64-3.62 (m, 1H), 3.45-3.42 (m, 2H), 3.40-3.38 (m, 2H), 3.07-3.05 (m, 2H), 2.41-2.36 (m, 2H), 2.28-2.25 (m, 6H), 2.22 (s, 6H), 1.91-1.90 (m, 2H), 1.61-1.33 (m, 24H),1.27-1.25 (m, 52H), 0.92-0.86 (m, 15H).

LC-MS (ESI): (M+H) 950.4.

### Example 10: Synthesis of Amino Lipid Compound 119

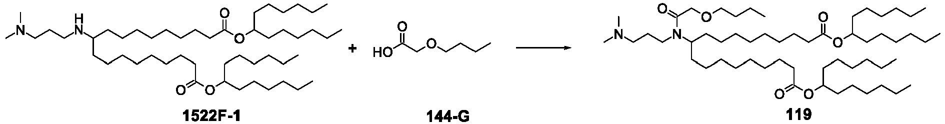

According to the general synthetic process, amino lipid compound 119 (0.55 g) was prepared from compound 1522F-1 (1.3 g, 1.64 mmol) and 0.28 g 144-G, as a colorless oil with a purity of 89.83% and a yield of 37%.

¹H NMR (CDCl₃) δ 4.86-4.85 (m, 2H), 4.13-4.09 (m, 2H), 3.49-3.48 (m, 3H), 3.13 (m, 2H), 2.26-2.21 (m, 12H), 1.74-1.45 (m, 20H), 1.37-1.25 (m, 54H), 0.91-0.85 (m, 15H).

LC-MS (ESI): (M+H) 908.4.

### Example 11: Synthesis of Amino Lipid Compound 121

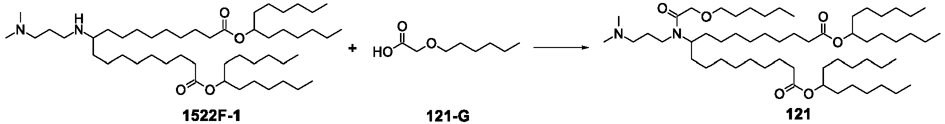

According to the general synthetic process, amino lipid compound 121 (0.88 g) was prepared from compound 1522F-1 (1.3 g, 1.64 mmol) and 0.34 g 121-G, as a colorless oil with a purity of 92.94% and a yield of 57%.

¹H NMR (CDCl₃) δ 4.86-4.85 (m, 2H), 4.13-4.09 (m, 2H), 3.55-3.46 (m, 3H), 3.14-3.13 (m, 2H), 2.31-2.21 (m, 12H), 1.75-1.45 (m, 20H), 1.33-1.25 (m, 58H), 0.87-0.85 (m, 15H).

LC-MS (ESI): (M+H) 936.5.

### Example 12: Synthesis of Amino Lipid Compound 124

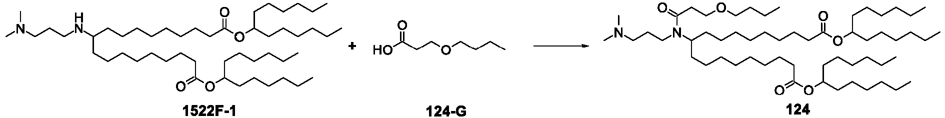

According to the general synthetic process, amino lipid compound 124 (1.2 g) was prepared from compound 1522F-1 (1.3 g, 1.64 mmol) and 0.28 g 124-G, as a colorless oil with a purity of 95.32% and a yield of 79%.

¹H NMR (CDCl₃) δ 4.86-4.85 (m, 2H), 3.75-3.72 (m, 2H), 3.64-3.62 (m, 1H), 3.44-3.42 (m, 2H), 3.15-3.10 (m, 2H), 2.61-2.59 (m, 2H), 2.28-2.21 (m, 12H), 1.72-1.44 (m, 20H), 1.35-1.25 (m, 54H), 0.91-0.86 (m, 15H).

LC-MS (ESI): (M+H) 922.3.

### Example 13: Synthesis of Amino Lipid Compound 129

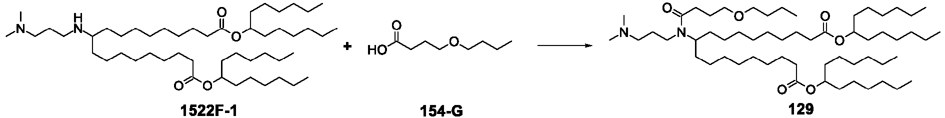

According to the general synthetic process, amino lipid compound 129 (0.79 g) was prepared from compound 1522F-1 (1.3 g, 1.64 mmol) and 0.34 g 154-G, as a colorless oil with a purity of 92.29% and a yield of 51%.

¹H NMR (CDCl₃) δ 4.87-4.85 (m, 2H), 3.65 (p, J=7.0 Hz, 1H), 3.44 (q, J=6.2 Hz, 2H), 3.39 (t, J=6.6 Hz, 2H), 3.13-3.10 (m, 2H), 2.41-2.22(m, 14H), 1.97-1.88 (m, 2H), 1.61-1.44 (m, 20H), 1.36-1.25 (m, 54H), 0.92-0.86 (m, 15H).

LC-MS (ESI): (M+H) 936.4.

### Example 14: Synthesis of Amino Lipid Compound 131

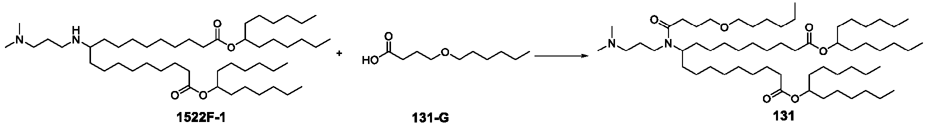

According to the general synthetic process, amino lipid compound 131 (1.05 g) was prepared from compound 1522F-1 (1.3 g, 1.64 mmol) and 0.40 g 131-G, as a colorless oil with a purity of 94.66% and a yield of 66%.

¹H NMR (CDCl₃) δ 4.88-4.84 (m, 2H), 3.65 (p, J=7.0 Hz, 1H), 3.44 (q, J=6.2 Hz, 2H), 3.38 (t, J=6.7 Hz, 2H), 3.13-3.10(m, 2H), 2.43-2.37 (m, 2H), 2.31-2.22(m, 12H), 1.95-1.88 (m, 2H), 1.74-1.67 (m, 2H),1.61-1.44 (m, 18H), 1.32-1.26 (m, 58H), 0.89-0.86 (m, 15H).

LC-MS (ESI): (M+H) 964.4.

### Example 15: Synthesis of Amino Lipid Compound 132

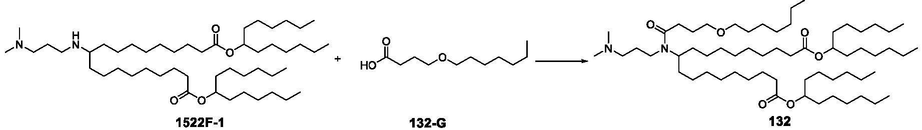

According to the general synthetic process, amino lipid compound 132 (1.08 g) was prepared from compound 1522F-1 (1.3 g, 1.64 mmol) and 0.43g 132-G, as a colorless oil with a purity of 97.18% and a yield of 67%.

¹H NMR (CDCl₃) δ 4.88-4.84 (m, 2H), 3.65 (p, J=7.0 Hz, 1H), 3.44 (q, J=6.2 Hz, 2H), 3.38 (t, J=6.6 Hz, 2H), 3.13-3.10(m, 2H), 2.43-2.37 (m, 2H), 2.30-2.22(m, 12H), 1.95-1.88 (m, 2H), 1.73-1.67 (m, 2H),1.61-1.44 (m, 18H), 1.30-1.26 (m, 60H), 0.88-0.86 (m, 15H).

LC-MS (ESI): (M+H) 978.4.

### Example 16: Synthesis of Amino Lipid Compound 133

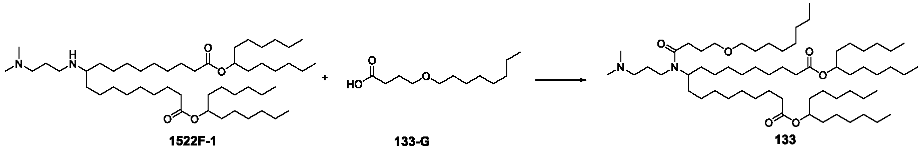

According to the general synthetic process, amino lipid compound 133 (1.26 g) was prepared from compound 1522F-1 (1.3 g, 1.64 mmol) and 0.46g 133-G, as a colorless oil with a purity of 95.54% and a yield of 77%.

¹H NMR (CDCl₃) δ 4.87-4.83 (m, 2H), 3.64 (p, J=7.0 Hz, 1H), 3.43 (q, J=6.2 Hz, 2H), 3.37 (t, J=6.7 Hz, 2H), 3.12-3.09(m, 2H), 2.42-2.36 (m, 2H), 2.30-2.20(m, 12H), 1.94-1.87 (m, 2H), 1.72-1.66 (m, 2H),1.62-1.42 (m, 18H), 1.29-1.24 (m, 62H), 0.87-0.85 (m, 15H).

LC-MS (ESI): (M+H) 992.4.

### Example 17: Synthesis of Amino Lipid Compound 036

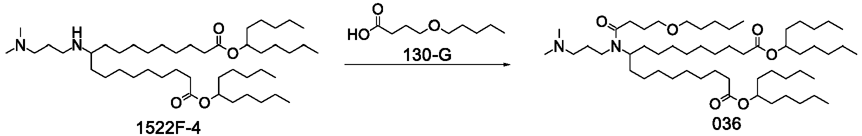

According to the general synthetic process, amino lipid compound 036 (1.1 g) was prepared from compound 1522F-4 (3.0 g, 4.07 mmol) and 0.93 g 130-G, as a light yellow oil with a purity of 96.18% and a yield of 30.3%.

¹H NMR (600 MHz, CDCl3) δ 4.95-4.85 (m, 2H), 3.73-3.63 (m, 1H), 3.48 (t, J = 6.2 Hz, 2H), 3.41 (t, J = 6.7 Hz, 2H), 3.17-3.11 (m, 2H), 2.45 (t, J = 7.3 Hz, 1H), 2.41 (t, J = 7.3 Hz, 1H), 2.34-2.28 (m, 6H), 2.24 (m, 6H), 2.04-1.86 (m, 4H), 1.79-1.70 (m, 2H), 1.65-1.43 (m, 16H), 1.38-1.10 (m, 48H), 0.96-0.84 (m, 15H).

LC-MS (ESI): (M+H) 893.9.

### Example 18: Synthesis of Amino Lipid Compound 037

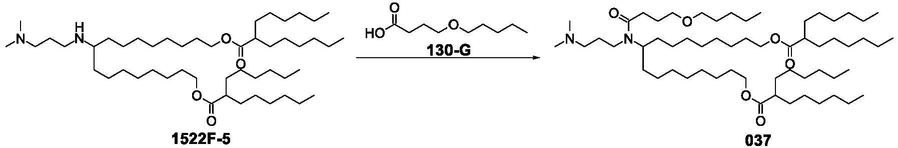

According to the general synthetic process, amino lipid compound 037 (0.25 g) was prepared from compound 1522F-5 (0.60 g, 0.75 mmol) and 0.27 g 130-G, as a light yellow oil with a purity of 95.17% and a yield of 35.4%.

¹H NMR (600 MHz, CDCl3) δ 4.08 (m, 4H), 3.73-3.64 (m, 1H), 3.48 (t, J = 6.1 Hz, 2H), 3.42 (t, J = 6.7 Hz, 2H), 3.15 (m, 2H), 2.45 (t, J = 7.3 Hz, 1H), 2.42 (t, J = 7.3 Hz, 1H), 2.36-2.28 (m, 4H), 2.25 (m, 6H), 1.98-1.87 (m, 4H), 1.78-1.71 (m, 2H), 1.66-1.55 (m, 10H), 1.52-1.42 (m, 6H), 1.39-1.20 (m, 56H), 0.94-0.84 (m, 15H).

LC-MS (ESI): (M+H) 950.0.

### Example 19: Synthesis of Amino Lipid Compound 038

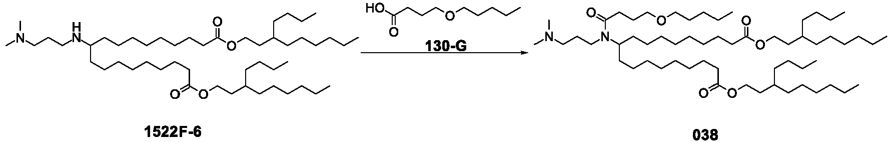

According to the general process, amino lipid compound 038 (0.65 g) was prepared from compound 1522F-6 (0.82 g, 1.03 mmol) and 0.20 g 130-G, as a light yellow oil with a purity of 95.32% and a yield of 66.2%.

¹H NMR (600 MHz, CDCl3) δ 3.90-3.85 (t, J = 6.5 Hz, 4H), 3.70-3.62 (m, 1H), 3.47-3.44 (m, 2H), 3.42-3.40 (t, J = 6.7 Hz, 2H), 3.13-3.10 (m, 2H), 2.46-2.42 (t, J = 7.3 Hz, 1H), 2.42-2.39 (t, J = 7.3 Hz, 1H), 2.33-2.27 (m, 6H), 2.23 (s, 6H), 1.90-1.86 (m, 4H), 1.67-1.60 (m, 6H), 1.58-1.54 (m, 2H), 1.44-1.40 (m, 4H), 1.36-1.13 (m, 60H), 0.93-0.80 (m, 15H).

LC-MS (ESI): (M+H) 950.6.

### Example 20: Synthesis of Amino Lipid Compound 039

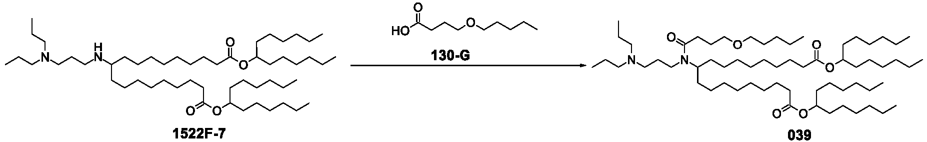

According to the general process, amino lipid compound 039 (0.92 g) was prepared from compound 1522F-7 (2.2 g, 2.59 mmol) and 0.59 g 130-G, as a light yellow oil with a purity of 95.23% and a yield of 35.3%.

¹H NMR (600 MHz, CDCl3) δ 4.91-4.87 (m, 2H), 3.74-3.71 (m, 1H), 3.45-3.42 (m, 2H), 3.41-3.39 (t, J = 6.7 Hz, 2H), 3.14-3.03 (m, 2H), 2.54-2.50 (m, 4H), 2.46-2.41 (m, 4H), 2.30-2.26 (m, 4H), 2.01-1.86 (m, 4H), 1.78-1.69 (m, 2H), 1.67-1.61 (m, 4H), 1.60-1.55 (m, 2H), 1.52-1.50 (m, 6H), 1.47-1.40 (m, 4H), 1.34-1.20 (m, 60H), 0.92-0.87 (m, 21H).

LC-MS (ESI): (M+H) 1006.4.

### Example 21: Synthesis of Amino Lipid Compound 040

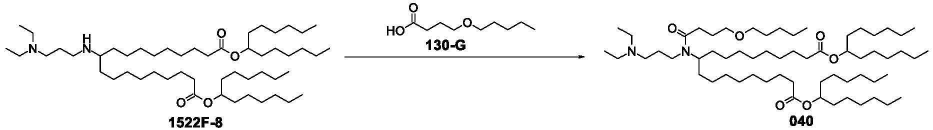

According to the general process, amino lipid compound 040 (2.4 g) was prepared from compound 1522F-8 (4.3 g, 5.23 mmol) and 1.19 g 130-G, as a light yellow oil with a purity of 95.77% and a yield of 46.9%.

¹H NMR (600 MHz, CDCl3) δ 4.91-4.87 (m, 2H), 3.78-3.76 (m, 1H), 3.48-3.46 (m, 2H), 3.42-3.40 (t, J = 6.7 Hz, 2H), 3.17-3.05 (m, 2H), 2.57-2.52 (m, 4H), 2.48-2.40 (m, 4H), 2.32-2.27 (m, 4H), 2.00-1.86 (m, 4H), 1.77-1.68 (m, 2H), 1.66-1.60 (m, 4H), 1.60-1.56 (m, 2H), 1.53-1.52 (m, 6H), 1.49-1.41 (m, 4H), 1.35-1.21 (m, 56H), 1.06-1.03 (t, J = 7.1 Hz, 6H), 0.91-0.88 (m, 15H).

LC-MS (ESI): (M+H) 978.3.

### Example 22: Synthesis of Amino Lipid Compound 041

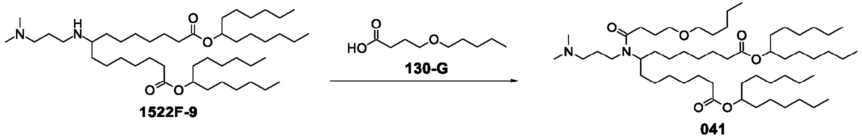

According to the general process, amino lipid compound 041 (0.56 g) was prepared from compound 1522F-9 (1.03 g, 1.4 mmol) and 0.32 g 130-G, as a light yellow oil with a purity of 96.26% and a yield of 44.9%.

¹H NMR (600 MHz, CDCl3) δ 4.93-4.83 (m, 2H), 3.78-3.58 (m, 1H), 3.47 (t, J = 5.9 Hz, 2H), 3.41 (t, J = 6.7 Hz, 2H), 3.14 (m, 2H), 2.49-2.37 (m, 3H), 2.36-2.24 (m, 11H), 1.99-1.87 (m, 2H), 1.80-1.69 (m, 2H), 1.66-1.43 (m, 18H), 1.37-1.22 (m, 48H), 0.97-0.85 (m, 15H).

LCMS (ESI): (M+H) 893.9.

### Example 23: Synthesis of Amino Lipid Compound 042

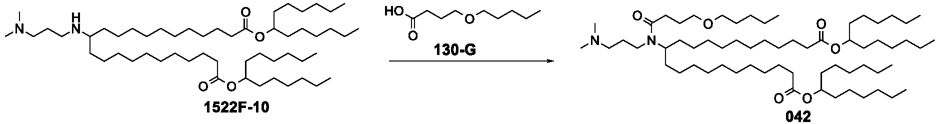

According to the general process, amino lipid compound 042 (0.93 g) was prepared from compound 1522F-10 (1.91 g, 2.25 mmol) and 0.47 g 130-G, as a light yellow oil with a purity of 95.58% and a yield of 41.1%.

¹H NMR (600 MHz, CDCl3) δ 4.95-4.82 (m, 2H), 3.73-3.62 (m, 1H), 3.50-3.44 (m, 2H), 3.44-3.39 (m, 2H), 3.16-3.13 (m, 2H), 2.44-2.41 (m, 4H), 2.32-2.29 (m, 4H), 2.29 (s, 4H), 2.26 (s, 2H), 1.98-1.86 (m, 4H), 1.79-1.71 (m, 2H), 1.66-1.61 (m, 4H), 1.60-1.56 (m, 2H), 1.55-1.51 (m, 6H), 1.47 (m, 4H), 1.38-1.14 (m, 64H), 0.94-0.89 (m, 15H).

LCMS (ESI): (M+H) 1006.3.

### Example 24: Synthesis of Amino Lipid Compound 044

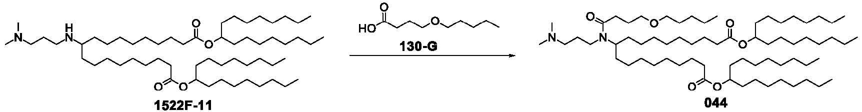

According to the general process, amino lipid compound 044 (0.32 g) was prepared from compound 1522F-11 (1.35 g, 1.49 mmol) and 0.34 g 130-G, as a light yellow oil with a purity of 95.13% and a yield of 20.2%.

¹H NMR (600 MHz, CDCl3) δ 4.95-4.81 (m, 2H), 3.70-3.66 (m, 1H), 3.49-3.46 (m, 2H), 3.43-3.40 (t, J = 6.7 Hz, 2H), 3.23-3.07 (m, 2H), 2.46-2.44 (t, J = 7.3 Hz, 1H), 2.43-2.40 (t, J = 7.3 Hz, 1H), 2.35-2.28 (m, 6H), 2.25 (s, 6H), 2.02-1.93 (m, 4H), 1.80-1.70 (m, 2H), 1.67-1.61 (m, 4H), 1.61-1.57 (m, 2H), 1.53 (m, 6H), 1.50-1.42 (m, 4H), 1.40-1.15 (m, 72H), 0.91 (m, 15H).

LC-MS (ESI): (M+H) 1062.5.

### Example 25: Synthesis of Amino Lipid Compound 045

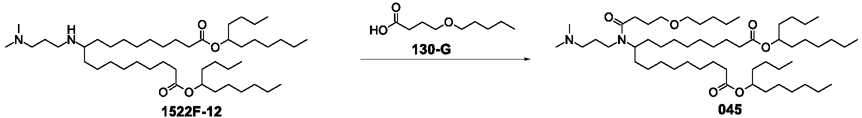

According to the general process, amino lipid compound 045 (1.6 g) was prepared from compound 1522F-12 (2.8 g, 3.8 mmol) and 0.86 g 130-G, as a light yellow oil with a purity of 97.38% and a yield of 47.2%.

¹H NMR (600 MHz, CDCl3) δ 4.97-4.84 (m, 2H), 3.70-3.66 (m, 1H), 3.49-3.47 (t, J = 6.1 Hz, 2H), 3.43-3.40 (t, J = 6.7 Hz, 2H), 3.16-3.13 (m, 2H), 2.46-2.44 (t, J = 7.2 Hz, 1H), 2.43-2.40 (t, J = 7.3 Hz, 1H), 2.34-2.27 (m, 6H), 2.25 (s, 6H), 1.98-1.92 (m, 4H), 1.78-1.70 (m, 2H), 1.67-1.61 (m, 4H), 1.60-1.57 (m, 2H), 1.56-1.53 (m, 6H), 1.48-1.47 (m, 4H), 1.39-1.17 (m, 48H), 0.99-0.86 (m, 15H).

LC-MS (ESI): (M+H) 893.9.

### Example 26: Synthesis of Amino Lipid Compound 046

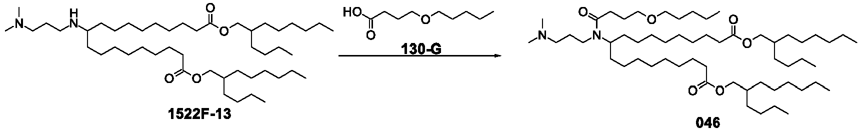

According to the general process, amino lipid compound 046 (0.71 g) was prepared from compound 1522F-13 (1.00 g, 1.31 mmol) and 0.30 g 130-G, as a light yellow oil with a purity of 95.61% and a yield of 59.0%.

¹H NMR (600 MHz, CDCl3) δ 4.00 (d, J = 1.8 Hz, 2H), 3.99 (d, J = 1.8 Hz, 2H), 3.72-3.63 (m, 1H), 3.49-3.46 (m, 2H), 3.43-3.41 (t, J = 6.7 Hz, 2H), 3.15 (m, 2H), 2.47-2.44 (t, J = 7.3 Hz, 1H), 2.43-2.40 (t, J = 7.3 Hz, 1H), 2.34-2.29 (m, 6H), 2.25 (s, 6H), 1.91-1.88 (m, 4H), 1.69-1.61 (m, 6H), 1.61-1.56 (m, 2H), 1.46 (m, 4H), 1.38-1.16 (m, 56H), 0.96-0.84 (m, 15H).

LC-MS (ESI): (M+H) Found 922.2.

### Example 27: Synthesis of Amino Lipid Compound 047

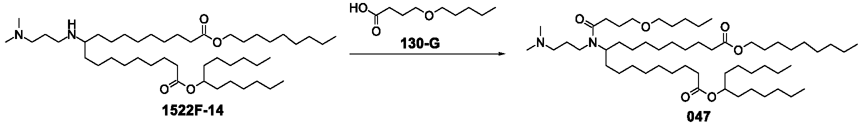

According to the general process, amino lipid compound 047 (0.13 g) was prepared from compound 1522F-14 (0.80 g, 1.09 mmol) and 0.25 g 130-G, as a light yellow oil with a purity of 96.01% and a yield of 13.4%.

¹H NMR (600 MHz, CDCl3) δ 4.94-4.85 (m, 1H), 4.09-4.07 (m, 2H), 3.72-3.63 (m, 1H), 3.49-3.47 (t, J = 6.0 Hz, 2H), 3.42-3.40 (t, J = 6.7 Hz, 2H), 3.16-3.13 (m, 2H), 2.46-2.44 (t, J = 7.3 Hz, 1H), 2.43-2.40 (t, J = 7.3 Hz, 1H), 2.35-2.28 (m, 6H), 2.25 (s, 6H), 1.97-1.90 (m, 2H), 1.76-1.71 (m, 2H), 1.67-1.61 (m, 6H), 1.61-1.56 (m, 2H), 1.55-1.53 (m, 4H), 1.48-1.47 (m, 4H), 1.41-1.20 (m, 52H), 0.95-0.86 (m, 12H).

LC-MS (ESI): (M+H) 893.9.

### Example 28: Synthesis of Amino Lipid Compound 048

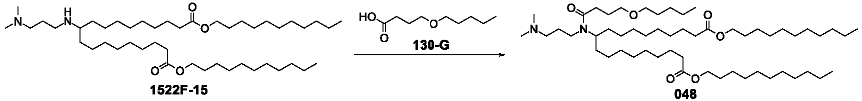

According to the general process, amino lipid compound 048 (0.276 g) was prepared from compound 1522F-15 (0.56 g, 0.76 mmol) and 0.17 g 130-G, as a light yellow oil with a purity of 95.01% and a yield of 40.7%.

¹H NMR (600 MHz, CDCl3) δ 4.07 (t, J = 6.8 Hz, 4H), 3.71-3.62 (m, 1H), 3.48-3.46 (t, J = 6.2 Hz, 2H), 3.42-3.40 (t, J = 6.7 Hz, 2H), 3.16-3.12 (m, 2H), 2.46-2.43 (t, J = 7.2 Hz, 1H), 2.42-2.40 (t, J = 7.3 Hz, 1H), 2.33-2.28 (m, 6H), 2.24 (s, 6H), 2.01-1.89 (m, 2H), 1.77-1.69 (m, 2H), 1.67-1.55 (m, 10H), 1.47-1.46(m, 4H), 1.40-1.21 (m, 56H), 0.93-0.89 (m, 9H).

LC-MS (ESI): (M+H) 893.9.

### Example 29: Synthesis of Amino Lipid Compound 049

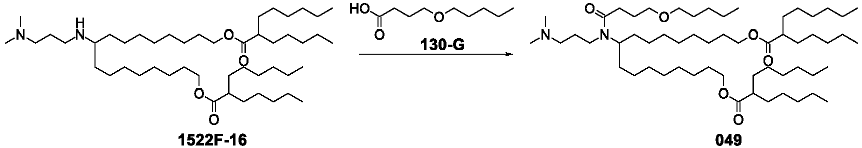

According to the general process, amino lipid compound 049 (0.058 g) was prepared from compound 1522F-16 (0.16 g, 0.21 mmol) and 0.086 g 130-G, as a light yellow oil with a purity of 95.29% and a yield of 30.1%.

¹H NMR (600 MHz, CDCl3) δ 4.09 (m, 4H), 3.73-3.64 (m, 1H), 3.48 (t, J = 6.1 Hz, 2H), 3.42 (t, J = 6.7 Hz, 2H), 3.15 (m, 2H), 2.46 (t, J = 7.3 Hz, 1H), 2.42 (t, J = 7.3 Hz, 1H), 2.38-2.28 (m, 4H), 2.26 (m, 6H), 2.05-1.89 (m, 4H), 1.78-1.71 (m, 2H), 1.68-1.56 (m, 10H), 1.53-1.43 (m, 6H), 1.39-1.18 (m, 52H), 0.96-0.85 (m, 15H).

LC-MS (ESI): (M+H) 922.0.

### Example 30: Synthesis of Amino Lipid Compound 122

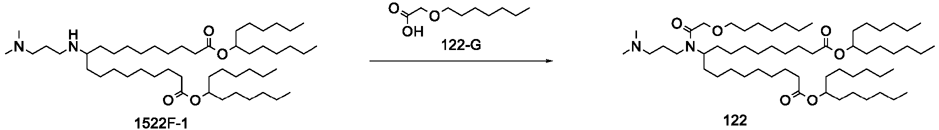

According to the general synthetic process, amino lipid compound 122 (0.51 g) was prepared from compound 1522F-1 (1 g, 1.26 mmol) and 0.33 g 122-G, as a light yellow oil with a purity of 92.94% and a yield of 43%.

¹H NMR in CDCl₃ δ 4.86-4.85 (m, 2H), 4.13-4.09 (m, 2H), 3.55 (m, 1H), 3.50-3.46 (m, 2H), 3.13 (m, 2H), 2.30-2.21 (m, 12H), 1.74-1.45 (m, 20H), 1.25 (m, 60H), 0.88-0.85 (m, 15H).

LC-MS (ESI): (M+H) 950.5.

### Example 31: Synthesis of Amino Lipid Compound 123

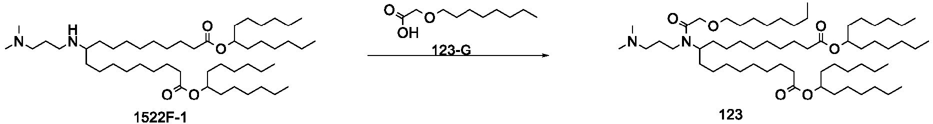

According to the general synthetic process, amino lipid compound 123 (0.88 g) was prepared from compound 1522F-1 (1.3 g, 1.64 mmol) and 0.46 g 123-G, as a light yellow oil with a purity of 90.77% and a yield of 55%.

¹H NMR in CDCl₃ δ 4.87-4.85 (m, 2H), 4.13-4.09 (m, 2H), 3.56-3.53 (m, 1H), 3.50-3.46 (m, 2H), 3.14-3.11 (m, 2H), 2.38-2.22 (m, 12H), 1.77-1.44 (m, 20H), 1.27-1.25 (m, 62H), 0.88-0.85 (m, 15H).

LC-MS (ESI): (M+H) 964.5.

### Example 32: Synthesis of Amino Lipid Compound 126

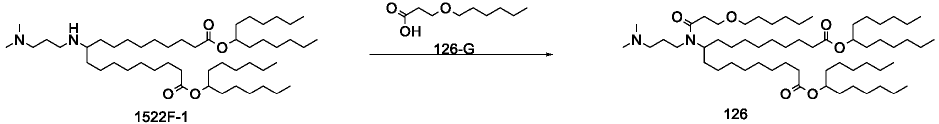

According to the general synthetic process, amino lipid compound 126 (0.83 g) was prepared from compound 1522F-1 (1.3 g, 1.64 mmol) and 0.43 g 126-G, as a light yellow oil with a purity of 93.73% and a yield of 53%.

1H NMR in CDCl₃ δ 4.89-4.84 (m, 2H), 3.76-3.72 (m, 2H), 3.63 (m, 1H), 3.45-3.41 (m, 2H), 3.15-3.10 (m, 2H), 2.62-2.59 (m, 2H), 2.31-2.22 (m, 12H), 1.72-1.26 (m, 78H), 0.89-0.86 (m, 15H).

LC-MS (ESI): (M+H) 950.3.

### Example 33: Synthesis of Amino Lipid Compound 127

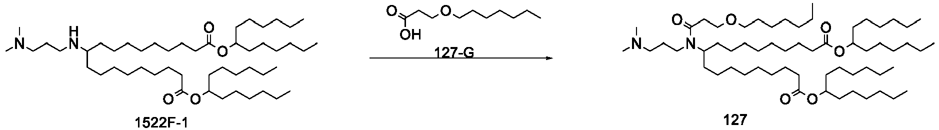

According to the general synthetic process, amino lipid compound 127 (0.75 g) was prepared from compound 1522F-1 (1.3 g, 1.64 mmol) and 0.46 g 127-G, as a light yellow oil with a purity of 95.58% and a yield of 48%.

¹H NMR in CDCl₃ δ 4.88-4.84 (m, 2H), 3.75-3.72 (m, 2H), 3.62 (m, 1H), 3.44-3.40 (m, 2H), 3.14-3.09 (m, 2H), 2.61-2.59 (m, 2H), 2.30-2.24 (m, 6H), 2.21 (s, 6H), 1.70-1.25 (m, 80H), 0.88-0.86 (m, 15H).

LC-MS (ESI): (M+H) 964.4.

### Example 34: Synthesis of Amino Lipid Compound 128

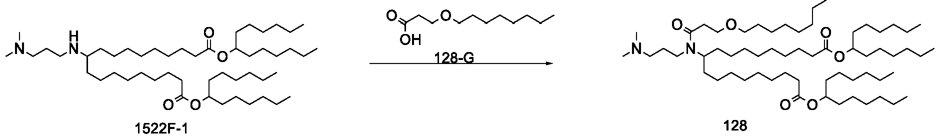

According to the general synthetic process, amino lipid compound 128 (0.76 mg) was prepared from compound 1522F-1 (1.3 g, 1.64 mmol) and 0.49 g 128-G, as a light yellow oil with a purity of 94.70% and a yield of 47%.

¹H NMR in CDCl₃ δ 4.88-4.84 (m, 2H), 3.75-3.72 (m, 2H), 3.62 (m, 1H), 3.44-3.40 (m, 2H), 3.15-3.09 (m, 2H), 2.61-2.59 (m, 2H), 2.29-2.25 (m, 6H), 2.21 (s, 6H), 1.71-1.43 (m, 20H), 1.27-1.25 (m, 62H), 0.88-0.86 (m, 15H).

LC-MS (ESI): (M+H) 978.4.

### Example 35: Synthesis of Amino Lipid Compound 145

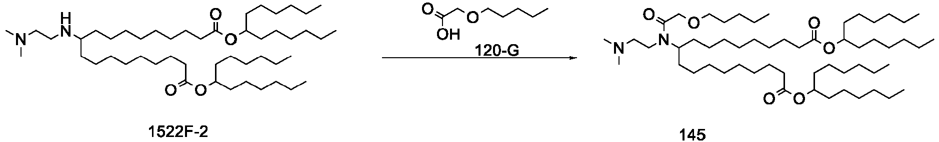

According to the general synthetic process, amino lipid compound 145 (0.24 g) was prepared from compound 1522F-2 (1 g, 1.28 mmol) and 0.28 g 120-G, as a light yellow oil with a purity of 90.77% and a yield of 20%.

¹H NMR (600 MHz, CDCl₃) δ 4.89-4.82 (m, 2H), 4.12 (d, *J* = 34.5 Hz, 2H), 3.59-3.44 (m, 3H), 3.32-3.20 (m, 2H), 2.43 (dd, *J=* 36.8, 28.9 Hz, 2H), 2.32 (s, 4H), 2.26 (td, *J=* 7.6, 3.5 Hz, 6H), 1.60 (dd, *J* = 11.6, 6.9 Hz, 6H), 1.54-1.40 (m, 12H), 1.36-1.12 (m, 56H), 0.88 (dt, *J =* 17.2, 7.0 Hz, 15H).

LC-MS (ESI): (M+H) 908.3

### Example 36: Synthesis of Amino Lipid Compound 146

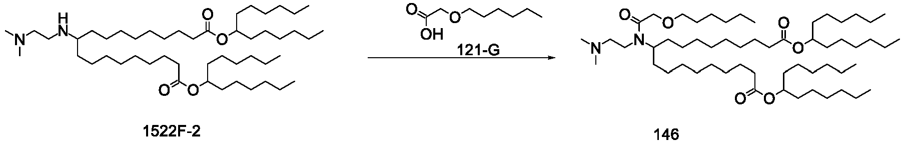

According to the general synthetic process, amino lipid compound 146 (0.55 g) was prepared from compound 1522F-2 (1 g, 1.28 mmol) and 0.31 g 121-G, as a light yellow oil with a purity of 97.28% and a yield of 47%.

¹H NMR (600 MHz, CDCl₃) δ 4.86 (p, *J* = 6.5 Hz, 2H), 4.12 (d, *J* = 34.8 Hz, 2H), 3.59-3.45 (m, 3H), 3.25 (dd, *J* = 14.6, 5.8 Hz, 2H), 2.52-2.37 (m, 2H), 2.33-2.22 (m, 10H), 1.65-1.55 (m, 6H), 1.55-1.39 (m, 12H), 1.38-1.14 (m, 58H), 0.91-0.83 (m, 15H).

LC-MS (ESI): (M+H) 922.3.

### Example 37: Synthesis of Amino Lipid Compound 147

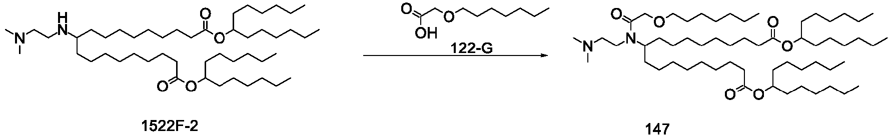

According to the general synthetic process, amino lipid compound 147 (0.92 g) was prepared from compound 1522F-2 (1.3 g, 1.67 mmol) and 0.44 g 122-G, as a light yellow oil with a purity of 92.84% and a yield of 59%.

¹H NMR (600 MHz, CDCl₃) δ 4.89-4.81 (m, 2H), 4.12 (d, *J =* 34.8 Hz, 2H), 3.59-3.42 (m, 3H), 3.24 (dd, *J* = 8.7, 6.6 Hz, 2H), 2.51-2.38 (m, 2H), 2.33-2.22 (m, 10H), 1.64-1.54 (m, 6H), 1.55-1.40 (m, 12H), 1.26 (dd, *J =* 8.2, 4.6 Hz, 60H), 0.87 (t*, J =* 7.0 Hz, 15H).

LC-MS (ESI): (M+H) 936.3.

### Example 38: Synthesis of Amino Lipid Compound 148

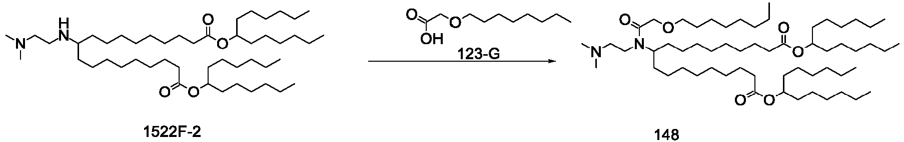

According to the general synthetic process, amino lipid compound 148 (0.85 g) was prepared from compound 1522F-2 (1.3 g, 1.67 mmol) and 0.47g 123-G, as a light yellow oil with a purity of 96.59% and a yield of 54%.

¹H NMR (600 MHz, CDCl₃) δ 4.86 (p, *J* = 6.6 Hz, 2H), 4.12 (d, *J* = 34.9 Hz, 2H), 3.59-3.43 (m, 3H), 3.24 (dd, *J* = 8.8, 6.6 Hz, 2H), 2.49-2.37 (m, 2H), 2.32-2.22 (m, 10H), 1.65-1.54 (m, 6H), 1.54-1.41 (m, 12H), 1.27 (dd, *J =* 15.6, 9.8 Hz, 62H), 0.87 (t, *J =* 7.0 Hz, 15H).

LC-MS (ESI): (M+H) 950.3.

### Example 39: Synthesis of Amino Lipid Compound 150

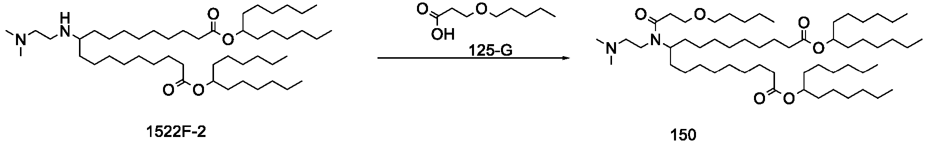

According to the general synthetic process, amino lipid compound 150 (1.0 g) was prepared from compound 1522F-2 (1.3 g, 1.67 mmol) and 0.40 g 125-G, as a light yellow oil with a purity of 96.39% and a yield of 65%.

¹H NMR (600 MHz, CDCl₃) δ 4.89-4.82 (m, 2H), 3.77-3.69 (m, 2H), 3.66-3.56 (m, 1H), 3.46-3.36 (m, 2H), 3.29-3.18 (m, 2H), 2.63-2.56 (m, 2H), 2.49-2.39 (m, 2H), 2.32-2.21 (m, 10H), 1.52 (dd, *J* = 40.8, 21.6, 14.0, 6.9 Hz, 18H), 1.34-1.13 (m, 56H), 0.87 (dt, *J =* 14.0, 7.0 Hz, 15H).

LC-MS (ESI): (M+H) 922.3.

### Example 40: Synthesis of Amino Lipid Compound 151

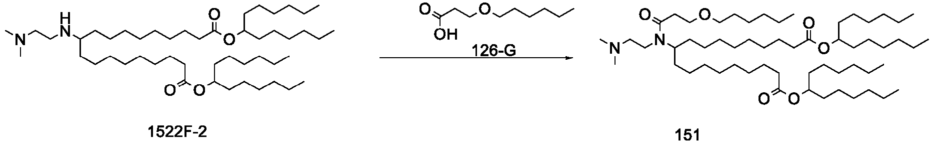

According to the general synthetic process, amino lipid compound 151 (1.0 g) was prepared from compound 1522F-2 (1.3 g, 1.67 mmol) and 0.44 g 126-G, as a light yellow oil with a purity of 95.27% and a yield of 64%.

¹H NMR (600 MHz, CDCl₃) δ 4.89-4.82 (m, 2H), 3.77-3.70 (m, 2H), 3.66-3.57 (m, 1H), 3.46-3.38 (m, 2H), 3.25 (dd, *J =* 10.9, 4.8 Hz, 2H), 2.64-2.56 (m, 2H), 2.44 (dd, *J=* 16.0, 8.7 Hz, 2H), 2.34-2.22 (m, 10H), 1.65-1.37 (m, 18H), 1.27 (dd, *J =* 12.0, 6.0 Hz, 58H), 0.91-0.84 (m, 15H).

LC-MS (ESI): (M+H) 936.3.

### Example 41: Synthesis of Amino Lipid Compound 152

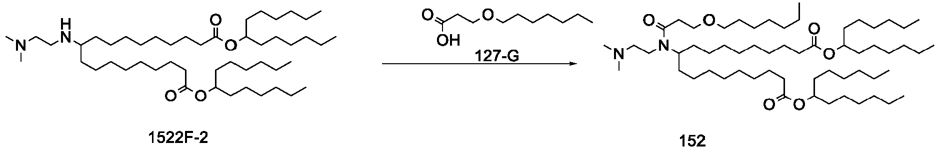

According to the general synthetic process, amino lipid compound 152 (1.0 g) was prepared from compound 1522F-2 (1.3 g, 1.67 mmol) and 0.47 g 127-G, as a light yellow oil with a purity of 95.65% and a yield of 63%.

¹H NMR (600 MHz, CDCl₃) δ 4.89-4.82 (m, 2H), 3.78-3.70 (m, 2H), 3.67-3.56 (m, 1H), 3.48-3.36 (m, 2H), 3.30-3.19 (m, 2H), 2.64-2.56 (m, 2H), 2.43 (dd, *J* = 15.6, 7.5 Hz, 2H), 2.31-2.22 (m, 10H), 1.66-1.36 (m, 18H), 1.35-1.11 (m, 60H), 0.87 (t*, J =* 7.0 Hz, 15H).

LC-MS (ESI): (M+H) 950.4.

### Example 42: Synthesis of Amino Lipid Compound 153

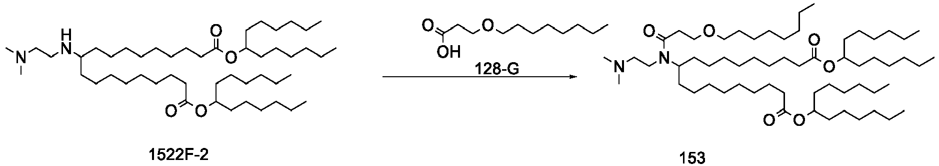

According to the general synthetic process, amino lipid compound 153 (1.07 g) was prepared from compound 1522F-2 (1.5 g, 1.92 mmol) and 0.58 g 128-G, as a light yellow oil with a purity of 93.89% and a yield of 58%.

¹H NMR (600 MHz, CDCl₃) δ 4.90-4.82 (m, 2H), 3.77-3.70 (m, 2H), 3.65-3.57 (m, 1H), 3.47-3.38 (m, 2H), 3.25 (dd, *J =* 9.5, 5.8 Hz, 2H), 2.59 (dd, *J =* 9.2, 5.5 Hz, 2H), 2.51-2.40 (m, 2H), 2.31 (s, 4H), 2.26 (dt, *J =* 11.7, 3.6 Hz, 6H), 1.64-1.36 (m, 18H), 1.26 (d, *J =* 12.4 Hz, 62H), 0.87 (t, *J =* 6.8 Hz, 15H).

LC-MS (ESI): (M+H) 964.4.

### Example 43: Synthesis of Amino Lipid Compound 155

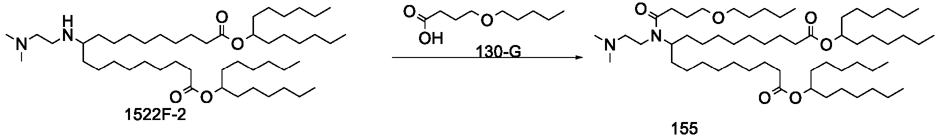

According to the general synthetic process, amino lipid compound 155 (1.0 g) was prepared from compound 1522F-2 (1.3 g, 1.67 mmol) and 0.44 g 130-G, as a light yellow oil with a purity of 93.39% and a yield of 64%.

¹H NMR (600 MHz, CDCl₃) δ 4.89-4.82 (m, 2H), 3.64 (p, *J =* 6.6 Hz, 1H), 3.43 (q, *J =* 6.0 Hz, 2H), 3.38 (t, *J =* 6.7 Hz, 2H), 3.28-3.18 (m, 2H), 2.52-2.34 (m, 4H), 2.34-2.21 (m, 10H), 1.96-1.84 (m, 2H), 1.52 (ddt, *J =* 21.3, 13.8, 6.6 Hz, 18H), 1.35-1.07 (m, 56H), 0.87 (dt, *J =* 7.2, 6.2 Hz, 15H).

LC-MS (ESI): (M+H) 936.4.

### Example 44: Synthesis of Amino Lipid Compound 156

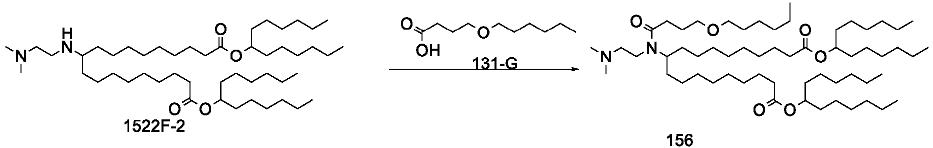

According to the general synthetic process, amino lipid compound 156 (1.0 g) was prepared from compound 1522F-2 (1.5 g, 1.92 mmol) and 0.54 g 131-G, as a light yellow oil with a purity of 96.96% and a yield of 44%.

¹H NMR (600 MHz, CDCl₃) δ 4.86 (p, *J =* 5.7 Hz, 2H), 3.64 (p, *J =* 6.8 Hz, 1H), 3.43 (q, *J* = 6.1 Hz, 2H), 3.38 (t, *J =* 6.7 Hz, 2H), 3.22 (dd, *J* = 15.6, 11.5 Hz, 2H), 2.48-2.34 (m, 4H), 2.32-2.21 (m, 10H), 1.96-1.85 (m, 2H), 1.66-1.37 (m, 18H), 1.34-1.18 (m, 58H), 0.91-0.82 (m, 15H).

LC-MS (ESI): (M+H) 950.3.

### Example 45: Synthesis of Amino Lipid Compound 157

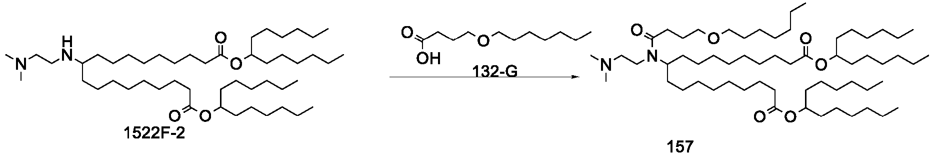

According to the general synthetic process, amino lipid compound 157 (1.1 g) was prepared from compound 1522F-2 (1.5 g, 1.92 mmol) and 0.58 g 132-G, as a light yellow oil with a purity of 90.77% and a yield of 59%.

¹H NMR (600 MHz, CDCl₃) δ 4.89-4.81 (m, 2H), 3.64 (p, *J =* 6.6 Hz, 1H), 3.43 (qd, *J =* 6.0, 1.1 Hz, 2H), 3.38 (t, *J =* 6.7 Hz, 2H), 3.27-3.18 (m, 2H), 2.49-2.34 (m, 4H), 2.32-2.22 (m, 10H), 1.96-1.84 (m, 2H), 1.51 (m, 18H), 1.26 (m, 60H), 0.91-0.81 (m, 15H).

LC-MS (ESI): (M+H) 964.4.

### Example 46: Synthesis of Amino Lipid Compound 158

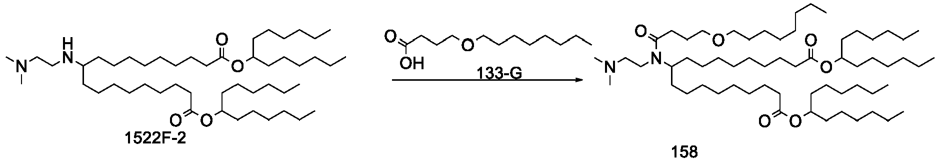

According to the general synthetic process, amino lipid compound 158 (0.96 g) was prepared from compound 1522F-2 (1.3 g, 1.67 mmol) and 0.54 g 133-G, as a light yellow oil with a purity of 97.59% and a yield of 59%.

¹H NMR (600 MHz, CDCl₃) δ 4.85 (m, 2H), 3.68-3.59 (m, 1H), 3.47-3.40 (m, 2H), 3.37 (m, 2H), 3.28-3.19 (m, 2H), 2.50-2.34 (m, 4H), 2.30 (s, 4H), 2.28-2.21 (m, 6H), 1.90 (m, 2H), 1.65-1.37 (m, 18H), 1.21 (m, 62H), 0.92-0.80 (m, 15H).

LC-MS (ESI): (M+H) 978.4.

### Example 47: Synthesis of Amino Lipid Compound 167

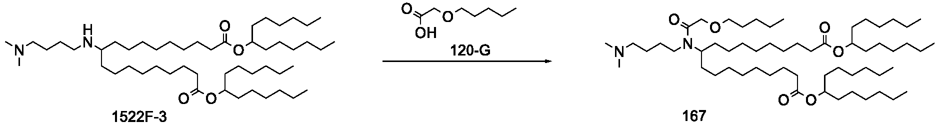

According to the general synthetic process, amino lipid compound 167 (0.45 g) was prepared from compound 1522F-3 (1 g, 1.24 mmol) and 0.27 g 120-G, as a light yellow oil with a purity of 92.68% and a yield of 39%.

¹H NMR (600 MHz, CDCl₃) δ 4.92-4.79 (m, 2H), 4.13 (s, 0.6H), 4.10 (s, 1.4H), 3.61-3.42 (m, 3H), 3.13-3.04 (t, *J =* 6.0 Hz, 2H), 2.35-2.26 (m, 6H), 2.25 (s, 4H), 2.23 (s, 2H), 1.68-1.31 (m, 26H), 1.31-1.16 (m, 52H), 0.93-0.82 (m, 15H).

LC-MS (ESI): (M+H) 936.2.

### Example 48: Synthesis of Amino Lipid Compound 168

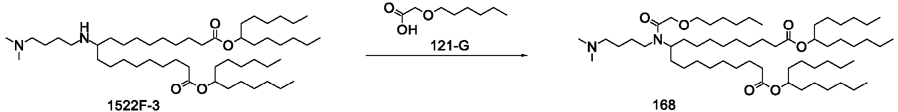

According to the general synthetic process, amino lipid compound 168 (0.65 g) was prepared from compound 1522F-3 (1.3 g, 1.61 mmol) and 0.39 g 121-G, as a light yellow oil with a purity of 91.4% and a yield of 42%.

¹H NMR (600 MHz, CDCl₃) δ 4.97-4.79 (m, 2H), 4.16 (s, 0.6H), 4.14 (s, 1.4H), 3.62-3.44 (m, 3H), 3.11 (t, *J* = 6.0 Hz, 2H), 2.35-2.28 (m, 6H), 2.25 (s, 6H), 1.68-1.35 (m, 24H), 1.42-1.16 (m, 56H), 0.93-0.87 (m, 15H).

LC-MS (ESI): (M+H) 950.1.

### Example 49: Synthesis of Amino Lipid Compound 169

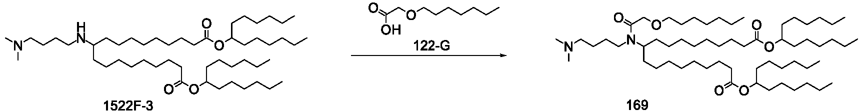

According to the general synthetic process, amino lipid compound 169 (0.58 g) was prepared from compound 1522F-3 (1.3 g, 1.61 mmol) and 0.42 g 122-G, as a light yellow oil with a purity of 91.97% and a yield of 37%.

¹H NMR (600 MHz, CDCl₃) δ 4.93-4.81 (m, 2H), 4.16 (s, 0.6H), 4.13 (s, 1.4H), 3.63-3.48 (m, 3H), 3.16-3.07 (t, *J =* 6.0 Hz, 2H), 2.35-2.28 (m, 6H), 2.26 (s, 6H), 1.69-1.42 (m, 22H), 1.40-1.17 (m, 60H), 0.91 (t, *J =* 6.8 Hz, 15H).

LC-MS (ESI): (M+H) 964.1.

### Example 50: Synthesis of Amino Lipid Compound 170

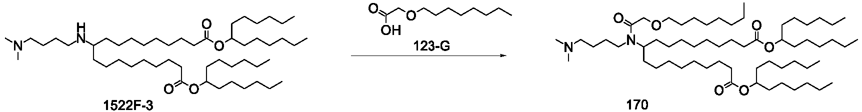

According to the general synthetic process, amino lipid compound 170 (0.41 g) was prepared from compound 1522F-3 (1 g, 1.24 mmol) and 0.35 g 123-G, as a light yellow oil with a purity of 92.56% and a yield of 34%.

¹H NMR (600 MHz, CDCl₃) δ 4.94-4.84 (m, 2H), 4.16 (s, 0.6H), 4.13 (s, 1.4H), 3.62-3.48 (m, 3H), 3.15-3.08 (t, *J =* 6.0 Hz, 2H), 2.38-2.29 (m, 6H), 2.28 (s, 4H), 2.26 (s, 2H), 1.71-1.42 (m, 22H), 1.41-1.16 (m, 62H), 0.91 (t, *J =* 7.0 Hz, 15H).

LC-MS (ESI): (M+H) 978.1.

### Example 51: Synthesis of Amino Lipid Compound 171

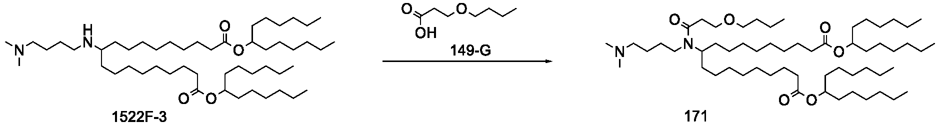

According to the general synthetic process, amino lipid compound 171 (0.52 g) was prepared from compound 1522F-3 (1 g, 1.24 mmol) and 0.27 g 149-G, as a light yellow oil with a purity of 91.21% and a yield of 46%.

¹H NMR (600 MHz, CDCl₃) δ 4.92-4.79 (m, 2H), 3.74 (q, *J* = 7.1 Hz, 2H), 3.66-3.57 (m, 1H), 3.44 (q, *J* = 6.6 Hz, 2H), 3.10-3.06 (m, 2H), 2.60 (t, *J* = 7.0 Hz, 2H), 2.30-2.24 (m, 6H), 2.22 (s, 6H), 1.65-1.33 (m, 22H), 1.32-1.17 (m, 54H), 0.95-0.82 (m, 15H).

LC-MS (ESI): (M+H) 936.1.

### Example 52: Synthesis of Amino Lipid Compound 173

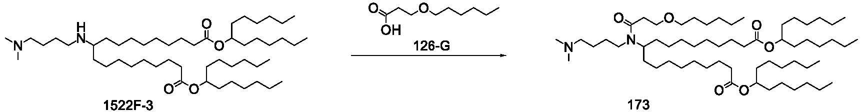

According to the general synthetic process, amino lipid compound 173 (0.42 g) was prepared from compound 1522F-3 (1 g, 1.24 mmol) and 0.32 g 126-G, as a light yellow oil with a purity of 92.16% and a yield of 36%.

¹H NMR (600 MHz, CDCl₃) δ 4.91-4.81 (m, 2H), 3.74 (q, *J* = 7.1 Hz, 2H), 3.66-3.57 (m, 1H), 3.43 (q, *J* = 6.6 Hz, 2H), 3.13-3.01 (m, 2H), 2.60 (t, *J* = 7.0 Hz, 2H), 2.30-2.24 (m, 6H), 2.22 (s, 6H), 1.64-1.37 (m, 22H), 1.35-1.16 (m, 58H), 0.95-0.84 (m, 15H).

LC-MS (ESI): (M+H) 964.1.

### Example 53: Synthesis of Amino Lipid Compound 174

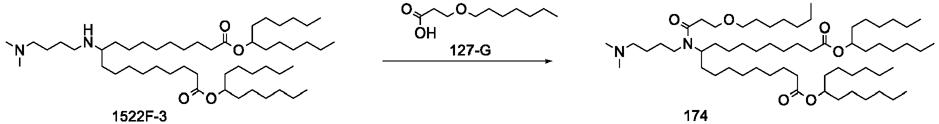

According to the general synthetic process, amino lipid compound 174 (0.53 g) was prepared from compound 1522F-3 (1.3 g, 1.61 mmol) and 0.45 g 127-G, as a light yellow oil with a purity of 97.18% and a yield of 34%.

¹H NMR (600 MHz, CDCl₃) δ 4.92-4.81 (m, 2H), 3.81-3.70 (m, 2H), 3.70-3.61 (m, 1H), 3.51-3.41 (m, 2H), 3.14-3.10 (m, 2H), 2.63 (t, *J* = 7.0 Hz, 2H), 2.58 (t, *J =* 7.1 Hz, 0.5H), 2.47-2.41 (t, *J* = 6.9 Hz, 1.5H), 2.38-2.28 (m, 10H), 1.71-1.40 (m, 22H), 1.38-1.17 (m, 60H), 0.91 (t*, J =* 7.0 Hz, 15H).

LC-MS (ESI): (M+H) 978.2.

### Example 54: Synthesis of Amino Lipid Compound 175

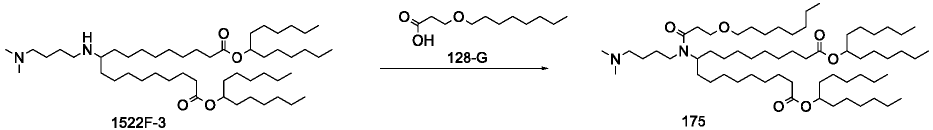

According to the general synthetic process, amino lipid compound 175 (0.6 g) was prepared from compound 1522F-3 (1.3 g, 1.61 mmol) and 0.49 g 128-G, as a light yellow oil with a purity of 91.10% and a yield of 38%.

¹H NMR (600 MHz, CDCl₃) δ 4.89-4.81 (m, 2H), 3.81-3.70 (m, 2H), 3.67-3.55 (m, 1H), 3.47-3.38 (m, 2H), 3.14-3.05 (m, 2H), 2.60 (t, *J* = 7.0 Hz, 2H), 2.54 (t, *J =* 7.1 Hz, 0.5H), 2.43 *(t, J=* 6.9 Hz, 1.5H), 2.36-2.24 (m, 10H), 1.69-1.36 (m, 22H), 1.35-1.10 (m, 62H), 0.88 (t, *J=* 7.0 Hz, 15H).

LC-MS (ESI): (M+H) 992.2.

### Example 55: Synthesis of Amino Lipid Compound 177

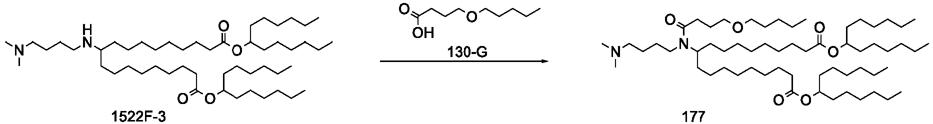

According to the general synthetic process, amino lipid compound 177 (0.6 g) was prepared from compound 1522F-3 (1.3 g, 1.61 mmol) and 0.42 g 130-G, as a light yellow oil with a purity of 92.65% and a yield of 39%.

¹H NMR (600 MHz, CDCl₃) δ 4.94-4.80 (m, 2H), 3.69-3.56 (m, 1H), 3.48-3.41 (m, 2H), 3.41-3.35 (m, 2H), 3.07 (t, *J* =7.0 Hz, 2H), 2.46-2.35 (m, 3H), 2.33-2.23 (m, 11H), 1.96-1.87 (m, 2H), 1.68-1.38 (m, 24H), 1.36-1.14 (m, 54H), 0.96-0.85 (m, 15H).

LC-MS (ESI): (M+H) 964.2.

### Example 56: Synthesis of Amino Lipid Compound 178

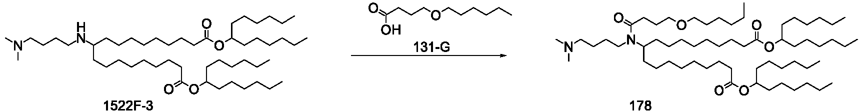

According to the general synthetic process, amino lipid compound 178 (0.43 g) was prepared from compound 1522F-3 (1.3 g, 1.61 mmol) and 0.42 g 131-G, as a light yellow oil with a purity of 94.43% and a yield of 27%.

¹H NMR (600 MHz, CDCl₃) δ 4.90-4.84 (m, 2H), 3.70-3.58 (m, 1H), 3.48-3.43 (m, 2H), 3.43-3.38 (m, 2H), 3.08 (t, *J* =7.0 Hz, 2H), 2.46-2.35 (m, 3H), 2.33-2.23 (m, 11H), 1.96-1.85 (m, 2H), 1.66-1.39 (m, 24H), 1.36-1.10 (m, 56H), 0.92-0.83 (m, 15H).

LC-MS (ESI): (M+H) 978.2.

### Comparative Example 1: Synthesis of Compound 118

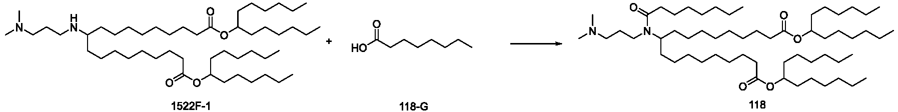

According to the general synthetic process, compound 118 (0.85 g) was prepared from 1.3 g compound 1522F-1 (1.3 g, 1.64 mmol) and 0.31 g 118-G, as a colorless oil with a purity of 90.77% and a yield of 57%.

¹H NMR in (CDCl₃) δ 4.86-4.85 (m, 2H), 3.62 (m, 1H), 3.11-3.09 (m, 2H), 2.30-2.26 (m, 8H), 2.22 (s, 6H), 1.70-1.44 (m, 20H), 1.25 (m, 60H), 0.88-0.86 (m, 15H).

LC-MS (ESI): (M+H) 920.5.

### Biological Tests

Compounds 118 and 1004 used in the biological tests of the present disclosure have the following structures:

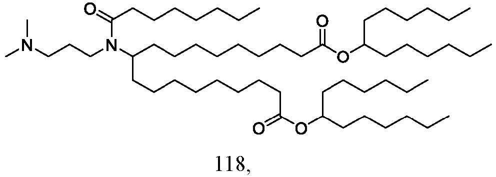

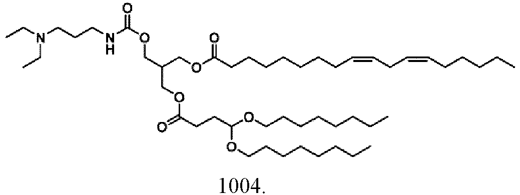

The Compound 118 can be prepared according to the synthetic method of Comparative Example 1, or of Compound 10 (Example 9) described in Chinese patent CN107922364A; and the Compound 1004 can be prepared according to the synthetic method of Compound 7 (Example 7) in CN114127044A.

### Experimental Example 1: Preparation of Lipid Nanoparticle Encapsulating Luciferase mRNA (Fluc mRNA)

### (1) Formulating:

A specified amount of Fluc mRNA stock solution, 0.2 M sodium acetate buffer, and DEPC water were added to a container, and mixed well to obtain a water phase;

The amino lipid compound of the present disclosure, a helper lipid, a structural lipid, and a PEG-lipid were separately dissolved in absolute ethanol to prepare respective solutions at concentrations of 20 mg/mL, 10 mg/mL, 20 mg/mL, and 25 mg/mL, respectively. The above four solutions were pipetted at a molar ratio of the amino lipid compound: DSPC: CHO-HP: PEG2000-DMG of 48:10:40.5:1.5, and mixed well to prepare an alcohol phase.

(2) Encapsulation: The water phase and the alcohol phase were injected into a microfluidic chip at a flow rate of water phase: alcohol phase = 12 mL/min: 4 mL/min by using a microfluidic preparation instrument (MPE-L2), and encapsulation was carried out at a flow rate of water phase: alcohol phase = 9 mL/min: 3 mL/min to obtain mRNA-encapsulating lipid nanoparticle (mRNA-LNP).

(3) Dialysis: The product of step (2) was loaded in a dialysis bag and placed in a Tris Buffer-8% sucrose solution for replacement to remove ingredients such as residual ethanol, unassembled lipids. Dialysis was conducted for 2 h with magnetic stirring at room temperature and under protection from light (dialysate was replaced every 1 hour).

(4) The product of step (3) was sterilized by passing through a 0.22 µm microporous membrane, and then packaged.

Lipid nanoparticle formulations encapsulating Fluc mRNA were prepared, with a concentration of Fluc mRNA of 0.2 µg/µL, a mass ratio of Fluc mRNA to Lipid of 1:10, a particle size of 80-130 nm, and a encapsulation efficiency of 80% or higher.

### Experimental Example 2: Performance Evaluation of in vivo Delivery of Lipid Nanoparticle

Animal preparation: Female BALB/c mice of 6-8 weeks old were selected and raised in an SPF grade breeding room. Animal testing was conducted in strict accordance with the guidelines of national health institutions and animal ethics requirements.

*In vivo* Delivery: prior to injection of the test LNP formulations, the LNP formulations were gently and repeatedly inverted to thoroughly mix the formulation samples. A corresponding amount of the formulation samples were aspirated with a 1 ml insulin syringe, and the LNP formulations were injected by tail vein injection (IV), with 3 mice in duplicate per formulation. Each mouse was injected with 75 µL of the luciferase mRNA (Fluc mRNA)-encapsulating lipid nanoparticle formulation prepared in Experimental Example 1.

6 hours after injection of LNP formulations, mice were injected with 200 µL D-Luciferin luciferase developing substrate (Catalog No.122799; Manufacturer: Perkin Elmer). After the substrate was injected, the mice were anesthetized by injection of 200 µl of 2.5% avertin, and the injection time of luciferase developing substrate was recorded. 10 minutes after the substrate injection, the animals were placed in supine position, and the signal distribution and expression intensity of luciferase in the body and various organs of the animals were observed with In Vivo Imaging System (IVIS).

The fluorescence expression intensity induced by the lipid nanoparticle encapsulating luciferase mRNA (Fluc mRNA) with a representative amino lipid compound is shown in Table 4, with the amino lipid compound 118 as a control.

**Table 4**

| Amino lipid compound | Total flux | Liver | Spleen | (Spleen/Total Flux) * 100% |
|---|---|---|---|---|
| 118 | 2.45E+08 | 9.59E+07 | 2.79E+06 | 1.14% |
| 119 | 2.65E+07 | - | 6.80E+06 | 25.66% |
| 120 | 3.48E+07 | - | 6.46E+06 | 18.56% |
| 121 | 3.07E+07 | - | 7.14E+06 | 23.26% |
| 122 | 3.56E+07 | - | 3.02E+06 | 8.48% |
| 123 | 5.95E+07 | - | 4.02E+06 | 6.76% |
| 124 | 3.45E+07 | - | 6.18E+06 | 17.91% |
| 125 | 5.40E+07 | - | 2.72E+06 | 5.04% |
| 126 | 5.39E+07 | - | 6.87E+06 | 12.75% |
| 127 | 3.56E+07 | - | 4.60E+06 | 12.92% |
| 128 | 5.62E+07 | - | 3.01E+06 | 5.36% |
| 129 | 3.53E+07 | - | 1.33E+07 | 37.68% |
| 130 | 5.73E+07 | - | 1.13E+07 | 19.72% |
| 131 | 3.26E+07 | - | 6.59E+06 | 20.21% |
| 132 | 2.70E+07 | - | 6.10E+06 | 22.59% |
| 133 | 3.58E+07 | - | 1.25E+07 | 34.92% |
| 145 | 1.21E+07 | - | 1.04E+05 | 0.86% |
| 146 | 1.37E+07 | - | 6.65E+04 | 0.49% |
| 147 | 4.97E+06 | - | 1.13E+05 | 2.27% |
| 148 | 4.45E+06 | - | 1.07E+05 | 2.40% |
| 149 | 5.80E+06 | - | 1.29E+05 | 2.22% |
| 150 | 2.06E+07 | 5.51E+06 | 2.27E+05 | 1.10% |
| 151 | 2.29E+07 | 8.91E+06 | 1.30E+05 | 0.57% |
| 156 | 3.07E+07 | 9.75E+06 | 2.34E+05 | 0.76% |
| 157 | 2.80E+07 | 1.07E+07 | 2.26E+05 | 0.81% |
| 166 | 1.25E+07 | 7.74E+06 | 8.07E+06 | 64.56% |
| 167 | 4.02E+07 | 4.42E+06 | 7.64E+06 | 19.00% |
| 168 | 2.68E+07 | 9.12E+06 | 8.95E+06 | 33.40% |
| 169 | 1.17E+07 | 6.59E+06 | 5.11E+06 | 43.68% |
| 170 | 2.89E+07 | 1.34E+07 | 1.07E+07 | 37.02% |
| 171 | 1.15E+07 | 5.33E+06 | 3.97E+06 | 34.52% |
| 172 | 8.64E+07 | 3.10E+07 | 9.69E+06 | 11.22% |
| 173 | 2.07E+07 | 9.15E+06 | 8.03E+06 | 38.79% |
| 174 | 9.59E+07 | 1.62E+07 | 7.28E+06 | 7.59% |
| 175 | 5.15E+07 | 1.57E+07 | 1.21E+07 | 23.50% |
| 177 | 2.52E+07 | 5.04E+06 | 3.23E+06 | 12.82% |
| 178 | 6.68E+07 | 1.15E+07 | 3.95E+06 | 5.91% |

The multiple of spleen delivery/total delivery (Spleen/Total Flux) for fluorescence expression intensity induced by lipid nanoparticle encapsulating luciferase mRNA (Fluc mRNA) with a representative amino lipid compound to Compound 118 is shown in Table 5.

**Table 5**

| Amino lipid compound | Multiple |
|---|---|
| 118 | 1.00 |
| 119 | 22.51 |
| 120 | 16.28 |
| 121 | 20.40 |
| 122 | 7.44 |
| 123 | 5.93 |
| 124 | 15.71 |
| 125 | 4.42 |
| 126 | 11.18 |
| 127 | 11.33 |
| 128 | 4.70 |
| 129 | 33.05 |
| 130 | 17.30 |
| 131 | 17.73 |
| 132 | 19.82 |
| 133 | 30.63 |
| 145 | 0.75 |
| 146 | 0.43 |
| 147 | 1.99 |
| 148 | 2.11 |
| 149 | 1.95 |
| 150 | 0.97 |
| 151 | 0.50 |
| 156 | 0.67 |
| 157 | 0.71 |
| 166 | 56.63 |
| 167 | 16.67 |
| 168 | 29.29 |
| 169 | 38.31 |
| 170 | 32.48 |
| 171 | 30.28 |
| 172 | 9.84 |
| 173 | 34.03 |
| 174 | 6.66 |
| 175 | 20.61 |
| 177 | 11.24 |
| 178 | 5.19 |

As can be seen from Table 5, the amino lipid compounds other than amino lipid compounds 145, 146, 150, 151, 156, 157, showed a stronger delivery preference to spleen, as compared to amino lipid compound 118.

### Experimental Example 3: Evaluation of the delivery efficiency of lipid nanoparticles to immune cell populations in different tissues and organs

C57BL/6 mice (3 mice per group) were intravenously injected with eGFP mRNA encapsulating LNP. After 6 hours, the mice were dissected, and the spleen, axillary lymph nodes, peripheral blood, bone marrow, lungs and liver were collected to prepare single-cell suspensions of various tissues and organs. After blocking with Fc blocking antibodies and surface staining with fluorescent-labeled antibodies, sample analysis was conducted by flow cytometry to analyze the expression levels of eGFP in various immune cell populations to confirm the delivery efficiency of lipid nanoparticles comprising different amino lipid compounds to immune cell populations in different tissues and organs.

The test results are shown in FIGs. 1A to 1F. As can be seen from the drawings, the lipid nanoparticles comprising the amino lipid compounds 130, 131, and 133 have a clear delivery preference to immune cells in various tissues and organs.

In addition to those described in the present disclosure, various modifications to the present disclosure will be apparent to those skilled in the art from the foregoing description. Such modifications are also intended to fall within the scope of the appended claims.

## Claims

1. An amino lipid compound having a structure of formula (I): 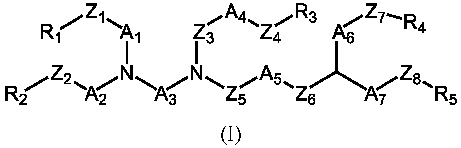
or a pharmaceutically acceptable salt thereof or a stereoisomer thereof,
wherein:
Z₁, Z₂, Z₅, Z₆, Z₇ and Z₈ are each independently -C(=O)-, -CH(OH)-, -C=C-, -C≡C-, -O-, -(C=O)O-, - O(C=O)-, -C(=O)S-, -SC(=O)-, -S-S-, or a bond;
Z₃ is -C(=O)- or a bond;
Z₄ is -O-, or -CH (OH)-;
A₁, A₂, A₃, A₄, A₅, A₆ and A₇ are each independently C₁-C₁₂ hydrocarbylene, cyclohydrocarbyl, phenyl, heterocycle, or a bond;
R₁ and R₂ are each independently H or C₁-C₁₈ hydrocarbyl, or cyclohydrocarbyl, phenyl, heterocycle; or R₁ and R₂, together with the nitrogen atom to which they are attached, form 4- to 7-membered heterocycle;
R₃ is H or C₁-C₁₈ hydrocarbyl, or cyclohydrocarbyl, phenyl, heterocycle; and
R₄ and R₅ are each independently C₁-C₂₄ hydrocarbyl, or cyclohydrocarbyl, phenyl, heterocycle;
preferably, A₅, Z₅ and Z₆ are bonds.

2. The amino lipid compound according to claim 1, or a pharmaceutically acceptable salt thereof or a stereoisomer thereof, wherein:
A₁ and A₂ are each independently C₁-C₈ hydrocarbylene or a bond, such as C₁-C₆ hydrocarbylene or a bond, or C₁-C₄ hydrocarbylene or a bond;
preferably, A₁ and A₂ are each independently C₁-C₈ alkylene, C₂-C₈ alkenylene, or C₂-C₈ alkynylene;
preferably, A₁ and A₂ are each independently C₁-C₈ alkylene, such as C₁-C₆ alkylene, C₁-C₄ alkylene, C₂-C₄ alkylene, or C₁-C₃ alkylene;
preferably, both A₁ and A₂ are bonds;
and/or
A₃ is C₁-C₁₂ hydrocarbylene or a bond;
preferably, A₃ is straight C₁-C₁₂ hydrocarbylene;
preferably, A₃ is straight C₁-C₁₂ alkylene, C₂-C₁₂ alkenylene, or C₂-C₁₂ alkynylene;
preferably, A₃ is straight C₁-C₅ alkylene, such as straight C₁, C₂, C₃, C₄, or C₅ alkylene;
preferably, A₃ is -CH₂-, -CH₂CH₂-, -CH₂CH₂CH₂-, or -CH₂CH₂CH₂CH₂-;
and/or
A₄ is C₁-C₁₂ hydrocarbylene or a bond;
preferably, A₄ is straight C₁-C₁₂ hydrocarbylene;
preferably, A₄ is straight C₁-C₁₂ alkylene, C₂-C₁₂ alkenylene, or C₂-C₁₂ alkynylene;
preferably, A₄ is straight C₁-C₇ alkylene, such as straight C₁, C₂, C₃, C₄, C₅, C₆, or C₇ alkylene;
preferably, A₄ is straight C₁-C₃ alkylene, such as straight C₁, C₂, or C₃ alkylene;
preferably, A₄ is -CH₂-, -CH₂CH₂-, or -CH₂CH₂CH₂-;
and/or
A₅ is C₁-C₈ hydrocarbylene or a bond, such as C₁-C₆ hydrocarbylene or a bond, C₁-C₄ hydrocarbylene or a bond, or C₁-C₃ hydrocarbylene or a bond;
preferably, A₅ is C₁-C₈ alkylene, C₂-C₈ alkenylene, or C₂-C₈ alkynylene;
preferably, A₅ is C₁-C₈ alkylene, such as C₁-C₈ alkylene, C₁-C₆ alkylene, C₁-C₄ alkylene, C₂-C₄ alkylene, or C₁-C₃ alkylene;
preferably, A₅ is a bond;
and/or
A₆ and A₇ are each independently C₁-C₁₂ hydrocarbylene;
preferably, A₆ and A₇ are each independently straight C₁-C₁₂ hydrocarbylene;
preferably, A₆ and A₇ are each independently straight C₁-C₁₂ alkylene, C₂-C₁₂ alkenylene, or C₂-C₁₂ alkynylene;
preferably, A₆ and A₇ are each independently straight C₁-C₁₂ alkylene, such as straight C₂-C₁₂ alkylene, C₄-C₁₀ alkylene, C₆-C₈ alkylene, C₆-C₁₀ alkylene, or C₄-C₈ alkylene;
preferably, A₆ and A₇ are each independently straight C₆-C₁₀ alkylene, such as straight C₆, C₇, C₈, C₉, or C₁₀ alkylene;
preferably, A₆ and A₇ are each independently -(CH₂)₆-, -(CH₂)₇-, -(CH₂)₈-, or -(CH₂)₉-;
and/or
R₁ and R₂ are each independently H; C₁-C₁₈ hydrocarbyl, such as C₁-C₁₆ hydrocarbyl, C₁-C₁₂ hydrocarbyl, C₁-C₁₀ hydrocarbyl, C₁-C₈ hydrocarbyl, C₁-C₆ hydrocarbyl, or C₁-C₄ hydrocarbyl; C₃-C₇ cyclohydrocarbyl, such as C₃-C₆ cyclohydrocarbyl or C₅-C₆ cyclohydrocarbyl; phenyl; or 3- to 7-membered heterocycle, such as 4- to 6-membered heterocycle; or R₁ and R₂, together with the nitrogen atom to which they are attached, form 4- to 7-membered heterocycle, such as 5- to 6-membered heterocycle;
preferably, R₁ and R₂ are each independently H; C₁-C₁₈ alkylene, C₂-C₁₈ alkenylene, or C₂-C₁₈ alkynylene;
preferably, R₁ and R₂ are each independently C₁-C₁₈ alkyl, such as C₁-C₄ alkyl; C₃-C₆ cycloalkyl, such as cyclopropyl, cyclobutyl, cyclopentyl, or cyclohexyl; phenyl; or 5- to 6-membered heterocycle; or R₁ and R₂, together with the nitrogen atom to which they are attached, form 5- to 6-membered heterocycle, such as pyrrolidine, piperidine, piperazine, or morpholine;
preferably, R₁ and R₂ are each independently C₁-C₄ alkyl, such as C₁, C₂, C₃, or C₄ alkyl;
preferably, R₁ and R₂ are each independently methyl, ethyl, n-propyl, isopropyl, n-butyl, sec-butyl, isobutyl, or tert-butyl;
preferably, R₁ and R₂ are each independently C₁-C₃ alkyl, such as C₁, C₂, or C₃ alkyl;
preferably, R₁ and R₂ are each independently methyl, ethyl, or n-propyl;
and/or
R₃ is H; C₁-C₁₈ hydrocarbyl, such as C₁-C₁₆ hydrocarbyl, C₂-C₁₂ hydrocarbyl, C₄-C₁₀ hydrocarbyl, or C₄-C₈ hydrocarbyl; C₃-C₇ cyclohydrocarbyl, such as C₃-C₆ cyclohydrocarbyl or C₅-C₆ cyclohydrocarbyl; phenyl; or 3- to 7-membered heterocycle, such as 4- to 6-membered heterocycle;
preferably, R₃ is C₁-C₁₈ alkyl, C₂-C₁₈ alkenyl, or C₂-C₁₈ alkynyl;
preferably, R₃ is C₁-C₁₈ alkyl, such as C₂-C₁₀ alkyl, C₄-C₈ alkyl; C₃-C₆ cycloalkyl, such as cyclopropyl, cyclobutyl, cyclopentyl, or cyclohexyl; phenyl; or 5- to 6-membered heterocycle;
preferably, R₃ is straight C₄-C₈ alkyl, such as straight C₄, C₅, C₆, C₇, or C₈ alkyl;
preferably, R₃ is n-butyl, n-pentyl, n-hexyl, n-heptyl, or n-octyl;
and/or
Z₁, Z₂, Z₅ and Z₆ are each independently -C(=O)-, -O(C=O)-, -O-, or a bond;
preferably, Z₁, Z₂, Z₅ and Z₆ are each independently a bond;
and/or
Z₃ is -C(=O)-;
and/or
Z₄ is -O-, or Z₄ is -CH(OH)-;
and/or
Z₇ and Z₈ are each independently -(C=O)O-, -O(C=O)-, or a bond.

3. The amino lipid compound according to claim 1 or 2, or a pharmaceutically acceptable salt thereof or a stereoisomer thereof, wherein one of Z₇ or Z₈ is -(C=O)O-, -O(C=O)-, or a bond.

4. The amino lipid compound according to any one of claims 1 to 3, or a pharmaceutically acceptable salt thereof or a stereoisomer thereof, wherein each of Z₇ and Z₈ is -(C=O)O-;
preferably, the (C=O) in Z₇ is attached to A₆, and the (C=O) in Z₈ is attached to A₇.

5. The amino lipid compound according to any one of claims 1 to 3, or a pharmaceutically acceptable salt thereof or a stereoisomer thereof, wherein each of Z₇ and Z₈ is -O(C=O)-;
preferably, the (C=O) in Z₇ is attached to R₄, and the (C=O) in Z₈ is attached to R₅.

6. The amino lipid compound according to any one of claims 1 to 3, or a pharmaceutically acceptable salt thereof or a stereoisomer thereof, wherein one of Z₇ or Z₈ is a bond;
preferably, wherein:
Z₇ is a bond, and Z₈ is -(C=O)O-; preferably, the (C=O) in Z₈ is attached to A₇; or
Z₇ is a bond, and Z₈ is -O(C=O)-; preferably, the (C=O) in Z₈ is attached to R₅; or
Z₈ is a bond, and Z₇ is -(C=O)O-; preferably, the (C=O) in Z₇ is attached to A₆; or
Z₈ is a bond, and Z₇ is -O(C=O)-; preferably, the (C=O) in Z₇ is attached to R₄.

7. The amino lipid compound according to any one of claims 1 to 3, or a pharmaceutically acceptable salt thereof or a stereoisomer thereof, wherein each of Z₇ and Z₈ is a bond.

8. The amino lipid compound according to any one of claims 1 to 7, wherein R₄ and R₅ are each independently C₁-C₂₀ hydrocarbyl; C₃-C₇ cyclohydrocarbyl, such as C₃-C₆ cyclohydrocarbyl or C₅-C₆ cyclohydrocarbyl; phenyl; or 3- to 7-membered heterocycle, such as 4- to 6-membered heterocycle;
preferably, R₄ and R₅ are each independently C₁-C₂₀ hydrocarbyl; C₃-C₆ cycloalkyl, such as cyclopropyl, cyclobutyl, cyclopentyl, or cyclohexyl; phenyl; or 5- to 6-membered heterocycle;
preferably, R₄ and R₅ are each independently C₁-C₂₀ hydrocarbyl.

9. The amino lipid compound according to any one of claims 1 to 8, or a pharmaceutically acceptable salt thereof or a stereoisomer thereof, wherein R₄ and R₅ are each independently C₁-C₂₀ alkyl.

10. The amino lipid compound according to any one of claims 1 to 9, or a pharmaceutically acceptable salt thereof or a stereoisomer thereof, wherein R₄ and R₅ are each independently branched C₃-C₂₀ alkyl;
preferably, R₄ and R₅ are each independently branched C₅-C₂₀ alkyl, branched C₇-C₁₉ alkyl, branched C₉-C₁₈ alkyl, branched C₁₀-C₁₈ alkyl, branched C₃-C₁₀ alkyl, branched C₁₁-C₁₇ alkyl, or branched C₁₈-C₂₀ alkyl;
preferably, R₄ and R₅ are each independently branched C₁₁-C₁₇ alkyl, such as branched C₁₁, C₁₂, C₁₃, C₁₄, C₁₅, C₁₆, or C₁₇ alkyl;
preferably, R₄ and R₅ are each independently branched C₁₁-C₁₃ alkyl, such as branched C₁₁, C₁₂, or C₁₃ alkyl;
preferably, R₄ is a branched alkyl group wherein branching occurs at the α, β, or γ position relative to Z₇; R₅ is a branched alkyl group wherein branching occurs at the α, β, or γ position relative to Zs;
preferably, R₄ is a branched alkyl group wherein branching occurs at the α or β position relative to Z₇; R₅ is a branched alkyl group wherein branching occurs at the α or β position relative to Zs;
preferably, R₄ and R₅ are each independently one of the following structures:

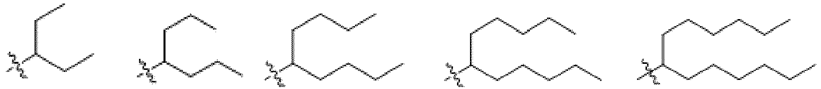

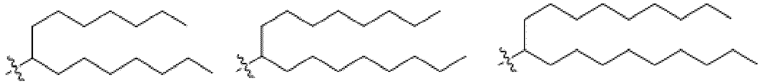

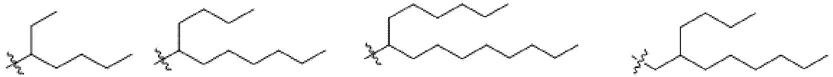

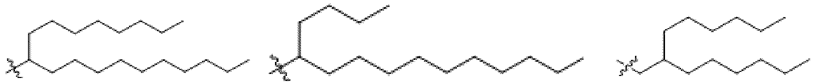

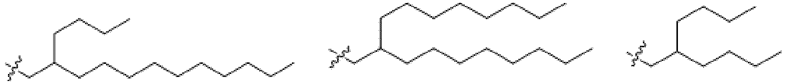

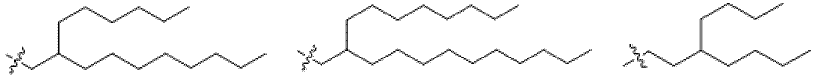

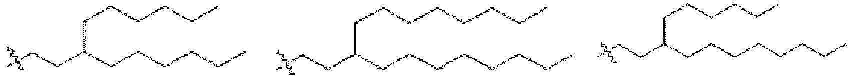

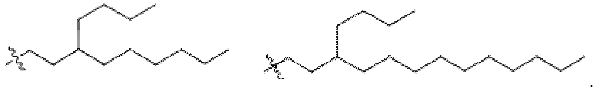

11. The amino lipid compound according to any one of claims 1 to 10, or a pharmaceutically acceptable salt thereof or a stereoisomer thereof, wherein R₄ and R₅ are each independently one of the following structures:

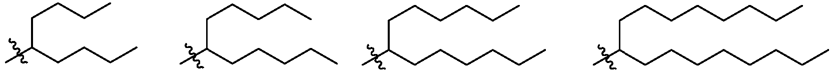

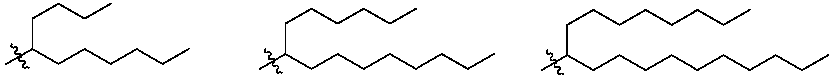

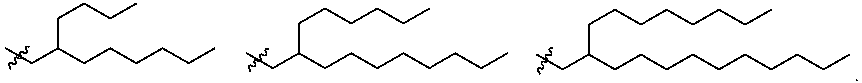

12. The amino lipid compound according to any one of claims 1 to 11, or a pharmaceutically acceptable salt thereof or a stereoisomer thereof, wherein R₄ and R₅ are each independently 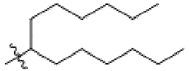 or

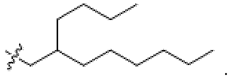

13. The amino lipid compound according to any one of claims 1 to 12, or a pharmaceutically acceptable salt thereof or a stereoisomer thereof, wherein:
Z₃ is -C(=O)-; and
A₄ is C₁-C₁₂ alkylene;
preferably, A₄ is straight C₁-C₁₂ alkylene, such as straight C₁-C₁₀ alkylene, C₁-C₈ alkylene, C₁-C₆ alkylene, C₁-C₄ alkylene, or C₁-C₃ alkylene;
preferably, A₄ is straight C₁-C₄ alkylene, such as straight C₁, C₂, C₃, or C₄ alkylene;
preferably, A₄ is -CH₂-, -CH₂CH₂-, -CH₂CH₂CH₂-, or -CH₂CH₂CH₂CH₂-;
and/or
Z₄ is -O-; or Z₄ is -CH(OH)-.

14. The amino lipid compound of any one of claims 1 to 13, having a structure of formula (II) or formula (III): 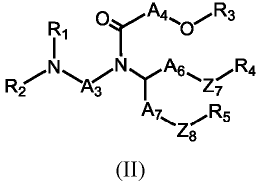 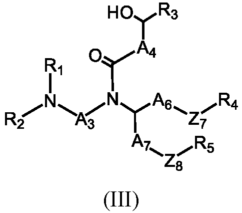
or a pharmaceutically acceptable salt thereof or a stereoisomer thereof,
wherein:
Z₇ and Z₈ are each independently -C(=O)-, -CH(OH)-, -C=C-, -C≡C-, -O-, -(C=O)O-, -O(C=O)-, - C(=O)S-, -SC(=O)-, -S-S-, or a bond;
A₃, A₄, A₆ and A₇ are each independently C₁-C₁₂ hydrocarbylene, cyclohydrocarbyl, phenyl, heterocycle, or a bond;
R₁ and R₂ are each independently H or C₁-C₁₈ hydrocarbyl, or cyclohydrocarbyl, phenyl, heterocycle; or R₁ and R₂, together with the nitrogen atom to which they are attached, form 4- to 7-membered heterocycle;
R₃ is H or C₁-C₁₈ hydrocarbyl, or cyclohydrocarbyl, phenyl, heterocycle; and
R₄ and R₅ are each independently C₁-C₂₄ hydrocarbyl, or cyclohydrocarbyl, phenyl, heterocycle.

15. The amino lipid compound according to claim 14, or a pharmaceutically acceptable salt thereof or a stereoisomer thereof, wherein:
Z₇ and Z₈ are each independently -(C=O)O-, or -O(C=O)-;
preferably, each of Z₇ and Z₈ is -(C=O)O-; preferably, the (C=O) in Z₇ is attached to A₆, and the (C=O) in Z₈ is attached to A₇;
or, each of Z₇ and Z₈ is -O(C=O)-; preferably, the (C=O) in Z₇ is attached to R₄, and the (C=O) in Z₈ is attached to R₅.

16. The amino lipid compound according to any one of claim 14 or 15, or a pharmaceutically acceptable salt thereof or a stereoisomer thereof, wherein A₃, A₄, A₆ and A₇ are each independently C₁-C₁₂ alkylene;
preferably, A₃, A₄, A₆ and A₇ are each independently C₁, C₂, C₃, C₄, C₅, C₆, C₇, C₈, C₉, C₁₀, C₁₁, or C₁₂ alkylene;
preferably, A₃ is straight C₁, C₂, C₃, C₄, C₅, C₆, C₇, C₈, C₉, C₁₀, C₁₁, or C₁₂ alkylene;
preferably, A₃ is straight C₁, C₂, C₃, C₄, or C₅ alkylene;
preferably, A₃ is straight C₂, C₃, or C₄ alkylene, such as -CH₂CH₂-, -CH₂CH₂CH₂-, or -CH₂CH₂CH₂CH₂-;
and/or
preferably, A₄ is straight C₁, C₂, C₃, C₄, C₅, C₆, C₇, C₈, C₉, C₁₀, C₁₁, or C₁₂ alkylene;
preferably, A₄ is straight C₁, C₂, C₃, or C₄ alkylene, such as -CH₂-, -CH₂CH₂-, or -CH₂CH₂CH₂-;
and/or
preferably, A₆ and A₇ are each independently straight C₁, C₂, C₃, C₄, C₅, C₆, C₇, C₈, C₉, C₁₀, C₁₁, or C₁₂ alkylene;
preferably, A₆ and A₇ are each independently straight C₆, C₇, C₈, C₉, or C₁₀ alkylene, such as -(CH₂)₆-, -(CH₂)₇-, -(CH₂)₈-, or -(CH₂)₉-.

17. The amino lipid compound according to any one of claims 14 to 16, or a pharmaceutically acceptable salt thereof or a stereoisomer thereof, wherein R₁ and R₂ are each independently C₁-C₁₈ alkyl;
preferably, R₁ and R₂ are each independently C₁-C₆ alkyl;
preferably, R₁ and R₂ are each independently methyl, ethyl, n-propyl, or isopropyl;
preferably, R₁ and R₂ are each independently C₁-C₄ alkyl, such as C₁, C₂, C₃, or C₄ alkyl;
preferably, R₁ and R₂ are each independently methyl;
or
R₁ and R₂, together with the nitrogen atom to which they are attached, form 4- to 7-membered heterocycle;
preferably, R₁ and R₂, together with the nitrogen atom to which they are attached, form 5- to 6-membered heterocycle.

18. The amino lipid compound according to any one of claims 14 to 17, or a pharmaceutically acceptable salt thereof or a stereoisomer thereof, wherein R₃ is C₁-C₁₈ alkyl;
preferably, R₃ is C₁, C₂, C₃, C₄, C₅, C₆, C₇, C₈, C₉, C₁₀, C₁₁, or C₁₂ alkyl;
preferably, R₃ is straight C₂, C₃, C₄, C₅, C₆, C₇, C₈, C₉, or C₁₀ alkyl;
preferably, R₃ is straight C₄, C₅, C₆, C₇, or C₈ alkyl, such as n-butyl, n-pentyl, n-hexyl, n-heptyl, or n-octyl.

19. The amino lipid compound according to any one of claims 14 to 18, or a pharmaceutically acceptable salt thereof or a stereoisomer thereof, wherein R₄ and R₅ are each independently C₁-C₂₀ hydrocarbyl;
preferably, R₄ and R₅ are each independently C₁-C₂₀ alkyl;
preferably, R₄ and R₅ are each independently branched C₃-C₂₀ alkyl;
preferably, R₄ and R₅ are each independently branched C₅-C₂₀ alkyl, branched C₇-C₁₉ alkyl, branched C₉-C₁₈ alkyl, branched C₁₀-C₁₈ alkyl, branched C₃-C₁₀ alkyl, branched C₁₁-C₁₇ alkyl, or branched C₁₈-C₂₀ alkyl;
preferably, R₄ and R₅ are each independently branched C₁₁-C₁₇ alkyl, such as branched C₁₁, C₁₂, C₁₃, C₁₄, C₁₅, C₁₆, or C₁₇ alkyl;
preferably, R₄ and R₅ are each independently branched C₁₁-C₁₃ alkyl, such as branched C₁₁, C₁₂, or C₁₃ alkyl;
preferably, R₄ is a branched alkyl group wherein branching occurs at the α, β, or γ position relative to Z₇; R₅ is a branched alkyl group wherein branching occurs at the α, β, or γ position relative to Zs;
preferably, R₄ is a branched alkyl group wherein branching occurs at the α or β position relative to Z₇; R₅ is a branched alkyl group whose branching occurs at the α or β position relative to Zs;
preferably, R₄ and R₅ are each independently one of the following structures:

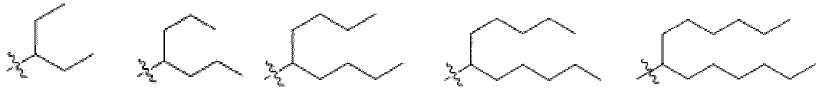

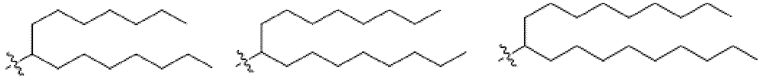

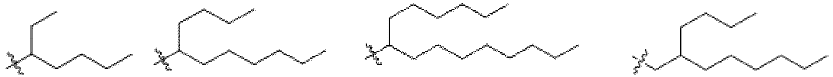

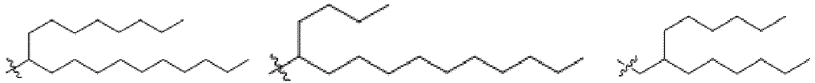

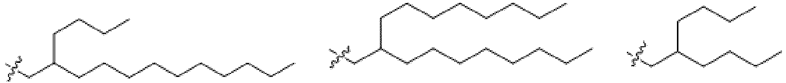

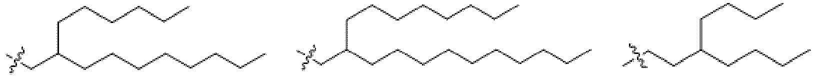

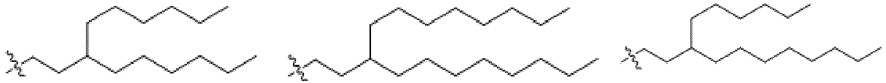

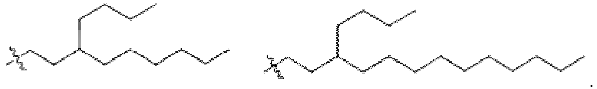

20. The amino lipid compound according to any one of claims 14 to 19, or a pharmaceutically acceptable salt thereof or a stereoisomer thereof, wherein R₄ and R₅ are each independently one of the following structures:

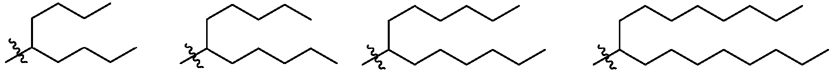

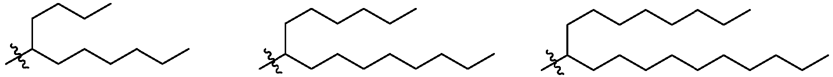

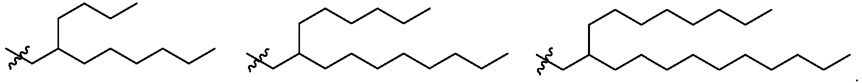

21. The amino lipid compound according to any one of claims 14 to 20, or a pharmaceutically acceptable salt thereof or a stereoisomer thereof, wherein R₄ and R₅ are each independently 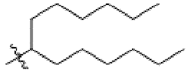 or

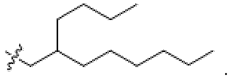

22. The amino lipid compound according to any one of claims 14 to 21, or a pharmaceutically acceptable salt thereof or a stereoisomer thereof, wherein:
Z₇ and Z₈ are each independently -(C=O)O- or -O(C=O)-;
A₃ is straight C₁-C₅ alkylene;
A₄ is straight C₁-C₄ alkylene;
A₆ and A₇ are each independently C₅-C₁₁ alkylene;
R₁ and R₂ are each independently C₁-C₄ alkyl;
R₃ is straight C₂-C₁₀ alkyl; and
R₄ and R₅ are each independently C₁-C₂₄ alkyl.

23. The amino lipid compound according to claim 22, or a pharmaceutically acceptable salt thereof or a stereoisomer thereof, wherein:
each of Z₇ and Z₈ is -(C=O)O-, and the (C=O) in Z₇ is attached to A₆, and the (C=O) in Z₈ is attached to A₇; or each of Z₇ and Z₈ is -O(C=O)-, and the (C=O) in Z₇ is attached to R₄, and the (C=O) in Z₈ is attached to R₅;
and/or
A₃ is straight C₁-C₄ alkylene;
preferably, A₃ is straight C₂-C₄ alkylene, such as straight C₂, C₃, or C₄ alkylene;
preferably, A₃ is -CH₂CH₂-, -CH₂CH₂CH₂-, or -CH₂CH₂CH₂CH₂-;
and/or
A₄ is straight C₁-C₃ alkylene, such as straight C₁, C₂, or C₃ alkylene;
preferably, A₄ is -CH₂-, -CH₂CH₂-, or -CH₂CH₂CH₂-;
and/or
preferably, A₆ and A₇ are each independently straight C₅-C₁₁ alkylene;
preferably, A₆ and A₇ are each independently straight C₆-C₁₀ alkylene, such as straight C₆, C₇, C₈, C₉, or C₁₀ alkylene;
preferably, A₆ and A₇ are each independently straight C₇, C₈, or C₉ alkylene, such as -(CH₂)₇-, -(CH₂)₈-, or -(CH₂)₉-;
and/or
R₁ and R₂ are each independently C₁, C₂, C₃, or C₄ alkyl;
preferably, R₁ and R₂ are each independently methyl, ethyl, n-propyl, or isopropyl;
preferably, R₁ and R₂ are each independently methyl;
and/or
R₃ is straight C₄-C₈ alkyl, such as C₄, C₅, C₆, C₇, or C₈ alkyl;
preferably, R₃ is n-butyl, n-pentyl, n-hexyl, n-heptyl, or n-octyl;
and/or
R₄ and R₅ are each independently branched C₃-C₂₀ alkyl, such as branched C₅-C₂₀ alkyl, branched C₇-C₁₉ alkyl, branched C₉-C₁₈ alkyl, branched C₁₀-C₁₈ alkyl, branched C₃-C₁₀ alkyl, branched C₁₁-C₁₇ alkyl, or branched C₁₈-C₂₀ alkyl;
preferably, R₄ and R₅ are each independently branched C₁₁-C₁₇ alkyl, such as branched C₁₁, C₁₂, C₁₃, C₁₄, C₁₅, C₁₆, or C₁₇ alkyl;
preferably, R₄ and R₅ are each independently branched C₁₁-C₁₃ alkyl, such as branched C₁₁, C₁₂, or C₁₃ alkyl;
preferably, R₄ is a branched C₃-C₂₀ alkyl group wherein branching occurs at the α, β, or γ position relative to Z₇; R₅ is a branched C₃-C₂₀ alkyl group wherein branching occurs at the α, β, or γ position relative to Zs;
preferably, R₄ is a branched C₃-C₂₀ alkyl group wherein branching occurs at the α or β position relative to Z₇; R₅ is a branched C₃-C₂₀ alkyl group wherein branching occurs at the α or β position relative to Z₈;
preferably, R₄ and R₅ are each independently one of the following structures: 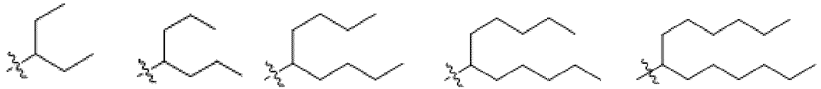 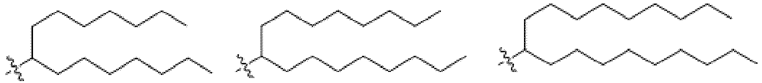 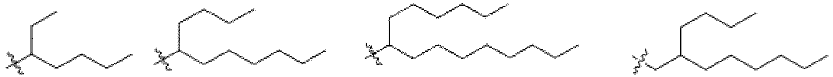 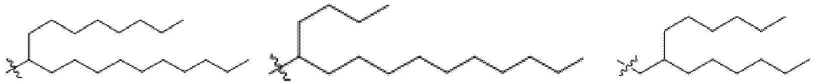 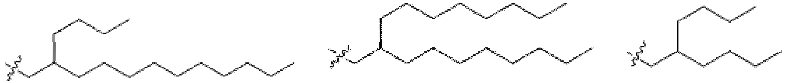 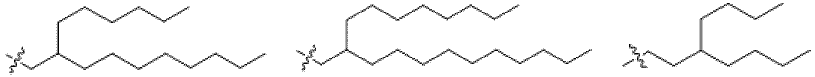 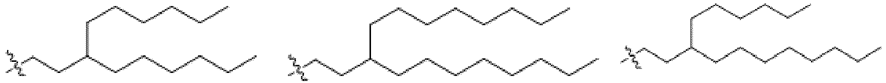 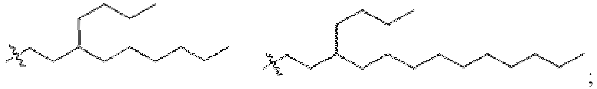
preferably, R₄ and R₅ are each independently one of the following structures: 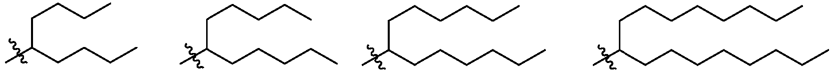 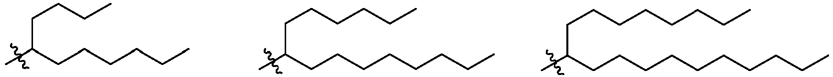 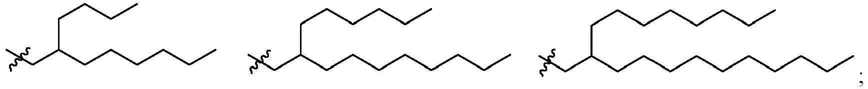
preferably, R₄ and R₅ are each independently

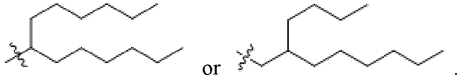

24. The amino lipid compound according to any one of claims 1 to 23, or a pharmaceutically acceptable salt thereof or a stereoisomer thereof, wherein the amino lipid compound has one of the structures shown below:
| Amino lipid compounds | Structural formulae |
|---|---|
| 036 | 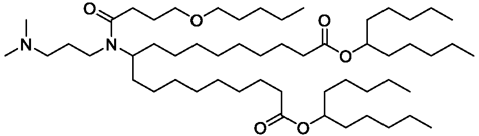 |
| 037 | 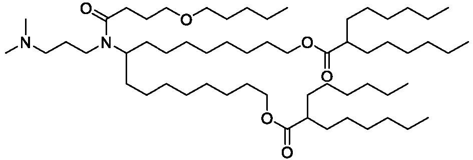 |
| 038 | 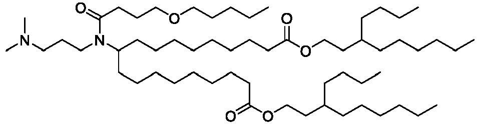 |
| 039 | 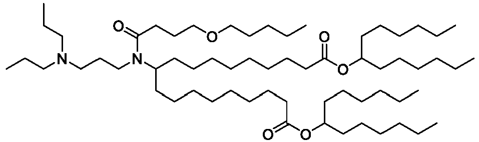 |
| 040 | 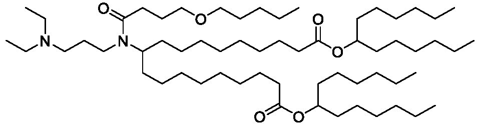 |
| 041 | 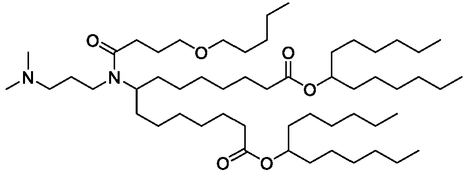 |
| 042 | 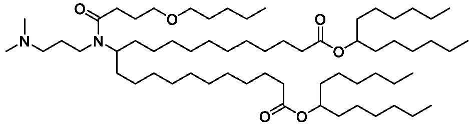 |
| 044 | 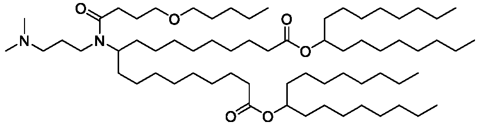 |
| 045 | 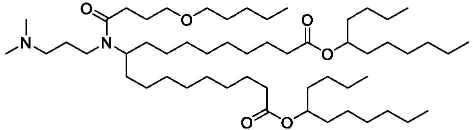 |
| 046 | 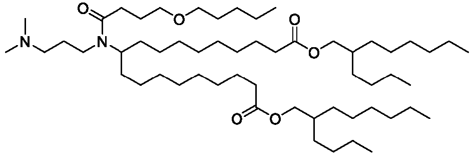 |
| 047 | 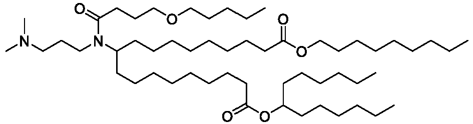 |
| 048 | 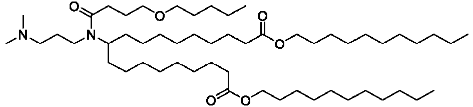 |
| 049 | 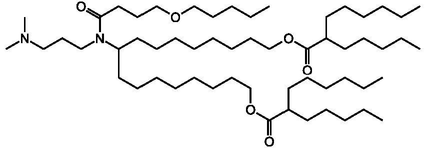 |
| 119 | 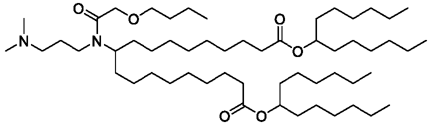 |
| 120 | 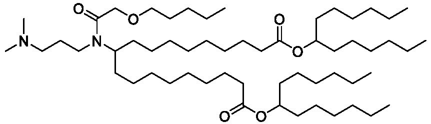 |
| 121 | 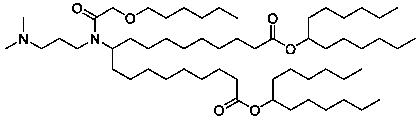 |
| 122 | 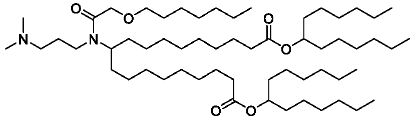 |
| 123 | 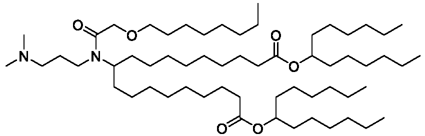 |
| 124 | 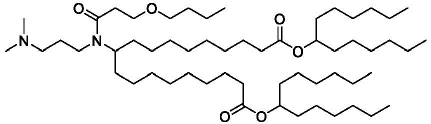 |
| 125 | 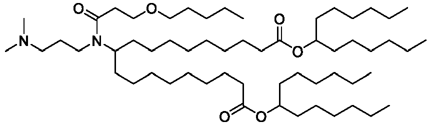 |
| 126 | 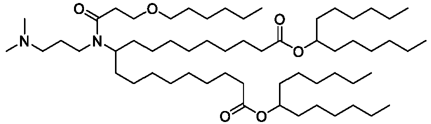 |
| 127 | 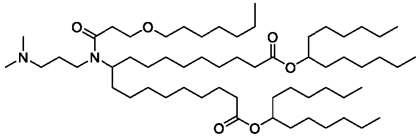 |
| 128 | 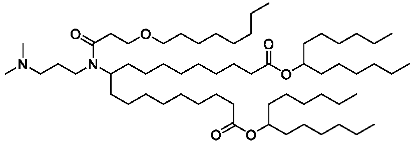 |
| 129 | 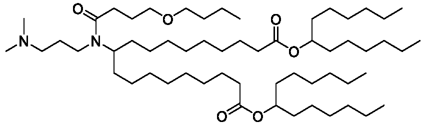 |
| 130 | 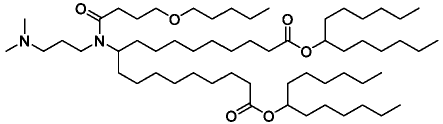 |
| 131 | 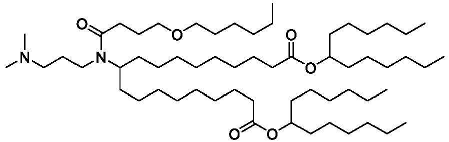 |
| 132 | 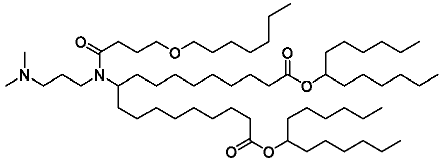 |
| 133 | 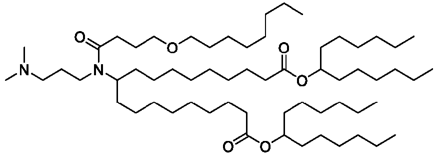 |
| 144 | 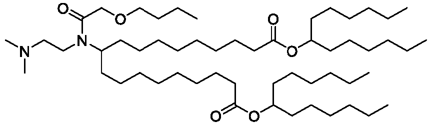 |
| 145 | 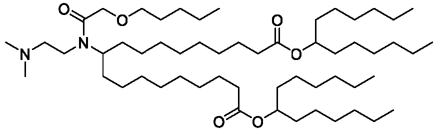 |
| 146 | 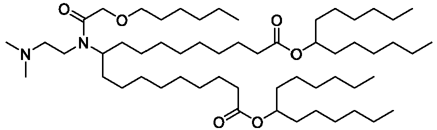 |
| 147 | 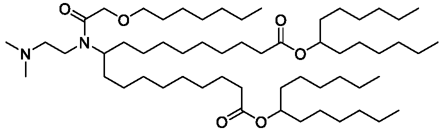 |
| 148 | 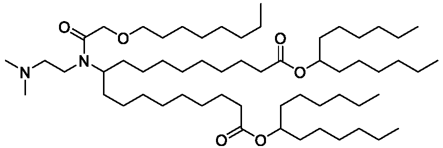 |
| 149 | 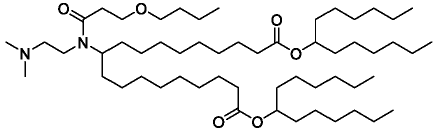 |
| 150 | 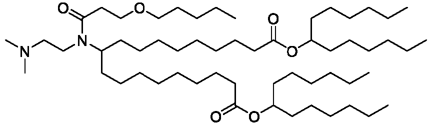 |
| 151 | 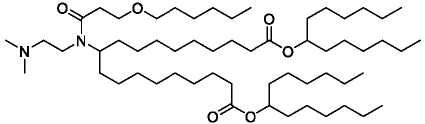 |
| 152 | 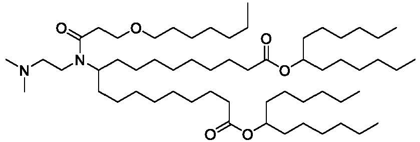 |
| 153 | 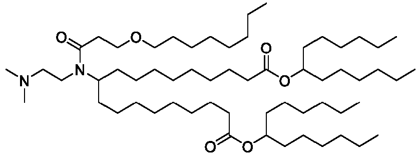 |
| 154 | 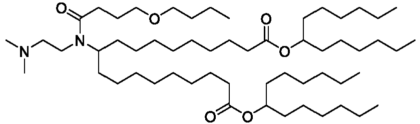 |
| 155 | 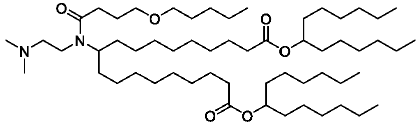 |
| 156 | 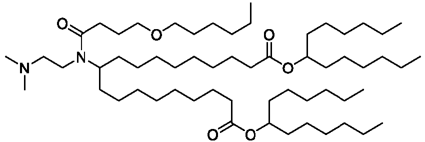 |
| 157 | 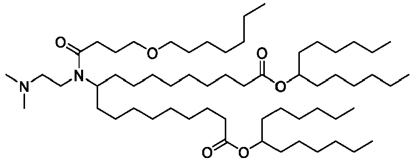 |
| 158 | 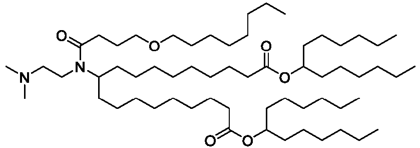 |
| 166 | 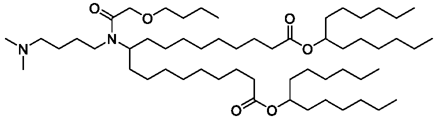 |
| 167 | 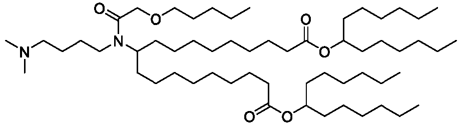 |
| 168 | 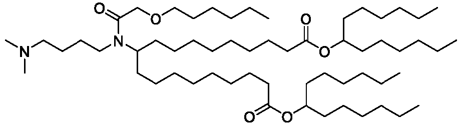 |
| 169 | 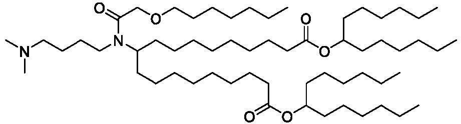 |
| 170 | 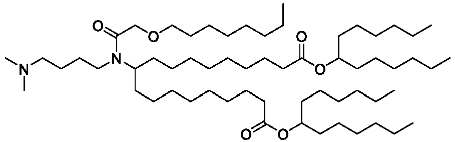 |
| 171 | 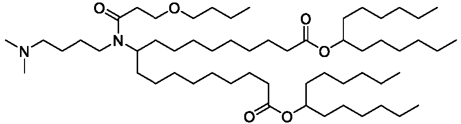 |
| 172 | 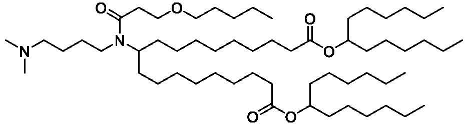 |
| 173 | 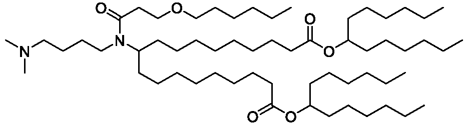 |
| 174 | 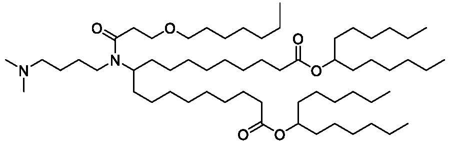 |
| 175 | 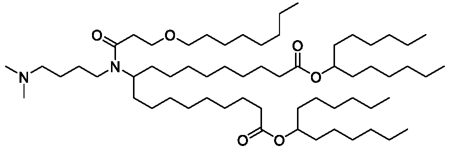 |
| 176 | 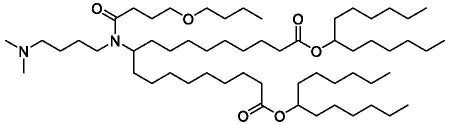 |
| 177 | 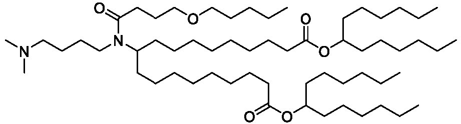 |
| 178 | 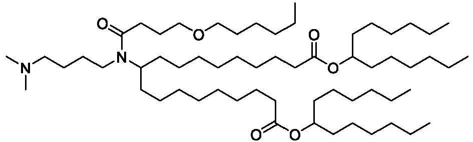 |
| 179 | 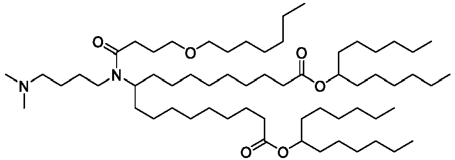 |
| 180 | 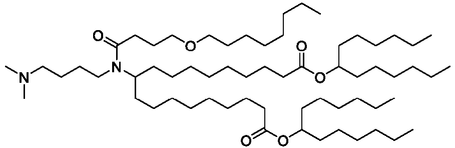 |

25. A lipid nanoparticle comprising the amino lipid compound of any one of claims 1 to 24.

26. The lipid nanoparticle according to claim 25, wherein the lipid nanoparticle further comprises one or more of a helper lipid, a structural lipid, and a PEG-lipid;
further, the lipid nanoparticle further comprises the helper lipid, the structural lipid, and the PEG-lipid.

27. The lipid nanoparticle according to claim 26, wherein the lipid nanoparticle comprises, based on the total amount of the amino lipid compound of any one of claims 1 to 24, the helper lipid, the structural lipid, and the PEG-lipid:
the amino lipid compound of any one of claims 1 to 24 in an amount (molar percent) of about 25.0% to 75.0%, such as about 25.0%-28.0%, 28.0%-32.0%, 32.0%-35.0%, 35.0%-40.0%, 40.0%-42.0%, 42.0%-45.0%, 45.0%-48.0%, 48.0%-55.0%, 55.0%-65.0%, 65.0%-75.0%, 45.0%-46.3%, 46.3%-48.0%, 48.0%-49.5%, 49.5%-50.0%, 50.0%-55.0%, or 60.0%-65.0%;
and/or
the helper lipid in an amount (molar percent) of about 5.0% to 45.0%, such as about 5.0%-10.0%, 10.0%-16.0%, 16.0%-25.0%, 25.0%-33.5%, 33.5%-37.0%, 37.0%-40.0%, 40.0%-42.0%, 42.0%-45.0%, 5.0%-9.0%, 9.0%-9.4%, 9.4%-10.0%, 10.0%-10.5%, 10.5%-11.0%, 11.0%-15.0%, 15.0%-16.0%, 16.0%-18.0%, 18.0%-20.0%, or 20.0%-25.0%;
and/or
the structural lipid in an amount (molar percent) of about 0.0% to 50.0%, such as about 0.0%-10.0%, 10.0%-15.5%, 15.5%-22.5%, 22.5%-35.0%, 35.0%-36.5%, 36.5%-39.5%, 39.5%-40.5%, 40.5%-41.5%, 41.5%-45.0%, 45.0%-46.5%, 46.5%-50.0%, 15.5%-18.5%, 18.5%-22.5%, 22.5%-23.5%, 23.5%-28.5%, 28.5%-33.5%, 33.5%-35.0%, 36.5%-38.0%, 38.0%-38.5%, 38.5%-39.0%, 39.0%-39.5%, 41.5%-42.5%, 42.5%-42.7%, 42.7%-43.0%, 43.0%-43.5%, 43.5%-45.0%, or 46.5%-48.5%; or about 50.0%-55.0%, such as about 50.0%-51.5%, 51.5%-53.5%, or 53.5%-55.0%;
and/or
the PEG-lipid in an amount (molar percent) of about 0.5% to 5.0%, such as about 0.5%-1.0%, 1.0%-1.5%, 1.5%-2.0%, 2.0%-2.5%, 2.5%-3.0%, 3.0%-3.5%, 3.5%-4.0%, 4.0%-4.5%, 4.5%-5.0%, 1.5%-1.6%, or 1.6%-2.0%.

28. The lipid nanoparticle according to any one of claims 26 or 27, wherein:
the helper lipid is a phospholipid;
preferably, the helper lipid is one or more selected from the group consisting of DSPC, DOPE, DOPC, DOPS, DSPG, DPPG, DPPC, DGTS and lysophospholipid,
preferably, the helper lipid is one or more selected from the group consisting of DSPC, DOPE, DOPC and DOPS;
preferably, the helper lipid is DSPC and/or DOPE;
and/or
the structural lipid is a sterol;
preferably, the structural lipid is at least one selected from the group consisting of cholesterol, cholesterol ester, steroid hormone, steroid vitamin, bile acid, cholesterin, ergosterol, β-sitosterol, and oxidized cholesterol derivative;
preferably, the structural lipid is at least one selected from the group consisting of cholesterol, cholesteryl ester, sterol hormone, sterol vitamin and bile acid;
preferably, the structural lipid is cholesterol, even more preferably high purity cholesterol, especially injection grade high purity cholesterol such as CHO-HP;
and/or
the PEG-lipid is a conjugate of polyethylene glycol and a lipid structure;
preferably, the PEG-lipid is selected from the group consisting of PEG-DMG and PEG-DSPE, preferably PEG-DMG;
preferably, the PEG-DMG is a polyethylene glycol (PEG) derivative of 1,2-dimyristoyl-sn-glycerol;
preferably, the PEG has an average molecular weight of about 2,000 to 5,000, preferably about 2,000, such as PEG2000-DMG.

29. The lipid nanoparticle according to any one of claims 26 to 28, wherein the molar ratio of the amino lipid compound of any one of claims 1 to 24: helper lipid: structural lipid: PEG-lipid is about 45 : 11 : 41.5 : 2.5, or 42.0 : 10.5 : 45.0 : 2.5, or 42.0 : 16.0 : 39.5 : 2.5, or 40.0 : 16.0 : 41.5 : 2.5, or 40.0 : 18.0 : 39.5 : 2.5, or 35.0 : 16.0 : 46.5 : 2.5, or 35.0 : 25.0 : 36.5 : 3.5, or 28.0 : 33.5 : 35.0 : 3.5, or 32.0 : 37.0 : 40.5 : 0.5, or 35.0 : 40.0 : 22.5 : 2.5, or 40.0 : 42.0 : 15.5 : 2.5, or 45 : 10 : 42.5 : 2.5, or 40.0 : 20.0 : 38.5 : 1.5, or 45.0 : 15.0 : 38.5 : 1.5, or 55.0 : 5.0 : 38.5 : 1.5, or 60.0 : 5.0 : 33.5 : 1.5, or 45.0 : 20.0 : 33.5 : 1.5, or 50.0 : 20.0 : 28.5 : 1.5, or 55.0 : 20.0 : 23.5 : 1.5, or 60.0 : 20.0 : 18.5 : 1.5, or 40.0 : 15.0 : 43.5 : 1.5, or 50.0 : 15.0 : 33.5 : 1.5, or 55.0 : 15.0 : 28.5 : 1.5, or 60.0 : 15.0 : 23.5 : 1.5, or 40.0 : 10.0 : 48.5 : 1.5, or 45.0 : 10.0 : 43.5 : 1.5, or 55.0 : 10.0 : 33.5 : 1.5, or 40.0 : 5.0 : 53.5 : 1.5, or 45.0 : 5.0 : 48.5 : 1.5, or 50.0 : 5.0 : 43.5 : 1.5;
preferably, wherein the helper lipid is DOPE, and the structural lipid is CHO-HP.

30. The lipid nanoparticle according to any one of claims 26 to 28, wherein the molar ratio of the amino lipid compound of any one of claims 1 to 24: helper lipid: structural lipid: PEG-lipid is about 48.0 : 10.0 : 40.5 : 1.5, or 50.0 : 10.0 : 38.5 : 1.5, or 50.0 : 9.0 : 38.0 : 3.0, or 49.5 : 10.0 : 39.0 : 1.5, or 46.3 : 9.4 : 42.7 : 1.6, or 45.0 : 9.0 : 43.0 : 3.0, or 45.0 : 11.0 : 41.5 : 2.5, or 42.0 : 10.5 : 45.0 : 2.5, or 42.0 : 16.0 : 39.5 : 2.5, or 40.0 : 16.0 : 41.5 : 2.5, or 40.0 : 18.0 : 39.5 : 2.5, or 35.0 : 40.0 : 22.5 : 2.5, or 40.0 : 20.0 : 38.5 : 1.5, or 45.0 : 15.0 : 38.5 : 1.5, or 55.0 : 5.0 : 38.5 : 1.5, or 60.0 : 5.0 : 33.5 : 1.5, or 45.0 : 20.0 : 33.5 : 1.5, or 50.0 : 20.0 : 28.5 : 1.5, or 55.0 : 20.0 : 23.5 : 1.5, or 60.0 : 20.0 : 18.5 : 1.5, or 40.0 : 15.0 : 43.5 : 1.5, or 50.0 : 15.0 : 33.5 : 1.5, or 55.0 : 15.0 : 28.5 : 1.5, or 60.0 : 15.0 : 23.5 : 1.5, or 40.0 : 10.0 : 48.5 : 1.5, or 45.0 : 10.0 : 43.5 : 1.5, or 55.0 : 10.0 : 33.5 : 1.5, or 40.0 : 5.0 : 53.5 : 1.5, or 45.0 : 5.0 : 48.5 : 1.5, or 50.0 : 5.0 : 43.5 : 1.5;
preferably, wherein the helper lipid is DSPC, and the structural lipid is CHO-HP.

31. The lipid nanoparticle according to any one of claims 25 to 30, further comprising a nucleic acid;
preferably, wherein the mass ratio of the amino lipid compound of any one of claims 1 to 24 to the nucleic acid is about (5-30): 1, such as about (5-10): 1, (10-15): 1, (15-20): 1, (20-25): 1, or (25-30): 1, preferably about 10:1;
and/or
preferably, wherein the nucleic acid is selected from the group consisting of RNA, antisense oligonucleotide, and DNA;
preferably, wherein the RNA is selected from the group consisting of messenger RNA (mRNA), ribosomal RNA (rRNA), microRNA (miRNA), transfer RNA (tRNA), small interfering RNA (siRNA), small nuclear RNA (snRNA), small hairpin RNA (shRNA), single guide RNA (sgRNA), Cas9 mRNA, or a mixture thereof;
and/or
preferably, wherein the DNA is a plasmid.

32. A composition comprising the amino lipid compound of any one of claims 1 to 24 or the lipid nanoparticle of any one of claims 25 to 31, and a pharmaceutically acceptable carrier, diluent or excipient.

33. The composition according to claim 32, wherein:
the composition further comprises a buffer;
preferably, wherein the buffer is selected from phosphate buffer and Tris buffer, preferably phosphate buffer; and/or
preferably, wherein the buffer has a concentration of about 5 mmol/L to about 30 mmol/L, preferably about 10 mmol/L; and/or
preferably, wherein the buffer has a pH of about 6-8, preferably about 7-8, and more preferably about 7-7.5;
and/or
the composition further comprises a cryoprotectant;
preferably, wherein the cryoprotectant is selected from sucrose and trehalose, preferably sucrose; and/or
preferably, wherein the cryoprotectant has a concentration of about 50 mg/ml to 100 mg/ml.

34. The composition according to any one of claim 32 or 33, which is a pharmaceutical composition.

35. Use of the compound of any one of claims 1 to 24 in the manufacture of a vehicle for an active ingredient;
preferably, wherein:
the vehicle is the lipid nanoparticle of any one of claims 25 to 31; and/or
the active ingredient is a pharmaceutical active ingredient, preferably a nucleic acid; and/or
preferably, the nucleic acid is selected from the group consisting of RNA, antisense oligonucleotides, and DNA;
preferably, wherein the RNA is selected from the group consisting of messenger RNA (mRNA), ribosomal RNA (rRNA), microRNA (miRNA), transfer RNA (tRNA), small interfering RNA (siRNA), and small nuclear RNA (snRNA); and/or
preferably, wherein the DNA is a plasmid.

36. Use of the compound of any one of claims 1 to 24, the lipid nanoparticle of any one of claims 25 to 31 or the composition of any one of claims 32 to 34 in the manufacture of a medicament,
preferably, the medicament is for use in gene therapy, gene vaccination, or protein replacement therapy, antisense therapy, or therapy by interfering RNA;
further preferably, wherein the gene therapy is for use in the treatment of cancers and genetic diseases, wherein the cancers are preferably one or more selected from the group consisting of lung cancer, stomach cancer, liver cancer, esophageal cancer, colon cancer, pancreatic cancer, brain cancer, lymphoma, blood cancer, and prostate cancer; and/or the genetic diseases are preferably one or more selected from the group consisting of hemophilia, thalassemia, and Gaucher's disease;
and/or
wherein the gene vaccination is for use in the treatment of cancer, allergy, toxicity, and pathogen infection, wherein the pathogen is preferably one or more selected from the group consisting of virus, bacterium, or fungi.

37. Use of the compound of any one of claims 1 to 24, the lipid nanoparticle of any one of claims 25 to 31 or the composition of any one of claims 32 to 34 in the manufacture of a medicament for nucleic acid transfer;
preferably, wherein the nucleic acid is selected from the group consisting of RNA, antisense oligonucleotide, and DNA;
preferably, wherein the RNA is selected from the group consisting of messenger RNA (mRNA), ribosomal RNA (rRNA), microRNA (miRNA), transfer RNA (tRNA), small interfering RNA (siRNA), and small nuclear RNA (snRNA); and/or
preferably, wherein the DNA is a plasmid.
